# EUROPEAN PATENT SPECIFICATION

(11) **EP 1 858 900 B1**
(45) Date of publication and mention of the grant of the patent: **07.01.2009**
(21) Application number: 06710058.6
(22) Date of filing: 10.03.2006
(51) Int. Cl.: C07D 513/04, A61K 31/55, A61P 25/00

(54) **FUSED THIAZOLE DERIVATIVES HAVING AFFINITY FOR THE HISTAMINE H3 RECEPTOR**
KONDENSIERTE THIAZOLDERIVATE MIT AFFINITÄT FÜR DEN HISTAMIN-H3-REZEPTOR
DERIVES DU THIAZOLE FONDUS AYANT UNE AFFINITE POUR LE RECEPTEUR DE L'HISTAMINE H3

(30) Priority: 14.03.2005 GB 0505205; 12.12.2005 GB 0525239
(43) Date of publication of application: 28.11.2007
(73) Proprietor: GLAXO GROUP LIMITED, Greenford, Middlesex UB6 ONN (GB)
(72) Inventor: BAILEY, Nicholas, Harlow, Essex CM19 5AW (GB); PICKERING, Paula Louise, Harlow, Essex CM19 5AW (GB); WILSON, David Matthew, Harlow, Essex CM19 5AW (GB)
(74) Representative: Breen, Anthony Paul
(86) International application number: PCT/GB2006/000846
(87) International publication number: WO 2006/097691

(56) References cited:
- WO-A-00/63208
- WO-A-20/04035544

## Description

The present invention relates to novel thiazole derivatives having pharmacological activity, processes for their preparation, to compositions containing them and to their use in the treatment of neurological and psychiatric disorders.

The histamine H3 receptor is predominantly expressed in the mammalian central nervous system (CNS), with minimal expression in peripheral tissues except on some sympathetic nerves (Leurs et al., (1998), Trends Pharmacol Sci. 19, 177-183). Activation of H3 receptors by selective agonists or histamine results in the inhibition of neurotransmitter release from a variety of different nerve populations, including histaminergic and cholinergic neurons (Schlicker et al., (1994), Fundam. Clin. Pharmacol. 8, 128-137). Additionally, *in vitro* and *in vivo* studies have shown that H3 antagonists can facilitate neurotransmitter release in brain areas such as the cerebral cortex and hippocampus, relevant to cognition (Onodera et al., (1998), In: The Histamine H3 receptor, ed Leurs and Timmerman, pp255-267, Elsevier Science B.V.). Moreover, a number of reports in the literature have demonstrated the cognitive enhancing properties of H3 antagonists (e.g. thioperamide, clobenpropit, ciproxifan and GT-2331) in rodent models including the five choice task, object recognition, elevated plus maze, acquisition of novel task and passive avoidance (Giovanni et al., (1999), Behav. Brain Res. 104, 147-155). These data suggest that novel H3 antagonists and/or inverse agonists such as the current series could be useful for the treatment of cognitive impairments in neurological diseases such as Alzheimer's disease and related neurodegenerative disorders.

WO2005/009387 (X-ceptor Therapeutics Inc.) disclose a series of azepine derivatives that are disclosed to be modulators of the farnesoid X receptor and are claimed to be useful in the treatment of a number of disorders including hyperlipidemia. US 5607944 (assigned to Karl Thomae: GmbH) describes bicyclic heterocyclic compounds and their use in inhibiting aggregation. JP 10017569 (Yamanouchi Pharmaceutical Co. Ltd.) describe a series of 2-phenyl -substituted thiazole derivatives which are 5HT3 agonists. The compounds are disclosed to be useful in the treatment of GI tract movement disorders. WO 96/04271 (Karl Thomae GmbH) describes a series of condensed azepine derivatives and their use in the treatment of a number of diseases including venous and arterial thrombosis.

Compounds having affinity for the histamine H3 receptor are disclosed in WO 2004/035544 and WO 00/63208.

The present invention provides, in a first aspect, a compound of formula (I) or a pharmaceutically acceptable salt thereof; 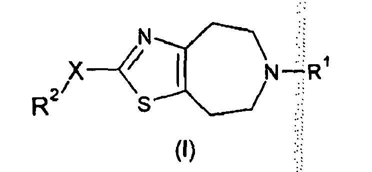 wherein:
R¹ represents -C₂₋₆ alkyl, -C₁₋₃ alkyl-C₃₋₈ cycloalkyl or -C₃₋₇ cycloalkyl, wherein the cycloalkyl groups may be optionally substituted by C₁₋₃ alkyl;
X represents aryl, heteroaryl or heterocyclyl;
R² represents hydrogen, halogen, hydroxy, cyano, nitro, =O, -NR⁸R⁹, -Y-H, -Y-C₁₋₆ alkyl, -Y-C₃₋₈ cycloalkyl, -Y-C₁₋₆alkylC₃₋₈cycloalkyl, -Y-aryl, -Y-heterocyclyl, -Y-heteroaryl, -Y-C₁₋₆ alkyl-aryl, -C₁₋₆ alkyl-Y-H, -C₁₋₆ alkyl-Y-C₁₋₆ alkyl, -C₁₋₆ alkyl-Y-C₃₋₈ cycloalkyl, -C₁₋₆ alkyl-Y-C₁₋₆alkylC₃₋₈cycloalkyl, -C₁₋₆ alkyl-Y-aryl, -C₁₋₆ alkyl-Y-heterocyclyl or -C₁₋₆ alkyl-Y-heteroaryl, wherein R⁸ and R⁹ are independently selected from the group consisting of hydrogen and methyl;
Y represents a bond, C₁₋₆ alkyl, CO, CO₂, CONR³, NR³CO, O, S, SO, SO₂, -SO₂-O-, SO₂NR³, NR³SO₂, OCONR³, NR³CO₂ or NR³CONR (wherein R³ and R⁴ independently represent hydrogen, -C₁₋₆ alkyl, -C₃₋₈ cycloalkyl, -C₁₋₆ alkylC₃₋₈ cycloalkyl, -aryl, -heterocyclyl or -heteroaryl);
wherein said aryl, heteroaryl and heterocyclyl groups of X may optionally be substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁₋₆ alkyl;
wherein said alkyl, cycloalkyl, aryl, heteroaryl and heterocyclyl groups of R² may be optionally substituted by 1, 2 or 3 substituents which may be the same or different, and which are halogen, cyano, nitro, =O, or a group -R⁵, -OR⁵, -OC₁₋₆ alkyl-R⁶, - C₁₋₆ alkyl-OR⁶, -CO₂R⁵, -COR⁵, COR⁵R⁶, -C₁₋₆ alkyl-COR⁵, -SR⁵, -SO₂R⁵, -SOR⁵, -OSO₂R⁵, - C₁₋₆ alkyl-SO₂R⁶, -C₁₋₆ alkyl-NR⁵SO₂R⁶, -C₁₋₆ alkyl-SO₂NR⁵R⁸,-NR⁵R⁶, -C₁₋₆ alkyl-NR⁵R⁶, -C₃₋₈ cycloalkyl-NR⁵R⁶, -CONR⁵R⁸, -NR⁵COR⁶, -C₁₋₆ alkyl-NR⁵COR⁶, -C₁₋₆ alkyl-CONR⁵R⁶, -NR⁵SO₂R⁶, -OCONR⁵R⁶, -NR⁵CO₂R⁶, -NR⁷CONR⁵R⁶ or -SO₂NR⁵R⁶ (wherein R⁵, R⁶ and R⁷ independently represent hydrogen, C₁₋₆ alkyl, -C₃₋₈ cycloalkyl, -C₁₋₆ alkyl-C₃₋₈ cycloalkyl, aryl, heterocyclyl or heteroaryl or wherein -NR⁵R⁶ may represent a nitrogen containing heterocyclyl group), provided that where R¹ represents -C₂₋₈ alkyl or -C₁₋₃ alkyl-C₃₋₈ cycloalkyl, the alkyl, cycloalkyl, aryl, heteroaryl and heterocyclyl groups of R² may not be substituted with -CO₂R⁵;
wherein R³, R⁴, R⁵, R⁶ and R⁷ may be optionally substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁₋₆ alkyl; and provided that the aryl and heteroaryl groups of X, R², R³, R⁴, R⁵, R⁶ and R⁷ may only be substituted by =O if the substituted group is aromatic;
or solvates thereof.

In another aspect, the aryl, heteroaryl or heterocyclyl group of X comprises a five or six membered ring, particularly a six membered ring.

In a further aspect, R² represents hydrogen, halogen, hydroxy, cyano, nitro, =O, -Y-H, -Y-C₁₋₆ alkyl, -Y-C₃₋₈ cycloalkyl, -Y-C₁₋₆alkylC₃₋₈cycloalkyl, -Y-aryl, -Y-heterocyclyl, -Y-heteroaryl, -Y-C₁₋₆ alkyl-aryl, -C₁₋₆ alkyl-Y-H, -C₁₋₆ alkyl-Y-C₁₋₆ alkyl, -C₁₋₆ alkyl-Y-C₃₋₈ cycloalkyl, -C₁₋₆ alkyl-Y-C₁-₆alkylC₃₋₈cycloalkyl, -C₁-₆ alkyl-Y-aryl, -C₁₋₆ alkyl-Y-heterocyclyl or -C₁₋₆ alkyl-Y-heteroaryl.

In a further aspect, the alkyl, cycloalkyl, aryl, heteroaryl and heterocyclyl groups of R² may be optionally substituted by 1, 2 or 3 substituents which may be the same or different, and which are halogen, cyano, nitro, =O, or a group -R⁵, -OR⁵, -OC₁₋₆ alkyl-R⁶, -C₁₋₆ alkyl-OR⁶, -COR⁵, COR⁵R⁶, -C₁₋₆ alkyl-COR⁵, -SR⁵, -SO₂R⁵, -SOR⁵, -OSO₂R⁵, - C₁₋₆ alkyl-SO₂R⁶, -C₁₋₆ alkyl-NR⁵SO₂R⁶, -C₁₋₆ alkyl-SO₂NR⁵R⁶, -NR⁵R⁶, -C₁₋₆ alkyl-NR⁵R⁶, -C₃₋₈ cycloalkyl-NR⁵R⁶, -CONR⁵R⁶, -NR⁵COR⁶, -C₁₋₆ alkyl-NR⁵COR⁶, -C₁₋₆ alkyl-CONR⁵R⁶, -NR⁵SO₂R⁶, -OCONR⁵R⁶ -NR⁵CO₂R⁶, -NR⁷CONR⁵R⁶ or -SO₂NR⁵R⁶ (wherein R⁵, R⁶ and R⁷ independently represent hydrogen, C₁₋₆ alkyl, -C₃₋₈ cycloalkyl, -C₁₋₆ alkyl-C₃₋₈ cycloalkyl, aryl, heterocyclyl or heteroaryl or wherein -NR⁵R⁶ may represent a nitrogen containing heterocyclyl group).

In a further aspect in which R¹ represents -C₂₋₆ alkyl and X represents phenyl, R² is other than halogen, -Y-H or -Y-C₁₋₆ alkyl, wherein Y represents a bond, O or S.

In one aspect, the invention provides compounds of formula (I) or pharmaceutically acceptable salts of solvates thereof, wherein:
R¹ represents -C₁₋₃ alkyl-C₃₋₈ cycloalkyl or -C₃₋₇ cycloalkyl, wherein the cycloalkyl group may be optionally substituted by C₁₋₃ alkyl;
X represents aryl, heteroaryl or heterocyclyl;
R² represents hydrogen, halogen, hydroxy, cyano, nitro, =O, -Y-H, -Y-C₁₋₆ alkyl, -Y-C₃₋₈ cycloalkyl, -Y-C₁₋₆alkylC₃₋₈cycloalkyl, -Y-aryl, -Y-heterocyclyl, -Y-heteroaryl, -Y-C₁₋₆ alkyl-aryl, -C₁₋₆ alkyl-Y-H, -C₁₋₆ alkyl-Y-C₁₋₆ alkyl, -C₁₋₆ alkyl-Y-C₃₋₈ cycloalkyl, -C₁₋₆ alkyl-Y-C₁₋₆ alkylC₃₋₈cycloalkyl, -C₁₋₆ alkyl-Y-aryl, -C₁₋₆ alkyl-Y-heterocyclyl, or -C₁₋₆ alkyl-Y-heteroaryl;
Y represents a bond, C₁₋₆ alkyl, CO, CO₂, CONR³, N R³CO, O, SH, SO, SO₂, -SO₂-O-, SO₂N R³, NR³SO₂, OCONR³, NR³CO₂ or NR³CONR⁴ (wherein R³ and R⁴ independently represent hydrogen -C₁₋₆ alkyl, -C₃₋₈ cycloalkyl, -C₁₋₆ alkylC₃₋₈ cycloalkyl, -aryl,heterocyclyl or-heteroaryl);
wherein said aryl, heteroaryl and heterocyclyl groups of X may optionally be substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁-₆ alkyl;
wherein said alkyl, cycloalkyl, aryl, heteroaryl and heterocyclyl groups of R² may be optionally substituted by 1, 2 or 3 substituents which may be the same or different, and which are halogen, hydroxy, cyano, nitro, =O, or a group -R⁵, -OR⁵, -OC₁₋₆ alkyl-R⁶, - C₁₋₆ alkyl-OR⁶, -CO₂R⁵, -COR⁵, COR⁵R⁶, -C₁₋₆ alkyl-COR⁵, -SR⁵, -SO₂R⁵, -SOR⁵, -OSO₂R⁵,C₁₋₆ alkyl-SO₂R⁶, -C₁₋₆ alkyl-NR⁵SO₂R⁶, -C₁₋₆ alkyl-SO₂NR⁵R⁶, -NR⁵R⁶, -C₁₋₆ alkyl-NR⁵R⁶, -C₃₋₈ cycloalkyl-NR⁵R⁶, -CONR⁵R⁶, -NR⁵COR⁶, -C₁₋₆ alkyl-NR⁵COR⁶, -C₁₋₆ alkyl-CONR⁵R⁶, -NR⁵SO₂R⁶, -OCONR⁵R⁶, -NR⁵CO₂R⁶, -NR⁷CONR⁵R⁶ or -SO₂NR⁵R⁶ (wherein R⁵, R⁶ and R⁷ independently represent hydrogen, alkyl, -C₃₋₈ cycloalkyl, -C₁₋₆ alkyl-C₃₋₈ cycloalkyl, aryl, heterocyclyl or heteroaryl or wherein -NR⁵R⁶ may represent a nitrogen containing heterocyclyl group); and
wherein R³, R⁴, R⁵, R⁶ and R⁷ may be optionally substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁-₆ alkyl.

In a more particular embodiment of this aspect, R¹ represents -C₃-₇ cycloalkyl, wherein the cycloalkyl group may be optionally substituted by C₁₋₃ alkyl, Most particularly, R¹ represents unsubstituted -C₃₋₇ cycloalkyl.

The term 'C_{x-y} alkyl' as used herein as a group or a part of the group refers to a linear or branched saturated hydrocarbon group containing from x to y carbon atoms. Examples of C₁₋₆alkyl groups include methyl, ethyl, n-propyl, isopropyl, n-butyl, isobutyl, sec-butyl, tert butyl, n-pentyl, isopentyl, neopentyl or hexyl and the like.

The term 'C_{x-y} alkoxy' as used herein refers to an -O-C_{x-y} alkyl group wherein C_{x-y} alkyl is as defined herein. Examples of C₁₋₆ alkoxy groups include methoxy, ethoxy, propoxy, butoxy, pentoxy or hexoxy and the like.

The term 'C_{x-y} cycloalkyl' as used herein refers to a saturated monocyclic hydrocarbon ring of x to y carbon atoms. Examples of C₃₋₈ cycloalkyl groups include cyclopropyl, cyclobutyl, cyclopentyl, cyclohexyl, cycloheptyl or cyclooctyl and the like.

The term 'halogen' as used herein refers to a fluorine, chlorine, bromine or iodine atom.

The term 'haloC_{x-y} alkyl' as used herein refers to a C_{x-y} alkyl group as defined herein wherein at least one hydrogen atom is replaced with halogen. Examples of haloC₁₋₆ alkyl groups include fluoroethyl, trifluoromethyl or trifluoroethyl and the like.

The term 'aryl' as used herein refers to a C₆₋₁₂ monocyclic or bicyclic hydrocarbon ring wherein at least one ring is aromatic. Examples of such groups include phenyl, naphthyl or tetrahydronaphthalenyl, and the like.

The term 'heteroaryl' as used herein refers to a 5-6 membered monocyclic aromatic or a fused 8-10 membered bicyclic aromatic ring, which monocyclic or bicyclic ring contains 1 to 4 heteroatoms selected from oxygen, nitrogen and sulphur. Examples of such monocyclic aromatic rings include thienyl, furyl, furazanyl, pyrrolyl, triazolyl, tetrazolyl, imidazolyl, oxazolyl, thiazolyl, oxadiazolyl, isothiazolyl, isoxazolyl, thiadiazolyl, pyranyl, pyrazolyl, pyrimidyl, pyridazinyl, pyrazinyl, pyridyl, triazinyl,tetrazinyl and the like. Examples of such fused aromatic rings include quinolinyl, isoquinolinyl, quinazolinyl, quinoxalinyl, pteridinyl, cinnolinyl, phthalazinyl, naphthyridinyl, indolyl, isoindolyl, azaindolyl, indolizinyl, indazolyl, purinyl, pyrrolopyridinyl, furopyridinyl, benzofuranyl, isobenzofuranyl, benzothienyl, benzoimidazolyl, benzoxazolyl, benzoisoxazolyl, benzothiazolyl, benzoisothiazolyl, benzoxadiazolyl, benzothiadiazolyl and the like.

The term 'heterocyclyl' refers to a 4-7 membered monocyclic ring or a bridged or fused 8-12 membered bicyclic ring which may be saturated or partially unsaturated, which monocyclic or bicyclic ring contains 1 to 4 heteroatoms selected from oxygen, nitrogen or sulphur. Examples of such monocyclic rings include pyrrolidinyl, azetidinyl, pyrazolidinyl, oxazolidinyl, piperidinyl, piperazinyl, morpholinyl, thiomorpholinyl, thiazolidinyl, hydantoinyl, valerolactamyl, oxiranyl, oxetanyl, dioxolanyl, dioxanyl, oxathiolanyl, oxathianyl, dithianyl, tetrahydrofuranyl, tetrahydrofuranyl, dihydropyranyl, tetrahydropyranyl, tetrahydropyridinyl, tetrahydropyrimidinyl, tetrahydrothiophenyl, tetrahydrothiopyranyl, diazepanyl, azepanyl and the like. Examples of such bicyclic rings include indolinyl, isoindolinyl, benzoxazinyl, benzopyranyl, quinuclidinyl, 2,3,4,5-tetrahydro-1*H*-3-benzazepine, tetrahydroisoquinolinyl and the like.

The term 'nitrogen containing heterocyclyl' refers to a monocyclic or bicyclic heterocyclyl ring as defined above which heterocyclyl ring contains at least one nitrogen atom.

In one embodiment, R¹ represents:
- C₃₋₇ cycloalkyl (e.g. cyclobutyl, cyclopentyl or cyclohexyl) optionally substituted by C₁₋₃ alkyl;
- C₁₋₃ alkyl-C₃₋₈ cycloalkyl (e.g. -CH₂-cyclopropyl or -CH₂-cyclohexyl), wherein the cycloalkyl group is substituted by C₁₋₃ alkyl; or
- C₂₋₆ alkyl (e.g. ethyl, methylpropyl or methylethyl).

In a more particular embodiment, R¹ represents:
-C₃₋₇ cycloalkyl (e.g. cyclobutyl, cyclopentyl or cyclohexyl) optionally substituted by C₁₋₃ alkyl; or
-C₁₋₃ alkyl-C₃₋₈ cycloalkyl (e.g. -CH2-cyclopropyl or -CH₂-cyclohexyl), wherein the cycloalkyl group is optionally substituted by C₁₋₃ alkyl.

In an even more particular aspect, R¹ represents -C₃₋₇ cycloalkyl (e.g. cyclobutyl, cyclopentyl or cyclohexyl) optionally substituted by one or more C₁₋₃ alkyl groups, particularly unsubstituted -C₃₋₇ cycloalkyl (e.g. cyclobutyl, cyclopentyl or cyclohexyl).

Most particularly, R¹ represents unsubstituted cyclobutyl.

In another embodiment, X represents:
aryl (e.g. phenyl);
heteroaryl (e.g. pyridinyl, pyrimidinyl or pyrazinyl); or
heterocyclyl (e.g. piperidinyl, pyrrolidinyl, (1H)-pyrimidinyl or benzoxazinyl).

More particularly, X represents:
aryl (e.g. phenyl);
heteroaryl (e.g. pyridin-3-yl, pyridin-2-yl, pyrimidin-5-yl or pyrazin-2-yl); or
heterocyclyl (e.g. piperidin-4-yl, pyrrolidin-3-yl or 1,4-benzoxazin-7-yl).

Even more particularly, X represents aryl (e.g. phenyl) or heterocyclyl (e.g. piperidin-4-yl or pyrrolidin-3-yl).

Most particularly, X represents heterocyclyl (e.g. piperidin-4-yl or pyrrolidin-3-yl), particularly, piperidin-4-yl.

In one embodiment, X may optionally be substituted by one or more substituents (e.g. 1, 2 or 3) which may be the same or different, and which are selected from the group consisting of halogen, C₁₋₆ alkyl, C₁₋₆ alkoxy or haloC₁₋₆ alkyl.

More particularly, X is unsubstituted.

In certain embodiments in which X represents piperidin-4-yl or pyrrolidin-3-yl, this is linked to R² through the nitrogen atom.

In a further embodiment, R² represents hydrogen, halogen, cyano, =O, -Y-H, -Y-C₁₋₆ alkyl, Y-aryl, -Y-heterocyclyl , -Y-heteroaryl or -NR⁸R⁹ wherein R⁸ and R⁹ are independently selected from the group consisting of hydrogen and methyl.

In one embodiment of embodiment, Y is a bond, O, CO, CO₂ or CONR³, where R³ represents hydrogen. More particularly, Y is a bond or CO.

In one aspect, the alkyl, cycloalkyl, aryl, heteroaryl and heterocyclyl groups of R² may be optionally substituted by 1, 2 or 3 substituents which may be the same or different, and which are selected from the group consisting of halogen, cyano, =O, R⁵, COR⁵, CO₂R⁵, and -CONR⁵R⁶ (wherein R⁵ and R⁶ independently represent hydrogen, -C₁₋₆alkyl or heterocyclyl (e.g. imidazolidin-1-yl), and wherein R⁵ and R⁶ may optionally be further substituted by 1, 2 or 3 substituents selected from the group consisting of halogen andC₁₋₆-alkyl). More particularly, R⁵ and R⁶ independently hydrogen or -C₁₋₆alkyl, wherein R⁵ and R⁶ may optionally be further substituted by one or more halogen atoms.

Even more particularly, the alkyl, cycloalkyl, aryl, heterocyclyl and heterocyclyl groups of R² may be optionally substituted by 1, 2 or 3 substituents which may be the same or different, and which are selected from the group consisting of:
halogen (e.g. bromo or chloro);
cyano;
=O;
-R⁵ such as C₁₋₆alkyl (e.g. Me) or haloC₁₋₆alkyl (e.g. -CF₃);
-CO₂R⁵ (e.g. -CO₂H); and
-CONR⁵R⁶ (e.g. -CONH₂, -CON(H)(Me), -CON(Me)(Me)).

Most particularly, the alkyl, cycloalkyl, aryl, heteroaryl and heterocyclyl groups of R² may be optionally substituted by 1, 2 or 3 substituents which may be the same or different, and which are selected from the group consisting of:
cyano;
=O;
-R⁵ such as C₁₋₆alkyl (e.g. Me) or haloC₁₋₆alkyl (e.g. -CF₃); and
-CONR⁵R⁶ (e.g. -CONH₂ or -CON(Me)(Me)).

In a more particular embodiment, R² represents
-hydrogen;
-halogen (e.g. Br);
cyano;
=O;
-Y-H (e.g. -CO₂H or -CONH₂)
-Y-C₁₋₆alkyl (e.g. methyl, -O-methyl, -CO₂-methyl, -CO₂-t-butyl, -CONH-methyl, - CONH-ethyl or -CONH-(1-methylethyl)) optionally substituted with one or more halogen (e.g. fluorine) atoms (i.e. -CF₃);
-Y-aryl (e.g. phenyl, -CO-phenyl or -CONH-phenyl) optionally substituted with one or more C₁₋₆ alkyl (e.g. methyl), cyano or halogen (e.g. chloro) groups;
-Y-heterocyclyl (e.g. pyrrolidinyl, imidazolidinyl, oxazolidinyl, piperidinyl, morpholinyl, -CO-pyrrolidinyl, -CO-isothiazolidinyl or-CO-morpholinyl) optionally substituted with one or more substituents selected from C₁₋₆ alkyl (e.g. methyl) and =O;
-Y-heteroaryl (e.g. -thiadiazolyl, -pyridinyl, oxadiazolyl, -CO-thiadiazolyl, -CO-pyridinyl, -CO-pyrazinyl, -CO-isoxazolyl, -CO-pyrazolyl, -CO-pyrimidinyl, -CO-pyridazinyl, -CO-imidazolyl or-CO-pyrrolyl) optionally substituted with one or more substituents selected from cyano, C₁₋₆alkyl (e.g. Me), haloC₁₋₆alkyl (e.g.CF₃), -COR⁵ (e.g -CO-imidazolidinyl)-CO₂R⁵ (e.g. -CO₂H) or -CONR⁵R⁶ (e.g.CONH₂ and -CON(Me)(Me)); or
-NR⁸R⁹ wherein R⁸ and R⁹ are independently selected from the group consisting of hydrogen and methyl.

Even more particularly, R² represents:
-Y-heterocyclyl (e.g. pyrrolidin-1-yl, imidazolidin-1-yl, oxazolidin-3-yl, piperidin-1-yl, morpholin-4-yl, -CO-pyrrolidin-1-yl, -CO-isothiazolidin-2-yl or-CO-morpholin-4-yl) optionally substituted with one or more substituents selected from C₁₋₆ alkyl (e.g. methyl) and =O (1-pyrrolidin-2-one or 1-imidazolidin-2-one); or
-Y-heteroaryl (e.g: 1,2,3-thiadiazol-4-yl, -pyridin-3-yl, -pyridin-2-yl, 1,2,4-oxadiazol-5-yl, -CO-1,2,3-thiadiazol-4-yl, -CO-pyridin-3-yl, -CO-pyridin-2-yl, -CO-pyridin-4-yl, -CO-pyrazin-2-yl, -CO-isoxazol-3-yl, -CO-isoxazol-5-yl, -CO-pyrazol-3-yl, -CO-pyrimidin-4-yl, -CO-pyrimidin-5-yl, -CO-pyridazin-3-yl, -CO-imidazol-5-yl, -CO-imidazol-4-yl or -CO-pyrrol-2-yl) optionally substituted with one or more substituents selected from cyano, C₁₋₆alkyl (e.g. Me), haloC₁₋₆alkyl (e.g. -CF₃),COR⁵ (e.g -CO-imidazolidinyl) -CO₂R⁵ (e.g. -CO₂H) or -CONR⁵R⁶ (e.g. -CONH₂ and -CON(Me)(Me)).

In certain embodiments where R² represents 1,2,3-thiadiazol-4-yl or -CO-1,2,3-thiadiazol-4-yl, the heteroaryl group is unsubstituted.

In one embodiment where R² represents -pyridin-3-yl, it may optionally be substituted on the heteroaryl by one or more substituents selected from haloC₁₋₆alkyl (e.g. -CF₃), -C₁₋₆ alkyl (e.g. methyl), -COR⁵ (e.g -CO-imidazolidinyl), -CO₂R⁵ (e.g. -CO₂H), -CONR⁵R⁶ (e.g. -CONH₂, -CONH(Me) and -CON(Me)(Me)) or cyano.

In one embodiment where R² represents -pyridin-2-yl, it may optionally be substituted on the heteroaryl by one or more C₁-₆alkyl (e.g. methyl) groups.

In one embodiment where R² represents 1,2,4-oxadiazol-5-yl, it may optionally be substituted on the heteroaryl by one or more C₁₋₆alkyl (e.g. methyl) groups.

In one embodiment where R² represents -CO-pyridin-3-yl; it may optionally be substituted on the heteroaryl by one or more substituents from haloC₁-₆alkyl (e.g. -CF₃), C₁₋₆alkyl (e.g. methyl) or cyano.

In one embodiment where R² represents -CO-pyridin-2-yl, it may optionally be substituted on the heteroaryl by one or more C₁₋₆alkyl (e.g. methyl) groups.

In one embodiment where R² represents -CO-pyridin-4-yl, Heteroaryl group is unsubstituted.

In one embodiment where R² represents -CO-pyrazin-2-yl, it may optionally be substituted on the heteroaryl by one or more C₁₋₆alkyl (e.g. methyl) groups.

In one embodiment where R² represents -CO-isoxazol-3-yl, it may optionally be substituted on the heteroaryl by one or more C₁₋₆alkyl (e.g. methyl) groups.

In one embodiment where R² represents -CO-isoxazol-5-yl, it may optionally be substituted on the heteroaryl by one or more C₁₋₆alkyl (e.g. methyl) groups.

In one embodiment where R² represents -CO-pyrazol-3-yl, it may optionally be substituted on the heteroaryl by one or more C₁₋₆alkyl (e.g. methyl) groups.

In one embodiment where R² represents -CO-pyrimidin-4-yl, it may optionally be substituted on the heteroaryl by one or more C₁₋₆alkyl (e.g. methyl) groups.

In one embodiment where R² represents -CO-pyrimidin-5-yl, it may optionally be substituted on the heteroaryl by one or more C₁₋₆alkyl (e.g. methyl) groups.

In one embodiment where R² represents -CO-pyridazin-3-yl, it may optionally be substituted on the heteroaryl by one or more C₁₋₆alkyl (e.g. methyl) groups.

In one embodiment where R² represents -CO-imidazol-5-yl, it may optionally be substituted on the heteroaryl by one or more C₁₋₆alkyl (e.g. methyl) groups.

In one embodiment where R² represents -CO-imidazol-4-yl, it may optionally be substituted on the heteroaryl by one or more C₁₋₆alkyl (e.g. methyl) groups.

In one embodiment where R² represents -CO-pyrrol-2-yl, it may optionally be substituted on the heteroaryl by one or more C₁₋₆alkyl (e.g. methyl) groups.

In one aspect, the invention provides compounds of formula (I) or pharmaceutically acceptable salts or solvates thereof, wherein:
R¹ represents-C₂₋₆ alkyl, -C₁₋₃alkyl-C₃₋₈ cycloalkyl or -C₃₋₇ cycloalkyl, wherein the cycloalkyl groups may be optionally substituted by C₁₋₃ alkyl;
X represents aryl, heteroaryl or heterocyclyl;
R² represents hydrogen, halogen, cyano, =O, -Y-H, -Y-C₁₋₆alkyl, Y-aryl, -Y-heterocyclyl, -Y-heteroaryl or -NR⁸R⁹ Wherein R⁸ and R⁹ are independently selected from the group consisting of hydrogen and methyl;
Y represents a bond, O, CO, CO₂ or CONR³, where R³ represents hydrogen;
wherein said aryl, heteroaryl and heterocyclyl groups of X optionally be substituted by 1, 2 or 3 substituents which may be the same or and which are selected from the group consisting of halogen, C₁₋₆ alkyl, C₁₋₈ alkoxy and haloC₁₋₆ alkyl;
wherein said alkyl, aryl, heteroaryl and heterocyclyl groups of R² may be optionally substituted by 1, 2 or 3 substituents which may be the same or different, and which are selected from the group consisting of cyano, halogen, =O, R⁵, COR⁵, CO₂R⁵, andCONR⁵R⁶ (wherein R⁵ and R⁶ independently represent hydrogen, -C₁₋₆alkyl or heterocyclyl, and wherein R⁵ and R⁶ may optionally be further substituted by 1, 2 or 3 substituents selected from the group consisting of halogen and -C₁₋₆alkyl); provided that where R¹ represents -C₂₋₆ alkyl or -C₁₋₃ alkyl-C₃₋₈ cycloalkyl, the alkyl, aryl, heteroaryl and heterocyclyl groups of R² may not be substituted with -CO₂R⁵ and provided that the aryl and heteroaryl groups of X and R² may only be substituted by =O if the substituted group is aromatic.

In one aspect, the invention provides compounds of formula (I) or pharmaceutically acceptable salts or solvates thereof, wherein:
R¹ represents -C₂₋₆ alkyl, C₁₋₃ alkyl-C₃₋₈ cycloalkyl or -C₃₋₇ cycloalkyl, wherein the cycloalkyl groups may be optionally substituted by C₁₋₃ alkyl;
X represents aryl, heteroaryl or heterocyclyl;
R² represents hydrogen, halogen, -Y-C₁₋₆alkyl, Y-aryl, -Y-heterocyclyl, or -Y-heteroaryl; Y represents a bond, CO or CO₂;
wherein said aryl, heteroaryl and heterocyclyl groups of X may optionally be substituted by 1, 2 or 3 substituents which may be the same or different, and which are selected from the group consisting of halogen, C₁₋₆ alkyl, C₁₋₆ alkoxy and haloC₁₋₆ alkyl;
wherein said alkyl, aryl, heteroaryl and heterocyclyl groups of R² may be optionally substituted by 1, 2 or 3 substituents which may be the same or different, and which are selected from the group consisting of cyano, =O, R⁵, COR⁵, CO₂R⁵, and -CONR⁵R⁶ (wherein R⁵ and R⁶ independently represent hydrogen, -C₁₋₆alkyl or heterocyclyl, and
wherein R⁵ and R⁶ may optionally be further substituted by 1, 2 or 3 substituents selected from the group consisting of halogen and -C₁₋₆alkyl);
provided that where R¹ represents -C₂₋₆ alkyl or -C₁₋₃ alkyl-C₃₋₈ cycloalkyl, the alkyl, aryl, heteroaryl and heterocyclyl groups of R² may not be substituted with -CO₂R⁵, and provided that the aryl and heteroaryl groups of R² may only be substituted by =O if the substituted group is aromatic.

In a more particular aspect, the invention provides compounds of formula (I) or pharmaceutically acceptable salts or solvate thereof, wherein:
R¹ represents -C₁₋₃ alkyl-C₃₋₈ cycloalkyl or -C₃₋₇ cycloalkyl;
X represents aryl, heteroaryl or heterocyclyl;
R² represents hydrogen, halogen, -Y-C₁₋₆alkyl, Y-aryl, -Y-heterocyclyl, or -Y-heteroaryl; Y represents a bond or CO;
wherein said alkyl, aryl, heteroaryl and heterocyclyl groups of R² may be optionally substituted by 1, 2 or 3 substituents which may be the same or different, and which are selected from the group consisting of cyano, =O, R⁵, -COR⁵, -CO₂R⁵, and -CONR⁵R⁶ (wherein R⁵ and R⁶ independently represent hydrogen or -C₁₋₆alkyl, and wherein R⁵ and R⁶ may optionally be further substituted by 1, 2 or 3 halogen atoms);
provided that where R¹ represents -C₁₋₃ alkyl-C₃₋₈ cycloalkyl, the alkyl, aryl, heteroaryl and heterocyclyl groups of R² may not be substituted with -CO₂R⁵, and provided that the aryl and heteroaryl groups of R² may only be substituted by =O if the substituted group is aromatic.

In a most particular aspect, the invention provides compounds of formula (I) or pharmaceutically acceptable salts or solvates thereof, wherein:
R¹ represents -C₁₋₃ alkyl-C₃₋₈ cycloalkyl or -C₃₋₇ cycloalkyl;
X represents aryl, heteroaryl or heterocyclyl;
R² represents hydrogen, halogen, -Y-C₁₋₆alkyl, Y-aryl, -Y-heterocyclyl, or -Y-heteroaryl; Y represents a bond or CO;
wherein said alkyl, aryl, heteroaryl and heterocyclyl groups of R² may be optionally substituted by 1, 2 or 3 substituents which may be the same or different, and which are selected from the group consisting of cyano, =O, R⁵, -COR⁵ and -CONR⁵R⁶ (wherein R⁵ and R⁶ independently represent hydrogen or -C₁₋₆alkyl, and wherein R⁵ and R⁶ may optionally be further substituted by 1, 2 or 3 halogen atoms), provided that the aryl and heteroaryl groups of R² may only be substituted by =O if the substituted group is aromatic.

Compounds according to the invention include the compounds of examples E1 to E116 as shown below, or pharmaceutically acceptable salts or solvates thereof.

In a more particular aspect, compounds according to the invention include:
1-[4-(6-cyclobutyl-5,6,7,8-tetrahydro-4*H*-[1,3]thiazolo[4,5-*d*]azepin-2-yl) phenyl]-2-pyrrolidinone;
6-cyclobutyl-2-{1-[(6-methyl-2-pyridinyl)carbonyl]-4-piperidinyl}-5,6,7,8-tetrahydro-4*H*-[1,3]thiazolo[4,5-*d*]azepine;
6-cyclobutyl-2-{1-[(5-methyl-3-pyridinyl)carbonyl]-4-piperidinyl}-5,6,7,8-tetrahydro-4*H*-[1,3]thiazolo[4,5-*d*]azepine;
5-[4-(6-cyclobutyl-5,6,7,8-tetrahydro-4*H*-[1,3]thiazolo[4,5-*d*]azepin-2-yl)-1-piperidinyl]-2-pyridinecarbonitrile;
5-[4-(6-Cyclobutyl-5,6,7,8-tetrahydro-4*H*-[1,3]thiazolo[4,5-*d*]azepin-2-yl)-1-piperidinyl]-*N*,*N-*dimethyl-2-pyridinecarboxamide;
1-[4-(6-Cyclobutyl-5,6,7,8-tetrahydro-4*H*-[1,3]thiazolo[4,5-*d*]azepin-2-yl)phenyl]-3-methyl-2-imidazolidinone;
1-[4-(6-Cyclobutyl-5,6,7,8-tetrahydro-4*H*-[1,3]thiazolo[4,5-*d*]azepin-2-yl)phenyl]-2-imidazolidinone;
(+)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile;
(±)-6-Cyclobutyl-2-[1-(6-methyl-3-pyridinyl)-3-pyrrolidinyl]-5,6,7,8-tetrahydro-4*H-*[1,3]thiazolo[4,5-*d*]azepine;
or pharmaceutically acceptable salts or solvates thereof.

Because of their potential use in medicine, the salts of the compounds of formula (I) are preferably pharmaceutically acceptable.

A pharmaceutically acceptable acid addition salt can be formed by reaction of a compound of formula (I) with a suitable inorganic or organic acid (such as hydrobromic, hydrochloric, sulfuric, nitric, phosphoric, succinic, maleic, formic, acetic, propionic, fumaric, citric, tartaric, lactic, benzoic, salicylic, glutamaic, aspartic, p-toluenesulfonic, benzenesulfonic, methanesulfonic, ethanesulfonic, naphthalenesulfonic such as 2-naphthalenesulfonic, or hexanoic acid), optionally in a suitable solvent such as an organic solvent, to give the salt which is usually isolated for example by crystallisation and filtration or by evaporation. A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salt.

Free base compounds may be converted into the corresponding hydrochloride salts by treatment in methanol with a solution of hydrogen chloride in diethyl ether followed by evaporation of solvents.

The invention includes within its scope all possible stoichiometric and non-stoichiometric forms of the salts of the compounds of formula (I) including hydrates and solvates.

Certain compounds of formula (I) are capable of existing in stereoisomeric forms. It will be understood that the invention encompasses all geometric and optical isomers of these compounds and the mixtures thereof including racemates. Tautomers also form an aspect of the invention.

The present invention also provides a process for the preparation of a compound of formula (I) or a pharmaceutically acceptable salt or solvate thereof, which process comprises:
(a) reacting a compound of formula (II) 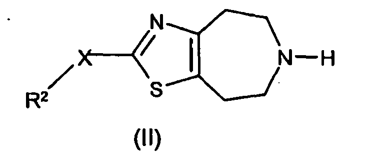 wherein X and R² are as defined above, with a compound of formula R¹'=O, wherein R¹' is =C₂₋₆ alkyl, =C₁₋₃ alkyl-C₃₋₈ cycloalkyl or =C₃₋₇ cycloalkyl, wherein the cycloalkyl groups may be optionally substituted by C₁₋₃ alkyl; or
(b) reacting a compound of formula (III) 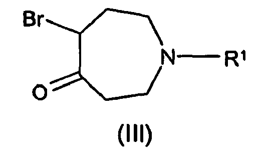 wherein R¹ is as defined above with a compound of formula R²X-C(=S)NH₂ wherein R² and X are as defined above; or
(c) deprotecting a compound of formula (I) which is protected;
(d) interconversion from one compound of formula (I) to another;
(e) reacting a compound of formula (X) 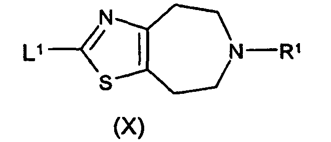 wherein R¹ is as defined above and L¹ represents a leaving group such as a halogen (e.g. iodine), with an organometallic compound of formula (XI), R²-X-L², wherein R² and X are as defined above and wherein L² is a leaving group (e.g. trimethylstannanyl); or
(f) reacting a compound of formula (II) 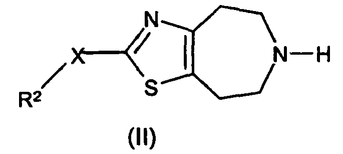 wherein X and R² are as defined above, with a compound of formula R¹-L³, wherein R¹ is as defined above, and wherein L³ is a leaving group such as a halogen (e.g iodine). Process (a) typically comprises the use of reductive conditions (such as treatment with a borohydride e.g. sodium triacetoxyborohydride), optionally in the presence of an acid, such as acetic acid, in an appropriate solvent such as dichloromethane at a suitable temperature such as room temperature.

Processes (b) may typically be performed in a suitable solvent, such as ethanol or propanol, at an appropriate temperature, for example under reflux.

In process (c), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid in dioxan or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (d) may be performed using conventional interconversion procedures such as epimerisation, oxidation, reduction, alkylation, decarboxylation, nucleophilic or electrophilic aromatic substitution, activation of an amine via nucleophilic substitution, ester hydrolysis, hydrolysis of a cyano group, amide bond formation or transition metal mediated coupling reactions. Examples of transition metal mediated coupling reactions useful as interconversion procedures include the following: Palladium catalysed coupling reactions between organic electrophiles, such as aryl halides, and organometallic reagents, for example boronic acids (Suzuki cross-coupling reactions); Palladium catalysed amination and amidation reactions between organic electrophiles, such as aryl halides, and nucleophiles, such as amines and amides; Copper catalysed amidation reactions between organic electrophiles (such as aryl halides) and nucleophiles such as amides; and Copper mediated coupling reactions between phenols and boronic acids.

Process (e) typically requires palladium catalysis (e.g. bis(triphenylphosphine) palladium (II) chloride optionally in the presence of a base (e.g. sodium carbonate) in a suitable solvent such as dioxane, at a suitable temperature, such as reflux.

Process (f) typically takes place in the presence of a base (e.g. potassium carbonate) in a suitable solvent (e.g. ethanol) at a suitable temperature, such as reflux).

Compounds of formula (II) and (X) may be prepared in accordance with the following scheme 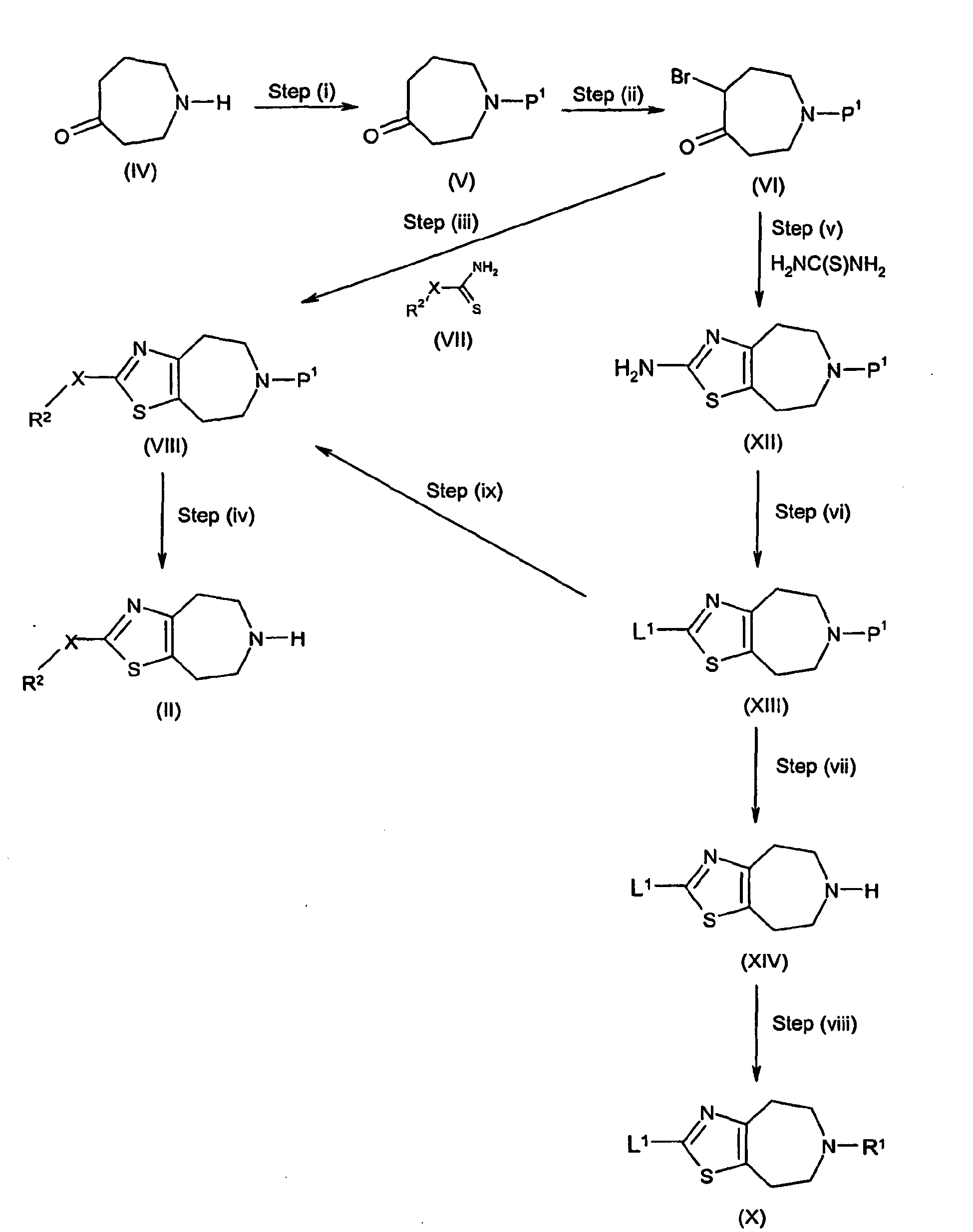 wherein R¹, R², X and L¹ are defined above and P¹ represents a suitable protecting group such as trifluoroacetate.

Step (i) typically comprises a suitable amine protection reaction. Suitable protecting groups are described above for process (c). Where P¹ represents trifluoroacetate, step (i) typically comprises reaction with trifluoroacetic anhydride in the presence of a base such as triethylamine in a suitable solvent such as dichloromethane at a suitable temperature, such as between -5°C and room temperature.

Step (ii) is a bromination reaction and may be performed using bromine in a suitable solvent such as acetic acid, at a suitable temperature, for example, room temperature or with heating at 60°C.

Step (iii) is a cyclisation reaction and may be preformed in a suitable solvent such as ethanol or propanol, at a suitable temperature, for example, under reflux.

Step (iv) comprises deprotection reaction and can be performed according to process (c). Where P¹ represents trifluoroacetate, step (iv) typically comprises treatment with a base such as potassium carbonate in a suitable solvent such as methanol at a suitable temperature, such as room temperature.

Step (v) is a cyclisation reaction with thiourea and may be preformed in a suitable solvent such as ethanol or propanol, at a suitable temperature, for example, under reflux.

When L¹ represents iodine, step (vi) typically involves reaction with sodium nitrite and potassium iodide in the presence of acid (e.g sulphuric acid) in a suitable solvent such as water, at a suitable temperature such as 10°C.

Step (vii) comprises deprotection reaction and can be performed according to process (c). Where P¹ represents trifluoroacetate, step (vii) typically comprises treatment with a base such as potassium carbonate in a suitable solvent such as methanol at a suitable temperature, such as room temperature.

Step (viii) may be performed under reducing conditions in an analogous manner to that described for process (a) above.

As indicated above (see step ix), compounds of formula (VIII) may be prepared from compounds of formula (XIII) using a coupling reaction as described in process (e).

Compounds of formula (III) may be prepared in accordance with the following scheme: 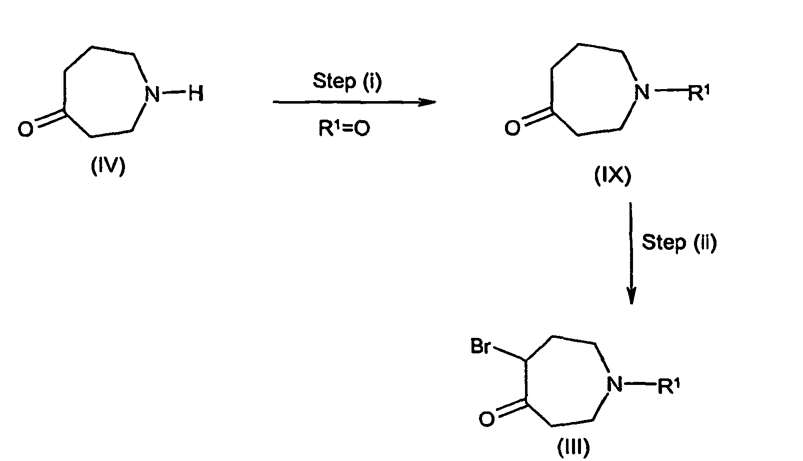 wherein R¹ is as defined above.

Step (i) may be performed under reducing conditions in an analogous manner to that described for process (a) above.

Step (ii) is a bromination reaction and may be performed in an analogous manner to step (ii) above.

The compound of formula (IV) may be prepared according to J. Heterocycl. Chem, 1992, 29, 4, 779-786.

Compounds of formula (VII) are either commercially available or readily prepared using established literature methods, for example, conditions described in Synthesis, 1992, 1219.

Compounds of formula (XI) may be prepared by reaction of a compound of formula R²-X-_{L} wherein L³ is a leaving group such as a halogen (e.g. bromine or iodine) with an appropriate tin compound in the presence of a palladium catalyst (e.g. tetrakis(triphenylphosphine)palladium (0)) in a suitable solvent such as toluene at a suitable temperature such as reflux.

Compounds of formula (I) and their pharmaceutically acceptable salts have affinity for and are antagonists and/or inverse agonists of the histamine H3 receptor and are believed to be of potential use in the treatment of neurological diseases including Alzheimer's disease, dementia (including Lewy body dementia and vascular dementia), age-related memory dysfunction, mild cognitive impairment, cognitive deficit, epilepsy, pain of neuropathic origin including neuralgias, neuritis and back pain, and inflammatory pain including osteoarthritis, rheumatoid arthritis, acute inflammatory pain and back pain, migraine, Parkinson's disease, multiple sclerosis, stroke and sleep disorders (including narcolepsy and sleep deficits associated with Parkinson's disease); psychiatric disorders including schizophrenia (particularly cognitive deficit of schizophrenia), attention deficit hypereactivity disorder, depression, anxiety and addiction; and other diseases including obesity and gastro-intestinal disorders.

It will also be appreciated that compounds of formula (I) are expected to be selective for the histamine H3 receptor over other histamine receptor subtypes, such as the histamine H1 receptor. Generally, compounds of the invention may be at least 10 fold selective for H3 over H1, such as at least 100 fold selective.

Thus the invention also provides a compound of formula (I) or a pharmaceutically acceptable salts or solvates thereof, for use as a therapeutic substance in the treatment or prophylaxis of the above disorders, in particular cognitive impairments in diseases such as Alzheimer's disease and related neurodegenerative disorders.

The compounds of the invention can be used in a method of treatment or prophylaxis of the above disorders, in mammals including humans, which comprises administering to the sufferer a therapeutically effective amount of a compound of formula (I) or a pharmaceutically acceptable salt or solvate thereof.

In another aspect, the invention provides the use of a compound of formula (I) or a pharmaceutically acceptable salt or solvate thereof in the manufacture of a medicament for use in the above disorders.

When used in therapy, the compounds of formula (I) are usually formulated in a standard pharmaceutical composition. Such compositions can be prepared using standard procedures.

Thus, the present invention further provides a pharmaceutical composition for use in the treatment of the above disorders which comprises the compound of formula (I) or a pharmaceutically acceptable salt or solvate thereof and a pharmaceutically acceptable carrier.

The present invention further provides a pharmaceutical composition which comprises the compound of formula (I) or a pharmaceutically acceptable salt or solvate thereof and a pharmaceutically acceptable carrier.

Compounds of formula (I) may be used in combination with other therapeutic agents, for example medicaments claimed to be useful as either disease modifying or symptomatic treatments of Alzheimer's disease. Suitable examples of such other therapeutic agents may be agents known to modify cholinergic transmission such as 5-HT₆ antagonists, M1 muscarinic agonists; M2 muscarinic antagonists or acetylcholinesterase inhibitors. When the compounds are used in combination with other therapeutic agents, the compounds may be administered either sequentially or simultaneously by any convenient route.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.1 to 200 mg and even more suitably 1.0 to 200 mg, and such unit doses may be administered more than once a day, for example two or three a day. Such therapy may extend for a number of weeks or months.

The following Descriptions and Examples illustrate the preparation of compounds of the invention. Where indicated, Mass Directed Auto-Purification or MDAP was carried out using a Supelco LCABZ++ column (20mm x 100mm). The stationary phase particle size is 5 µm. The solvent systems used comprised solvent A (water + 0.1 % formic acid) and solvent B (acetonitrile:water 95:5 + 0.05% formic acid). Compounds were eluted with gradients of solvent B in solvent A.

### Description 1

### 1-Cyclobutylhexahydro-4H-azepin-4-one (D1)

### Method A

To a suspension of the hydrochloride salt of hexahydro-4*H*-azepin-4-one (3 g, 20.1 mmol) (may be prepared as described in J. Heterocycl. Chem, 1992, 29, 4, 779-786) in dichloromethane (75 ml) was added cyclobutanone (15 ml, 201 mmol), followed by triethylamine (2.80 ml, 20.1 mmol). The resulting mixture was allowed to stir at room temperature for 18 hours. After this time sodium triacetoxyborohydride (6.38 g, 30.2 mmol) was added and stirring continued for a further three hours. The reaction was quenched using 1 N NaOH solution (200 ml) and the mixture extracted with dichloromethane (3 x 150 ml). The combined extracts were dried over magnesium sulfate, concentrated and purified on silica gel eluting with a mixture of 2M ammonia/methanol solution and dichloromethane over a gradient (0-4% 2M ammonia/methanol) to give the title compound; (3.06 g); ¹H NMR(CDCl₃) 2.94-2.86 (1H, m), 2.63-2.51 (8H, m), 2.09-2.02 (2H, m), 1.88-1.78 (4H, m), 1.72-1.56 (2H, m).

### Method B

The hydrochloride salt of hexahydro-4*H*-azepin-4-one (3 g, 20.1mmol) (may be prepared as described in J. Heterocycl. Chem, 1992, 29, 4, 779-786) was suspended in dichloromethane (40ml), treated with triethylamine (2.80ml, 20.1mmol) and cyclobutanone (15ml, 201 mmol). The resulting mixture was allowed to stir at room temperature under argon for 18 hours. The mixture was cooled in an ice bath and sodium triacetoxyborohydride (6.39g, 30.2mmol) was added portionwise and the mixture stirred for 5 minutes. The mixture was allowed to warm to room temperature and stirred for 2 hours. The mixture was cooled in an ice bath and the reaction was quenched by portionwise addition of 2N NaOH solution (50ml). The mixture was allowed to warm to room temperature and extracted with dichloromethane (x 3). The combined extracts were dried over magnesium sulfate, evaporated and purified on silica gel eluting with dichloromethane followed by a mixture of 2M ammonia/methanol solution and dichloromethane (2:98) to give the title compound (D1); ¹H NMR(CDCl₃) 2.94-2.86 (1H, m), 2.63-2.51 (8H, m), 2.09-2.02 (2H, m), 1.88-1.78 (4H, m), 1.72-1.56 (2H, m).

### Description 2

### 5-Bromo-1-cyclobutylhexahydro-4H-azepin-4-one (D2)

A mixture of 1-cyclobutylhexahydro-4*H*-azepin-4-one (may be prepared as described in Description 1) (0.2g, 1.2mmol) and bromine (0.061 ml, 1.2mmol) in acetic acid was stirred at room temperature for 8 hours. The mixture was reduced in vacuo and the crude product (D2) may be used directly without further purification.

### Description 3

### 1-(Trifluoroacetyl)hexahydro-4H-azepin-4-one (D3)

Trifluoroacetic anhydride was added dropwise to a suspension of the hydrochloride salt of hexahydro-4*H*-azepin-4-one (20g, 0.134mol) (may be prepared as described in J. Heterocycl. Chem, 1992, 29, 4, 779-786) and triethylamine (18.6ml, 0.268mol) in dichloromethane (75 ml) cooled in ice/methanol at such a rate to keep the internal temperature below -5°C. After complete addition the mixture was allowed to warm to room temperature and stirred for 18 hours. The mixture was washed with water (2x100ml), saturated sodium bicarbonate solution (2x100ml), brine (2x100ml) and citric acid (100ml), dried over sodium sulphate and evaporated to afford the title compound (D3); ¹H NMR (CDCl₃) δ 3.86-3.74 (4H, m), 2.76-2.69 (4H, m), 1.96-1.88 (2H,m).

### Description 4

### 6-(Trifluoroacetyl)-5,6,7,8-tetrahydro-4H-[1,3]thiazolo[4,5-d]azepin-2-amine (D4)

A solution of bromine (5.2ml, 0.102mol) in acetic acid (120ml) was added slowly to a solution of 1-(trifluoroacetyl)hexahydro-4*H-*azepin-4-one (may be prepared as described in Description 3) (21.37g, 0.102mol) in acetic acid (120ml) at such a rate as to have decolourised the solution before the next addition. On completion of addition the mixture was evaporated to give a yellow mobile oil, which was dissolved in ethanol (200ml), treated with thiourea (7.76g, 0,102mol) and heated at reflux for 18 hours. The solvent was removed by evaporation and the residue dissolved in water (250ml). The mixture was basified using a saturated sodium bicarbonate solution and extracted with dichloromethane (6x100ml). The combined extracts were dried over sodium sulphate and evaporated to give a cream solid which was triturated with diethyl ether and filtered to afford the title compound (D4); MS (ES+) m/e 266 [M+H]⁺.

### Description 5

### 2-Iodo-6-(trifluoroacetyl)-5,6,7,8-tetrahydro-4H-[1,3]thiazolo[4,5-d]azepine (D5)

6-(Trifluoroacetyl)-5,6,7,8-tetrahydro-4*H*-[1,3]thiazolo[4,5-d]azepin-2-amine (may be prepared as described in Description 4) (4.79 g, 18.1 mmol) was suspended in water (50 ml), cooled in an ice bath and treated with concentrated sulfuric acid (25 ml) dropwise. The resulting mixture was cooled in an ice/methanol bath and a solution of sodium nitrite (1.25 g, 18.1 mmol) in water was added dropwise. The resulting mixture was stirred at10°C for 25 minutes and a solution of potassium iodide (4.5 g, 27.2 mmol) in water (25 ml) was added dropwise. The resulting brown slurry was allowed to warm to room temperature and stirred for 30 minutes. The reaction mixture was poured cautiously into saturated sodium bicarbonate solution (800 ml). This solution was extracted with dichloromethane (x 4). The dichloromethane extracts were combined, dried under magnesium sulfate and evaporated *in vacuo.* The residue was purified by column chromatography eluting with dichloromethane followed by ethyl acetate/dichloromethane (5:95) to afford the title product (D5). MS (AP+) m/e 377 [M+H]⁺.

### Description 6

### 2-(4-Bromophenyl)-6-(trifluoroacetyl)-5,6,7,8-tetrahydro-4H-[1,3]thiazolo[4,5-d]azepine (D6)

2-Iodo-6-(trifluoroacetyl)-5,6,7,8-tetrahydro-4*H*-[1,3]thiazolo[4,5-*d*]azepine (may be prepared as described in Description 5) (100 mg, 0.27 mmol), (4-bromophenyl)boronic acid (59 mg, 0.30 mmol), bis(triphenylphosphine)palladium (II) chloride (19 mg, 0.03 mmol) and sodium carbonate (63 mg, 0.59 mmol) were added together in toluene (4 ml) and water (1 ml) and the resulting mixture was heated under reflux under argon for 18 hours. A further quantity of (4-bromophenyl)boronic acid (59 mg, 0.30 mmol) and bis(triphenylphosphine)palladium (II) chloride (19 mg, 0.03 mmol) were added and the resulting mixture was heated under reflux for 2 hours. The reaction mixture was allowed to cool to room temperature, diluted with water and ethyl acetate and extracted with ethyl acetate (x 2). The ethyl acetate layers were combined, dried under magnesium sulfate and evaporated *in vacuo.* The residue was purified by column chromatography eluting with ethyl acetate/pentane (1:9) to afford the title product (D6). MS (AP+) m/e 407 [M+2H]⁺.

### Description 7

### 1-Methyl-3-{4-[6-(trifluoroacetyl)-5,6,7,8-tetrahydro-4H-[1,3]thiazolo[4,5-d]azepin-2-yl]phenyl}-2-imidazolidinone (D7)

2-(4-Bromophenyl)-6-(trifluoroacetyl)-5,6,7,8-tetrahydro-4*H*-[1,3]thiazolo[4,5-*d*]azepine (may be prepared as described in Description 6) (50 mg, 0.12 mmol), 1-methyl-2-imidazolidinone (24 mg, 0.24 mmol), tris(dibenzylideneacetone)dipalladium (0) (6 mg, 0.006 mmol), 9,9-dimethyl-4,5-bis(diphenylphosphino)xanthene (10 mg, 0.018 mmol) and caesium carbonate (59 mg, 0.18 mmol) were added together in dioxane (2 ml) and the resulting mixture was heated under reflux under argon for 4 hours. The reaction mixture was allowed to cool to room temperature, diluted with water and extracted with ethyl acetate (x 2). The ethyl acetate layers were combined, dried under magnesium sulfate and evaporated *in vacuo.* The residue was purified by column chromatography eluting with ethyl acetate/pentane (4:1) to afford the title product (D7). MS (AP+) m/e 425 [M+H]⁺.

### Description 8

### 1-Methyl-3-[4-(5,6,7,8-tetrahydro-4H-[1,3]thiazolo[4,5-d]azepin-2-yl)phenyl]-2-imidazolidinone (D8)

1-Methyl-3-{4-[6-(trifluoroacetyl)-5,6,7,8-tetrahydro-4*H*-[1,3]thiazolo[4,5-*d*]azepin-2-yl]phenyl}-2-imidazolidinone (may be prepared as described in Description 7) (32 mg, 0.08 mmol) was suspended in methanol (1 ml), treated with potassium carbonate (55 mg) and water (1 ml) and stirred at room temperature for 2 hours. The reaction mixture was diluted with methanol and passed down an SCX column eluting with methanol and 2M ammonia/methanol. The basic fractions were combined and evaporated *in vacuo* to afford the title product (D8). MS (AP+) m/e 329 [M+H]⁺.

### Description 9

### 1,1-Dimethylethyl 3-(aminocarbonothioyl)-1-pyrrolidinecarboxylate (D9)

To a solution of 1,1-dimethylethyl 3-cyano-1-pyrrolidinecarboxylate (5.00g, 25.5mmol) in 1,3-dimethyl-2-imidazolidinone (50ml) was added hexamethyldisilathiane (12.7g, 71.4mmol) and a 30% solution of sodium methoxide in methanol (9.70ml) simultaneously dropwise. The resulting blue/green mixture was allowed to stir at room temperature overnight, poured into water (100ml) and extracted with ethyl acetate (3x100ml). The combined extracts were washed with water, dried over magnesium sulphate and evaporated. The crude mixture was purified using silica gel chromatography to afford the product (D9); ¹H NMR (CDCl₃) δ 1.46 (H, s), 2.12-2.25 (2H, m), 3.21-3.42 (2H,m), 3.55-3.74 (3H,m), 7.04 (1 H,br s), 7.48 (1H, br s).

### Description 10

### 4-(5,6,7,8-Tetrahydro-4H-[1,3]thiazolo[4,5-d]azepin-2-yl)benzonitrile (D10)

2-Iodo-6-(trifluoroacetyl)-5,6,7,8-tetrahydro-4*H*-[1,3]thiazolo[4,5-*d*]azepine (may be prepared as described in Description 5) (0.065g, 0.17mmol), (4-cyanophenyl)boronic acid (0.03g, 0.21mmol), bis(triphenylphosphine)palladium (II) chloride (12mg, 0.017 mmol) and sodium carbonate (0.043g, 0.41 mmol) were added together in toluene (2 ml) and water (0.5 ml) and the resulting mixture was heated under reflux for 18 hours. The reaction mixture was allowed to cool to room temperature, diluted with water and methanol and then applied to an ion exchange cartridge (SCX) and washed with methanol and then a 2M ammonia in methanol solution. The basic fractions were then reduced and purified by column chromatography eluting with a mixture of 2M ammonia/methanol and dichloromethane (5:95) to afford the product (D10); MS (ES+) m/e 256 [M+H]⁺.

### Description 11

### 2-Iodo-5,6,7,8-tetrahydro-4H-[1,3]thiazolo[4,5-d]azepine (D11)

### Method A

2-Iodo-6-(trifluoroacetyl)-5,6,7,8-tetrahydro-4*H*-[1,3]thiazolo[4,5-*d*]azepine (may be prepared as described in Description 5) (350mg, 0.93mmol) and potassium carbonate (642mg, 4.65mmol) were added together in methanol (3ml) and water (3ml) and the resulting mixture was stirred at room temperature for 2 hours. The mixture was diluted with methanol and applied to an ion exchange cartridge (SCX) and washed with methanol and then a 2M ammonia in methanol solution. The basic fractions were combined and evaporated to afford the product (D11); MS (ES+) m/e 281 [M+H]⁺.

### Method B

To a suspension of 2-iodo-6-(trifluoroacetyl)-5,6,7,8-tetrahydro-4H-[1,3]thiazolo[4,5-d]azepine (may be prepared as described in Description 5) (300mg, 0.8mmol) in methanol-water (20mL-10mL) was added potassium carbonate (55 mg, 4.0mmol) and the resulting mixture was stirred at room temperature for 3 hours. After this time the reaction was acidify with 2M hydrochloric acid and applied to an ion exchange cartridge (SCX), washed with methanol and then a 2M ammonia in methanol solution. The basic fractions were then evaporated *in vacuo* to afford the product (D11); MS (ES+) m/e 281 [M+H]⁺.

### Description 12

### 6-Cyclobutyl-2-iodo-5,6,7,8-tetrahydro-4H-[1,3]thiazolo[4,5-d]azepine (D12)

### Method A

2-Iodo-5,6,7,8-tetrahydro-4*H*-[1,3]thiazolo[4,5-*d*]azepine (may be prepared as described in Description 11) (245mg, 0.88mmol) was dissolved in dichloromethane (5ml), treated with acetic acid (2 drops) and cyclobutanone (0.13ml, 1.76mmol). The mixture was stirred for 15 minutes. Sodium triacetoxyborohydride (373mg, 1.76mmol) was added and the mixture stirred at room temperature under argon for 1 hour. The mixture was diluted with methanol and applied to an ion exchange cartridge (SCX) and washed with methanol and then a 2M ammonia in methanol solution. The basic fractions were combined and evaporated to afford the product (D12); MS (ES+) m/e 335 [M+H]⁺.

### Method B

To a suspension of 2-iodo-5,6,7,8-tetrahydro-4H-[1,3]thiazolo[4,5-d]azepine (may be prepared as described in Description 11, method B) (180mg, 0.64mmol) in dichloromethane (7mL) was added 3 drops of acetic acid, cyclobutanone (72 uL, 0.96mmol), Sodium triacetoxyborohydride (203mg, 0.96mmol). The resulting mixture was allowed to stir at room temperature for 0.5 hour. Reaction mixture was acidified with 2M Hydrochloric acid and applied to an ion exchange cartridge (SCX),washed with methanol and then a 2M ammonia in methanol solution. The basic fractions were then evaporated *in vacuo* to afford the product (D12); MS (ES+) m/e 334 [M+H]⁺.

### Description 13

### 5-(Trimethylstannanyl)-2-pyridinecarbonitrile (D13)

### 5-Bromo-2-pyridinecarbonitrile (500mg, 2.73mmol),

tetrakis(triphenylphosphine)palladium (0) (158mg, 0.14mmol) and tetrabutylammonium iodide (1.11g, 3.00mmol) were added together in toluene (10ml), treated with a solution of hexamethylditin (985mg, 3.00mmol) in toluene and the resulting mixture was heated under reflux under argon for 30 minutes. The mixture was allowed to cool to room temperature. The solvent was removed under reduced pressure and the residue was purified by column chromatography eluting with a mixture of ethyl acetate/pentane (1:9). The product was further purified by column chromatography eluting with dichloromethane (100%) to afford the product (D13); MS (ES+) m/e 268 [M+H]⁺.

### Description 14

### 2-Chloro-5-(trimethylstannanyl)pyridine (D14)

A mixture of 2-chloro-5-iodopyridine (1.00g, 4.18mmol), hexamethylditin (1.94g, 5.92mmol) and tetrakis(triphenylphosphine)palladium(0) (0.49g, 0.42mmol) in toluene (10ml) was heated at reflux under argon for 3 hours. The mixture was allowed to cool to room temperature and filtered. The filtrate was evaporated and purified using silica gel chromatography, eluting with pentane to afford the product (D14); MS (ES+) m/e 277 [M+H]⁺.

### Description 15

### 2-Chloro-5-(tributylstannanyl)pyridine (D15)

To a solution of hexabutylditin (5.20ml, 10.4mmol) in tetrahydrofuran (30ml) at 0 °C was added n-butyllithium (1.6M solution in hexanes) (6.5ml, 10.4mmol). The resulting yellow solution was allowed to stir at room temperature for 15 minutes. After this time a solution of 2-chloro-5-bromopyridine (1.00g, 5.20mmol) in tetrahydrofuran (10ml) was added and the resulting yellow/brown solution allowed to warm to room temperature over 4 hours. The mixture was evaporated and purified by column chromatography eluting with pentane to afford the product (D15); MS (ES+) m/e 402 & 404 [M+H]⁺.

### Description 16

### 2-(6-Chloro-3-pyridinyl)-6-(trifluoroacetyl)-5,6,7,8-tetrahydro-4H-[1,3]thiazolo[4,5-d]azepine (D16)

### Method A

2-Iodo-6-(trifluoroecetyl)-5,6,7,8-tetrahydro-4*H*-[1,3]thiazolo[4,5-*d*]azepine (may be prepared as described in Description 5) (100mg, 0.27mmol), 2-chloro-5-(trimethylstannanyl)pyridine (may be prepared as described in Description 14) (97mg, 0.35mmol) and tetrakis(triphenylphosphine)palladium (0) (19mg, 0.016mmol) were added together in toluene (2ml) and the resulting mixture was heated under reflux under argon for 6 hours. Tetrakis(triphenylphosphine)palladium (0) (19mg, 0.016mmol) was added and the mixture was heated under reflux under argon for 18 hours. The mixture was allowed to cool to room temperature and the solvent removed under reduced pressure. The residue was purified by column chromatography eluting with a mixture of ethyl acetate/pentane (1:4 to 1:1) to afford the product (D16); MS (ES+) m/e 362 [M+H]⁺.

### Method B

2-Iodo-6-(trifluoroacetyl)-5,6,7,8-tetrahydro-4*H*-[1,3]thiazolo[4,5-*d*]azepine (may be prepared as described in Description 5) (200mg, 0.53mmol), 2-chloro-5-(trimethylstannanyl)pyridine (may be prepared as described in Description 14) (190mg, 0.69mmol) and tetrakis(triphenylphosphine)palladium (0) (73mg, 0.06mmol) were added together in toluene (5ml) and the resulting mixture was heated under reflux under argon for 18 hours. The mixture was allowed to cool to room temperature and the solvent removed under reduced pressure. The residue was purified by column chromatography eluting with a mixture of ethyl acetate/pentane (1:4) to afford the product (D16); MS (ES+) m/e 362 [M+H]⁺.

### Method C

2-Iodo-6-(trifluoroacetyl)-5,6,7,8-tetrahydro-4*H*-[1,3]thiazolo[4,5-*d*]azepine (may be prepared as described in Description 5) (200mg, 0.53mmol), 2-chloro-5-(trimethylstannanyl)pyridine (may be prepared as described in Description 14) (191mg, 0.69mmol) and tetrakis(triphenylphosphine)palladium(0) (74.0mg, 0.064mmol) in toluene (4ml) was heated at reflux under argon overnight. A further portion of tetrakis(triphenylphosphine)palladium(0) (37.0mg, 0.032mmol) was added and the mixture heated at reflux for 2.5 hours. The reaction mixture was allowed to cool to room temperature, evaporated and purified using silica gel chromatography, eluting with a mixture of ethyl acetate and pentane (25-50%) to afford the product (D16); MS (ES+) m/e 362 [M+H]⁺.

### Method D

2-Iodo-6-(trifluoroacetyl)-5,6,7,8-tetrahydro-4*H*-[1,3]thiazolo[4,5-*d*]azepine (may be prepared as described in Description 5) (150mg, 0.40mmol), 2-chloro-5-(tributylstannanyl)pyridine (may be prepared as described in Description 15) (357mg, 0.89mmol) and bis(triphenylphosphine)palladium(II) chloride (28.0mg, 0.04mmol) in toluene (10ml) was heated at reflux for 2.5 hours and then over weekend. The reaction mixture was allowed to cool to room temperature, evaporated and purified using silica gel chromatography, eluting with a mixture of ethyl acetate and pentane (0-100%) to afford the product (D16); MS (ES+) m/e 362 [M+H]⁺.

### Method E

A solution of 2-Iodo-6-(trifluoroacetyl)-5,6,7,8-tetrahydro-4*H*-[1,3]thiazolo[4,5-*d*]azepine (may be prepared as described in Description 5) (250mg 0.665mmol), 2-chloro-5-(trimethylstannanyl)pyridine (maybe prepared as described Description 14) (239mg, 0.865mmol) and tetrakis(triphenylphosphine)palladium(0) (46mg, 0.04mmol) in toluene (2ml) was heated under reflux under argon for 3 hours. A further quantity of (tetrakis(triphenylphosphine)palladium(0) (46mg, 0.04mmol) was added and the resulting mixture was heated under reflux under argon overnight. The reaction was allowed to cool down and purified using silica gel chromatography to afford the product (D16); MS (ES+) m/e 362 [M+H]+.

### Method F

A solution of 2-Iodo-6-(trifluoroacetyl)-5,6,7,8-tetrahydro-4*H*-[1,3]thiazolo(4,5-*d*]azepine (maybe prepared as described in Description 5) (120mg 0.319mmol), 2-chloro-5-(tributylstannanyl)pyridine (maybe prepared as described in Description 15) (160mg, 0.415mmol) and tetrakis(triphenylphosphine)palladium(0) (22mg, 6mol%) in 2ml of toluene was heated under reflux under argon for 3 hours. A further quantity of (tetrakis(triphenylphosphine)palladium(0) (22mg, 6mol%) was added and the resulting mixture was heated under reflux under argon overnight. The reaction was allowed to cool down and purified using silica gel chromatography to afford the product (D16); MS (ES+) m/e 362 [M+H]+.

### Method G

2-Iodo-6-(trifluoroacetyl)-5,6,7,8-tetrahydro-4*H*-[1,3]thiazolo[4,5-*d*]azepine (may be prepared as described in Description 5) (200mg, 0.53mmol), 2-chloro-5-(trimethylstannanyl)pyridine (may be prepared as described in Description 14) (191mg, 0.69mmol) and tetrakis(triphenylphosphine)palladium(0) (74.0mg, 0.064mmol) in toluene (4ml) was heated at reflux under argon overnight. A further portion of tetrakis(triphenylphosphine)palladium(0) (37.0mg, 0.032mmol) was added and the mixture heated at reflux for 2.5 hours. The reaction mixture was allowed to cool to room temperature, evaporated and purified using silica gel chromatography, eluting with a mixture of ethyl acetate and pentane (25-50%) to afford the product (D16); MS (ES+) m/e 362 [M+H]⁺.

### Method H

2-Iodo-6-(trifluoroacetyl)-5,6,7,8-tetrahydro-4*H*-[1,3]thiazolo[4,5-*d*]azepine (may be prepared as described in Description 5) (150mg, 0.40mmol), 2-chloro-5-(tributylstannanyl)pyridine (may be prepared as described in Description 15) (357mg, 0.89mmol) and bis(triphenylphosphine)palladium(II) chloride (28.0mg, 0.04mmol) in toluene (10ml) was heated at reflux for 2.5 hours and then over weekend. The reaction mixture was allowed to cool to room temperature, evaporated and purified using silica gel chromatography, eluting with a mixture of ethyl acetate and pentane (0-100%) to afford the product (D16); MS (ES+) m/e 362 [M+H]⁺.

### Description 17

### 1-{5-[6-(Trifluoroacetyl)-5,6,7,8,tetrahydro-4H-[1,3]thiazolo[4,5-d]azepin-2-yl]-2pyridinyl}-2-pyrrolidinone (D17)

2-(6-Chloro-3-pyridinyl)-6-(trifluoroacetyl)-5,6,7,8-tetrahydro-4*H*-[1,3]thiazolo[4,5-*d*]azepine (may be prepared as described in Description 16, method A) (51 mg, 0.14mmol), 2-pyrrolidinone (0.02ml, 0.28mmol), tris(dibenzylideneacetone)dipalladium (0) (6mg, 0.007mmol), 9,9-dimethyl-4,5-bis(diphenylphosphino)xanthene (12mg, 0.021 mmol) and caesium carbonate (68mg, 0.21 mmol) were added together in dioxane (2ml) and the resulting mixture was heated under reflux under argon for 1.5 hours. The reaction mixture was allowed to cool to room temperature, diluted with ethyl acetate and water was added. The ethyl acetate layer was separated, dried under magnesium sulfate and evaporated. The residue was purified by column chromatography eluting with a mixture of ethyl acetate/pentane (1:1 to 4:1) to afford the product (D17); MS (ES+) m/e 411 [M+H]⁺.

### Description 18

### 1-[5-(5,6,7,8-Tetrahydro-4H-[1,3]thiazolo[4,5-d]azepin-2-yl)-2-pyridinyl]-2-pyrrolidinone (D18)

1-[5-(5,6,7,8-Tetrahydro-4*H*-[1,3]thiazolo[4,5-*d*]azepin-2-yl)-2-pyridinyl]-2-pyrrolidinone was prepared from 1-{5-[6-(trifluoroacetyl)-5,6,7,8-tetrahydro-4*H*-[1,3]thiazolo[4,5-*d*]azepin-2-yl]-2-pyridinyl}-2-pyrrolidinone (may be prepared as described in Description 17) and potassium carbonate using an analogous process to that described in Description 11; MS (ES+) m/e 315 [M+H]⁺.

### Description 19

### 1-Methyl-3-{5-[6-(trifluoroacetyl)-5,6,7,8-tetrahydro-4H-[1,3]thiazolo[4,5-d]azepin-2-yl]-2-pyridinyl}-2-imidazolidinone (D19)

1-Methyl-3-{5-[6-(trifluoroacetyl)-5,6,7,8-tetrahydro-4*H*-[1,3]thiazolo[4,5-*d*]azepin-2-yl]-2-pyridinyl}-2-imidazolidinone was prepared from 2-(6-chloro-3-pyridinyl)-6-(trifluoroacetyl)-5,6,7,8-tetrahydro-4*H*-[1,3]thiazolo[4,5-*d*]azepine (may be prepared as described in Description 16, Method B) and 1-methyl-2-imidazolidinone using an analogous process to that described in Description 17; MS (ES+) m/e 426 [M+H]⁺.

### Description 20

### 1-Methyl-3-[5-(5,6,7,8-tetrahydro-4H-[1,3]thiazolo[4,5-d]azepin-2-yl)-2-pyridinyl]-2-imidazolidinone (D20)

1-Methyl-3-[5-(5,6,7,8-tetrahydro-4*H*-[1,3]thiazolo[4,5-*d*]azepin-2-yl)-2-pyridinyl]-2-imidazolidinone was prepared from 1-methyl-3-{5-[6-(trifluoroacetyl)-5,6,7,8-tetrahydro-4*H*-[1,3]thiazolo[4,5-*d*]azepin-2-yl]-2-pyridinyl}-2-imidazolidinone (may be prepared as described in Description 19) and potassium carbonate using an analogous process to that described in Description 11; MS (ES+) m/e 330 [M+H]⁺.

### Description 21

### 2-(6-Chloro-3-pyridinyl)-5,6,7,8-tetrahydro-4H-[1,3]thiazolo[4,5-d]azepine (D21) Method A

2-(6-Chloro-3-pyridinyl)-5,6,7,8-tetrahydro-4*H*-[1,3]thiazolo[4,5-*d*]azepine was prepared from 2-(6-chloro-3-pyridinyl)-6-(trifluoroacetyl)-5,6,7,8-tetrahydro-4*H*-[1,3]thiazolo[4,5-*d*]azepine (may be prepared as described in Description 16, Method D) and potassium carbonate using an analogous process to that described in Description 11, method A; MS (ES+) m/e 266 [M+H]⁺.

### Method B

2-(6-Chloro-3-pyridinyl)-6-(trifluoroacetyl)-5,6,7,8-tetrahydro-4*H*-[1,3]thiazolo[4,5-*d*]azepine (may be prepared as described in Description 16) (110mg, 0.31 mmol) was dissolved in methanol (3ml) and water (3ml) and treated with potassium carbonate (210mg, 1.52mmol). The mixture was allowed to stir at room temperature for 3 hours. The mixture was diluted with methanol and passed down an ion exchange cartridge (SCX) and washed with methanol and then a 2M ammonia in methanol solution. The basic fractions were then reduced to afford the product (D21); MS (ES+) m/e 266 [M+H]⁺.

### Method C

To a suspension of 2-(6-chloro-3-pyridinyl)-6-(trifluoroacetyl)-5,6,7,8-tetrahydro-4H-[1,3]thiazolo[4,5-d]azepine (maybe prepared as described in Description 16, Method E or F) (175mg, 0.485mmol) in methanol-water (8ml-4ml) was added potassium carbonate (335mg, 2.425mmol) and the resulting mixture stirred at room temperature for 2 hours. After this time the reaction was acidified with 2M hydrochloric acid and applied to an ion exchange cartridge (SCX), washed with methanol and then a 2M ammonia in methanol solution. The basic fractions were then evaporated *in vacuo* to afford the product (D21); MS (ES+) m/e 266 [M+H]⁺.

### Description 22

### 6-(Tributylstannanyl)-3-pyridinecarbonitrile (D22)

To a solution of hexabutylditin (3.98ml, 7.94mmol) in tetrahydrofuran (40ml) at 0 °C was added n-butyllithium (2.7M solution in heptane) (2.94ml, 7.94mmol). The resulting mixture was allowed to stir at 0 °C for 15 minutes. After this time a solution of 6-chloro-3-pyridinecarbonitrile (1.00g, 7.22mmol) in tetrahydrofuran (5ml) was added. The resulting brown solution was allowed to warm slowly to room temperature overnight. Evaporated and purified using column chromatography eluting with a mixture of ethyl acetate in pentane (0-10%) to afford the product (D22); ¹H NMR (CDCl₃) δ 0.89 (9H, m), 1.14 (6H, m), 1.35 (6H, m), 1.53 (6H, m), 7.55 (1H, d), 7.71 (1 H, dd), 8.95 (1H, d).

### Description 23

### 6-[6-(Trifluoroacetyl)-5,6,7,8-tetrahydro-4H-[1,3]thiazolo[4,5-d]azepin-2-yl]-3-pyridinecarbonitrile (D23)

A mixture of 2-iodo-6-(trifluoroacetyl)-5,6,7,8-tetrahydro-4*H*-[1,3]thiazolo[4,5-*d*]azepine (may be prepared as described in Description 5) (120mg, 0.32mmol), 6-(tributylstannanyl)-3-pyridinecarbonitrile (may be prepared as described in Description 22) (188mg, 0.48mmol) and bis(triphenylphosphine)palladium(II) chloride (11.0mg, 0.016mmol) in toluene (5ml) was heated at reflux for 3.5 hours. A further portion of bis(triphenylphosphine)palladium (II) chloride (11.0mg, 0.016mmol) was added and refluxing continued for 3 hours and then overnight. The mixture was evaporated and purified by column chromatography eluting with a mixture of ethyl acetate in pentane (0-50%) to afford the product (D23); MS (ES+) m/e 353 [M+H]⁺.

### Description 24

### 6-(5,6,7,8-Tetrahydro-4H-[1,3]thiazolo[4,5-d]azepin-2-yl)-3-pyridinecarbonitrile (D24)

To a solution of 6-[6-(trifluoroacetyl)-5,6,7,8-tetrahydro-4*H*-[1,3]thiazolo[4,5-*d*]azepin-2-yl]-3-pyridinecarbonitrile (may be prepared as described in Description 23) (70.0mg, 0.20mmol) in dioxan (2ml) and water (0.5ml) was added sodium carbonate (42.0mg, 0.40mmol) and the resulting mixture heated at reflux for 3.5 hours. Allowed to cool to room temperature and acidified using 2M HCl. The mixture was then passed down an ion exchange cartridge (SCX) and washed with methanol and then a 2M ammonia in methanol solution. The basic fractions were then combined and evaporated to afford the product (D24); MS (ES+) m/e 257 [M+H]⁺.

### Description 25

### 6-Methyl-2-pyrazinecarboxylic acid (D25)

2,6-Dimethylpyrazine (500mg, 4.60mmol) was dissolved in water (10ml), heated at 70°C and KMnO₄ in water (25ml) was added dropwise. The mixture was stirred and heated overnight. After cooling to room temperature the MnO₂ cake was filtered and washed with water several times. The filtrate was acidified with 5M HCl solution (pH 1.5) and extracted with ethyl acetate (3 x 50ml). The residue was dried over magnesium sulphate, filtered and evaporated to afford the product (D25); MS (ES+) m/e 139 [M+H]⁺.

### Description 26

### 2-Methyl-4-pyridinecarboxylic acid (D26)

A hydrogen filled balloon was attached to a flask containing 2-chloro-6-methyl-4-pyridinecarboxylic acid (350mg, 2.10mmol), 10% palladium on activated carbon (88.0mg, 0.08mmol), triethylamine (1ml) and ethanol (15ml). The mixture was stirred during the afternoon and overnight at room temperature. The reaction mixture was filtered through celite and washed with ethanol. The solvent was evaporated and the residue triturated with dichloromethane and filtered to afford the product (D26); MS (ES+) m/e 138 [M+H]⁺.

### Description 27

### 2-(4-Piperidinyl)-6-(trifluoroacetyl)-5,6,7,8-tetrahydro-4H-[1,3]thiazolo[4,5-d]azepine (D27)

1-(Trifluoroacetyl)hexahydro-4*H*-azepin-4-one (may be prepared as described in Description 3) (1.00g, 4.78mmol) was dissolved in acetic acid (10ml) and the mixture heated at 60°C. Bromine (0.25ml, 4.78mmol) in acetic acid (10ml) was then added dropwise at such a rate that the solution decolourised between drops. The mixture was left stirring at 60 °C for 30 minutes. The acetic acid was evaporated and azeotroped with toluene. The mixture was re-dissolved in ethanol, treated with 1,1-dimethylethyl 4-(aminocarbonothioyl)-1-piperidinecarboxylate (2.34g, 9.57mmol) and heated at reflux for 4 hours and then overnight. The mixture was diluted with methanol and passed down an ion exchange cartridge (SCX), washed with methanol and then a 2M ammonia in methanol solution. The basic fractions were then combined and evaporated to afford the crude product which was purified using column chromatography eluting with a mixture of 2M ammonia in methanol and dichloromethane (10%) to afford the product (D27); MS (ES+) m/e 334 [M+H]⁺.

### Description 28

### 2-[1-(4-Pyridinylcarbonyl)-4-piperidinyl]-5,6,7,8-tetrahydro-4H-[1,3]thiazolo[4,5-d]azepine (D28)

A mixture of 4-pyridinecarboxylic acid (74.0mg, 0.60mmol), 1*H*-1,2,3-benzotriazol-1-ol (81.0mg, 0.60mmol), and N-cyclohexylcarbodiimide, N'-methyl polystyrene (2.1 mmol/g) (286mg, 0.60mmol) in dimethylformamide (5ml) was stirred at room temperature for 30 minutes. After this time a solution of 2-(4-piperidinyl)-6-(trifluoroacetyl)-5,6,7,8-tetrahydro-4*H*-[1,3]thiazolo[4,5-*d*]azepine (may be prepared as described in Description 27) (100mg, 0.30mmol) in dimethylformamide (5ml) was added and the resulting mixture stirred at room temperature for 3 hours and then at 70°C for 1.5 hours. The mixture was filtered and the solvent evaporated. Potassium carbonate (249mg, 1.8mmol), methanol (3ml) and water (0.5ml) were added and the mixture stirred at 60 °C overnight. The mixture was then acidified to pH 4 with acetic acid and passed down an ion exchange cartridge (SCX) and washed with methanol and then a 2M ammonia in methanol solution. The basic fractions were then reduced and purified using silica gel chromatography, eluting with a mixture of 2M ammonia in methanol and dichloromethane (7%) to afford the product (D28); MS (ES+) m/e 343 [M+H]⁺.

### Description 29

### 2-Methylpyrimidine 1-oxide (D29)

2-Methylpyrimidine (may be prepared according to the procedure described in J. Med. Chem., 2005, 48, 1367) (250mg, 2.66mmol) was dissolved in acetic acid (5ml), treated with aqueous hydrogen peroxide (27.5% solution in water) and heated at 70°C under argon for 5.5 hours. The mixture was allowed to cool to room temperature and the solvent evaporated, redissolved in water and re-evaporated. Basified by adding 1 M sodium carbonate solution (5ml) and extracted with chloroform (5 x 30ml). The chloroform layers were combined, dried over magnesium sulphate, filtered and evaporated to afford the product (D29); ¹H NMR (CDCl₃) δ 2.75 (3H, s), 7.21 (1H, dd), 8.17 (1 H, dd), 8.40 (1H, dd).

### Description 30

### 2-Methyl-4-pyrimidinecarbonitrile (D30)

2-Methylpyrimidine 1-oxide (may be prepared as described in Description 29) (100mg, 0.91mmol) was dissolved in acetonitrile (4ml) and treated with trimethylsilyl cyanide (363µl, 2.73mmol) and triethylamine (252µl, 1.82mmol). The mixture was heated under argon at reflux for 5 hours. The mixture was allowed to cool to room temperature and the solvent evaporated. The residue was partitioned between sodium carbonate solution (1 M) (10ml) and dichloromethane (3 x 50ml). The dichloromethane layers were combined, dried over magnesium sulphate, filtered and evaporated to afford the product (D30); MS (ES+) m/e 120 [M+H]⁺.

### Description 31

### 2-Methyl-4-pyrimidinecarboxylic acid (D31)

2-methyl-4-pyrimidinecarbonitrile (may be prepared as described in Description 30) (62.0mg, 0.52mmol) was dissolved in ethanol (3ml), treated with 10% sodium hydroxide solution (3ml) and heated at reflux for 5 hours. The ethanol was evaporated and the mixture dissolved in water and acidified with 5M HCl solution (pH 1). Extracted with ethyl acetate (3 x 50ml), dried over magnesium sulphate, filtered and evaporated to afford the product (D31); MS (ES+) m/e 139 [M+H]⁺.

### Description 32

### Ethyl 2-formyl-3-oxopropanoate (D32)

To a dry 250ml 3-necked flask, sodium hydride (60% in oil) (778mg, 32.4mmol) was added in diethyl ether (35ml). A condenser was attached and the flask placed under an atmosphere of argon. The mixture was stirred and cooled to 0 °C. Ethyl formate (22ml, 270mmol) was added dropwise, followed by a solution of methyl 3,3-bis(methyloxy)propanoate (3.83ml, 27.0mmol) in diethyl ether (25ml) added dropwise over 10 minutes. The mixture was stirred at 0 °C for 1.5 hours and then warmed to room temperature overnight. Poured into 100ml of ice-water and extracted with diethyl ether (3 x 50 ml) which was discarded. The aqueous phase was acidified (pH 3) with concentrated HCl (3ml) and extracted with dichloromethane (5 x 50ml), dried, filtered and evaporated. The residue was purified using silica gel chromatography, eluting with a mixture of methanol and dichloromethane (0-25%) to afford the product (D32); MS (ES+) m/e 143 [M-H]-.

### Description 33

### Ethyl 2-methyl-5-pyrimidinecarboxylate (D33)

Ethyl 2-formyl-3-oxopropanoate (may be prepared as described in Description 32) (745mg, 5.17mmol) was dissolved in ethanol (15ml). Acetamidine hydrochloride (489mg, 5.17mmol) and sodium ethoxide in ethanol (0.41 ml, 5.17mmol) were added and the mixture heated under argon at reflux for 6 hours and then overnight. The solvent was evaporated and the residue was treated with water (50ml) and extracted with diethyl ether (4 x 30ml). The diethyl ether layers were combined, dried over magnesium sulphate, filtered and evaporated. The crude product was purified using silica gel chromatography, eluting with a mixture of ethyl acetate and pentane (20-50%) to afford the product (D33); MS (ES+) m/e 167 [M+H]⁺.

### Description 34

### 2-Methyl-5-pyrimidinecarboxylic acid (D34)

Ethyl 2-methyl-5-pyrimidinecarboxylate (may be prepared as described in Description 33) (60.0mg, 0.36mmol) was dissolved in ethanol (3ml), treated with 2M sodium hydroxide solution (0.54ml, 1.08mmol) and the resulting mixture stirred at room temperature for 3 hours. The ethanol was evaporated, water (50ml) added and the solution acidified with 2M HCl solution. Extracted with ethyl acetate (3 x 50ml), dried over magnesium sulphate, filtered and evaporated to afford the product (D34); MS (ES+) m/e 139 [M+H]⁺.

### Description 35

### (1Z)-N-hydroxyethanimidamide (D35)

A mixture of acetonitrile (1.7ml, 32.9mmol) and a 50% aqueous solution of hydroxylamine (2.5ml, 37.9mmol) in ethanol (5ml) was heated at reflux for 4.5 hours. The solvent was evaporated to give a white crystalline solid which was triturated with diethyl ether, filtered and dried in the vacuum oven to afford the product (D35); ¹H NMR (d⁶-DMSO) δ 1.62 (3H, s), 5.34 (2H, br s), 8.65 (1 H, s).

### Description 36

### Methyl 5-(trimethylstannanyl)-2-pyrazinecarboxylate (D36)

Methyl 5-chloro-2-pyrazinecarboxylate (1.0g 5.81mmol) and tetrabutylammonium iodide (2.36g 6.4mmol) were degassed in 20 ml of dry toluene for 10 minutes, while hexamethylditin (2.1g 6.4mmol) was added to 10 ml of dry degassed toluene. Tetrakis(triphenylphosphine)palladium(0) (340mg, 5%mol) was added to the reaction mixture along with the hexamethylditin in dry toluene. The resulting mixture was heated under reflux under argon for 2 hours. The reaction was allowed to cool down, volatiles were removed under reduce pressure and the residue purified using silica gel chromatography to afford the product (D36); MS (ES+) m/e 302 [M+H]+.

### Description 37

### 3-(5,6,7,8-Tetrahydro-4H-[1,3]thiazolo[4,5-d]azepin-2-yl)benzonitrile (D37)

A mixture of 2-iodo-6-(trifluoroacetyl)-5,6,7,8-tetrahydro-4*H*-[1,3]thiazolo[4,5-*d*]azepine (150 mg, 0.4 mmol) (may be prepared as described in Description 5), (3-cyanophenyl)boronic acid (87 mg, 0.6 mmol) and tetrakis triphenylphosphine palladium (0) (5 mg, 0.04 mmol) in a mixture of 1,4-dioxan (5 ml) and 1 M sodium carbonate solution (1 ml) was heated at reflux for 18 hours. The mixture was allowed to cool and was purified on a 5g SCX ion exchange cartridge eluting with methanol and then 2M ammonia in methanol. The basic fractions were combined and evaporated to afford the title compound (D37); MS (ES+) m/e 256 [M+H]⁺.

### Description 38

### N,N-Dimethyl-4-(5,6,7,8-tetrahydro-4H-[1,3]thiazolo[4,5-d]azepin-2-yl)aniline (D38)

2-Iodo-6-(trifluoroacetyl)-5,6,7,8-tetrahydro-4H-[1,3]thiazolo[4,5-d]azepine (may be prepared as described in Description 5) (100mg,0.266mmol) [4-(dimethylamino)phenyl]boronic acid (53mg, 0.32mmol) bis(triphenylphosphine)palladium (II) chloride (19 mg, 10%mol) and sodium carbonate (106 mg, 1.0 mmol) were added together in dioxan (2 ml) and water (0.5 ml) and the resulting mixture was heated under reflux under argon for 2 hours. After that time, a further quantity of sodium carbonate (106 mg, 1.0 mmol) in 0.5 mL of water was added and the resulting mixture was heated under argon under reflux for one more hour. After that time, the reaction mixture was allowed to cool down, acidified to pH=1 with 2N hydrochloric acid and applied to an ion exchange cartridge (SCX), washing with methanol and then a 2M ammonia in methanol solution. The basic fractions were then evaporated *in vacuo* to afford the product (D38); MS (ES+) m/e 274 [M+H]⁺.

### Example 1

### 6-Cyclobutyl-2-[4-(trifluoromethyl)phenyl]-5,6,7,8-tetrahydro-4H-[1,3]thiazolo[4,5-d]azepine (E1)

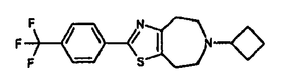

A crude mixture of 5-bromo-1-cyclobutylhexahydro-4*H*-azepin-4-one (may be prepared as described in Description 2) (1.2mmol) and 4-(trifluoromethyl)benzenecarbothioamide (0.38g, 1.85mmol) in ethanol was heated under reflux for 18 hours. The reaction was then cooled, applied to an ion exchange cartridge (SCX) and washed with methanol and then a 2M ammonia in methanol solution. The basic fractions were then reduced and the residue purified using reverse phase chromatography to afford the product (E1); MS (ES+) m/e 353 [M+H]⁺.

### Example 2

### 2-(4-Bromophenyl)-6-cyclobutyl-5,6,7,8-tetrahydro-4H-[1,3]thiazolo[4,5-d]azepine (E2)

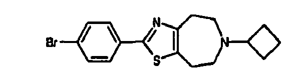

### Method A

2-(4-Bromophenyl)-6-cyclobutyl-5,6,7,8-tetrahydro-4*H*-[1,3]thiazolo[4,5-*d*]azepine (E2) was prepared from 5-bromo-1-cyclobutylhexahydro-4*H*-azepin-4-one (may be prepared as described in Description 2) and 4-bromobenzenecarbothioamide using an analogous process to that described in example 1; MS (ES+) m/e 363 and 365 [M+H]⁺.

### Method B

1-Cyclobutylhexahydro-4*H*-azepin-4-one (may be prepared as described in Description 1, method B) (500mg, 2.99mmol) was dissolved in acetic acid (3ml), treated with bromine (0.15ml, 2.99mmol) and stirred at room temperature under argon for 6 hours. The mixture was evaporated and azeotroped with toluene. The crude product was dissolved in ethanol (5ml), treated with 4-bromobenzenecarbothioamide (969mg, 4.49mmol) and heated under reflux for 18 hours. The mixture was allowed to cool to room temperature, the resulting solid was collected by filtration and redissolved in a mixture of dichloromethane/methanol/dimethylformamide. This solution was applied to an ion exchange cartridge (SCX) and washed with methanol and then a 2M ammonia in methanol solution. The basic fractions were combined and evaporated to afford the product (E2); MS (ES+) m/e 364 [M+H]⁺.

### Example 3

### 1-[4-(6-cyclobutyl-5,6,7,8-tetrahydro-4H-[1,3]thiazolo[4,5-d]azepin-2-yl)phenyl]-2-pyrrolidinone (E3)

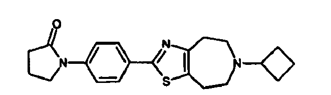

A mixture of 2-(4-bromophenyl)-6-cyclobutyl-5,6,7,8-tetrahydro-4*H*-[1,3]thiazolo[4,5-*d*]azepine (may be prepared as described in Example 2) (0.05g, 0.14mmol), pyrrolidinone (0.014g, 0.17mmol), cesium carbonate (0.064g, 0.2mmol), tris(dibenzylideneacetone)dipalladium(0) (0.0065g, 0.007mmol), and 4,5-bis(diphenylphosphino)-9,9-dimethyl-xanthene (Xantphos) (0.012g, 0.021mmol) in 1,4-dioxane (3ml) was heated under argon at 100°C. The reaction was then cooled to room temperature, diluted with dichloromethane, washed with water, dried with Na₂SO₄ and reduced. The crude reaction mixture was then purified using reverse phase chromatography to afford the title product (E3); MS (ES+) m/e 368 [M+H]⁺.

### Example 4

### 6-Cyclobutyl-2-[6-(trifluoromethyl)-3-pyridinyl]-5,6,7,8-tetrahydro-4H-[1,3]thiazolo[4,5-d]azepine (E4)

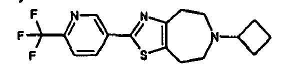

6-Cyclobutyl-2-[6-(trifluoromethyl)-3-pyridinyl]-5,6,7,8-tetrahydro-4*H*-[1,3]thiazolo[4,5-*d*]azepine(E4) was prepared from 5-bromo-1-cyclobutylhexahydro-4*H*-azepin-4-one (may be prepared as described in Description 2) and 6-(trifluoromethyl)-3-pyridinecarbothioamide using an analogous process to that described in Example 1; MS (ES+) m/e 354 [M+H]⁺.

### Example 5

### 6-Cyclobutyl-2-[4-(1,2,3-thiadiazol-4-yl)phenyl]-5,6,7,8-tetrahydro-4H-[1,3]thiazolo[4,5-d]azepine (E5)

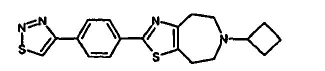

6-Cyclobutyl-2-[4-(1,2,3-thiadiazol-4-yl)phenyl]-5,6,7,8-tetrahydro-4*H*-[1,3]thiazolo[4,5-*d*]azepine (E5) was prepared from 5-bromo-1-cyclobutylhexahydro-4H-azepin-4-one (may be prepared as described in Description 2) and 4-(1,2,3-thiadiazol-4-yl)benzenecarbothioamide using an analogous process to that described in example 1; MS (ES+) m/e 369 [M+H]⁺.

### Example 6

### 1,1-Dimethylethyl 4-(6-cyclobutyl-5,6,7,8-tetrahydro-4H-[1,3]thiazolo[4,5-d]azepin-2-yl)-1-piperidinecarboxylate (E6)

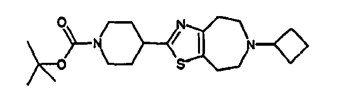

1,1-Dimethylethyl 4-(6-cyclobutyl-5,6,7,8-tetrahydro-4*H*-[1,3]thiazolo[4,5-*d*]azepin-2-yl)-1-piperidinecarboxylate (E6) was prepared from 5-bromo-1-cyclobutylhexahydro-4H-azepin-4-one (may be prepared as described in Description 2) and 1,1-dimethylethyl 4-(aminocarbonothioyl)-1-piperidinecarboxylate using an analogous process to that described in example 1; MS (ES+) m/e 392 [M+H]⁺.

### Example 7

### 6-Cyclobutyl-2-(4-piperidinyl)-5,6,7,8-tetrahydro-4H-[1,3]thiazolo[4,5-d]azepine (E7)

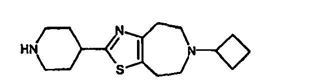

A solution of 1,1-dimethylethyl 4-(6-cyclobutyl-5,6,7,8-tetrahydro-4*H*-[1,3]thiazolo[4,5-*d*]azepin-2-yl)-1-piperidinecarboxylate (may be prepared as described in Example 6) (0.30g, 0.77mmol) in dichloromethane (4ml) was treated with trifluoroacetic acid (2ml) at 0 °C. The resulting mixture was allowed to stir at room temperature for one hour. The reaction was then applied to an ion exchange cartridge (SCX) and washed with methanol and then a 2M ammonia in methanol solution. The basic fractions were then reduced to afford the product (E7); MS (ES+) m/e 292 [M+H]⁺.

### Example 8

### 6-Cyclobutyl-2-{1-[(6-methyl-3-pyridinyl)carbonyl]-4-piperidinyl}-5,6,7,8-tetrahydro-4H-[1,3]thiazolo[4,5-d]azepine (E8)

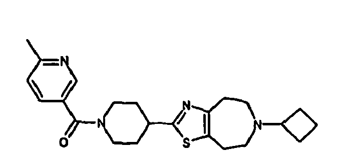

A suspension of 6-methyl-3-pyridinecarboxylic acid (0.019g, 0.14mmol), 1*H*-1,2,3-benzotriazol-1-ol (19mg, 0.14mmol), and N-cyclohexylcarbodiimide, N'-methyl polystyrene (2.1 mmol/g) (0.067g, 0.14mmol) in dimethylformamide (2ml) was stirred at room temperature for one hour. After this time a solution of 6-cyclobutyl-2-(4-piperidinyl)-5,6,7,8-tetrahydro-4*H*-[1,3]thiazolo[4,5-*d*]azepine (may be prepared as described in Example 7) (0.02g, 0.07mmol) in dichloromethane (2ml) was added and the resulting mixture stirred at room temperature for 18 hours. The reaction was then applied to an ion exchange cartridge (SCX) and washed with methanol and then a 2M ammonia in methanol solution. The basic fractions were then reduced and purified using silica gel chromatography to afford the product (E8); MS (ES+) m/e 411 [M+H]⁺.

### Example 9

### 5-{[4-(6-Cyclobutyl-5,6,7,8 tetrahydro-4H-[1,3]thiazolo[4,5-d]azepin-2-yl)-1-piperidinyl]carbonyl}-2-pyridinecarbonitrile (E9)

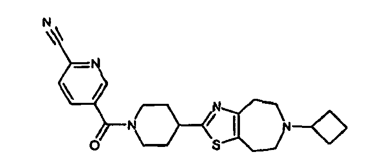

5-{[4-(6-Cyclobutyl-5,6,7,8-tetrahydro-4*H*-[1,3]thiazolo[4,5-*d*]azepin-2-yl)-1-piperidinyl]carbonyl}-2-pyridinecarbonitrile (E9) was prepared from 6-cyclobutyl-2-(4-piperidinyl)-5,6,7,8-tetrahydro-4*H*-[1,3]thiazolo[4,5-*d*]azepine (may be prepared as described in Example 7) and 6-cyano-3-pyridinecarboxylic acid using an analogous process to that described in example 8; MS (ES+) m/e 422 [M+H]⁺.

### Example 10

### 6-Cyclobutyl-2-(1-{[6-(trifluoromethyl)-3-pyridinyl]carbonyl}-4-piperidinyl)-5,6,7,8-tetrahydro-4H-[1,3]thiazolo[4,5-d]azepine (E10)

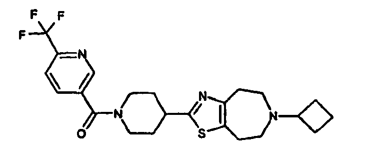

6-Cyclobutyl-2-(1-{[6-(triftuoromethyl)-3-pyridinyl]carbonyl}-4-piperidinyl)-5,6,7,8-tetrahydro-4*H*-[1,3]thiazolo[4,5-*d*]azepine (E10) was prepared from 6-cyclobutyl-2-(4-piperidinyl)-5,6,7,8-tetrahydro-4*H*-[1,3]thiazolo[4,5-*d*]azepine (may be prepared as described in Example 7) and 6-(trifluoromethyl)-3-pyridinecarboxylic acid using an analogous process to that described in example 8; MS (ES+) m/e 465 [M+H]⁺.

### Example 11

### 6-Cyclobutyl-2-{1-[(6-methyl-2-pyridinyl)carbonyl]-4-piperidinyl}-5,6,7,8-tetrahydro-4H-[1,3]thiazolo[4,5-d]azepine (E11)

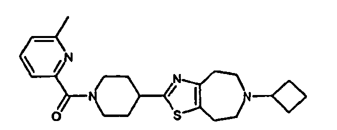

6-Cyclobutyl-2-{1-[(6-methyl-2-pyridinyl)carbonyl]-4-piperidinyl}-5,6,7,8-tetrahydro-4*H-*[1,3]thiazolo[4,5-*d*]azepine (E11) was prepared from 6-cyclobutyl-2-(4-piperidinyl)-5,6,7,8-tetrahydro-4*H*-[1,3]thiazolo[4,5-*d*]azepine (may be prepared as described in Example 7) and 6-methyl-2-pyridinecarboxylic acid using an analogous process to that described in example 8; MS (ES+) m/e 411 [M+H]⁺. ¹H-NMR (CDCl₃) δ 1.60-1.78 (2H, m), 1.80-1.92 (4H, m), 2.01-2.20 (4H, m), 2.58 (3H, s), 2.61, (4H, m), 2.87-2.89 (2H, m), 2.93-3.05 (4H, m), 3.13-3.22 (2H, m), 3.98 (1H, m), 4.79 (1H, m), 7.19 (1H, d), 7.37 (1 H, d), 7.66 (1 H, dd).

### Example 12

### 6-Cyclobutyl-2-{1-[(5-methyl-2-pyrazinyl)carbonyl]-4-piperidinyl}-5,6,7,8-tetrahydro-4H-[1,3]thiazolo[4,5-d]azepine (E12)

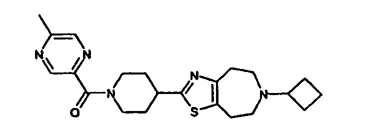

6-Cyclobutyl-2-{1-[(5-methyl-2-pyrazinyl)carbonyl]-4-piperidinyl}-5,6,7,8-tetrahydro-4*H-*[1,3]thiazolo[4,5-*d*]azepine (E12) was prepared from 6-cyclobutyl-2-(4-piperidinyl)-5,6,7,8-tetrahydro-4*H*-[1,3]thiazolo[4,5-*d*]azepine (may be prepared as described in Example 7) and 5-methyl-2-pyrazinecarboxylic acid using an analogous process to that described in example 8; MS (ES+) m/e 412 [M+H]⁺.

### Example 13

### 6-Cyclobutyl-2-{1-[(5-methyl-3-pyridinyl)carbonyl]-4-piperidinyl}-5,6,7,8-tetrahydro-4H-[1,3]thiazolo[4,5-d]azepine (E13)

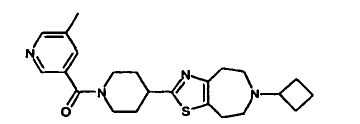

6-Cyclobutyl-2-{1-[(5-methyl-3-pyridinyl)carbonyl]-4-piperidinyl}-5,6,7,8-tetrahydro-4*H-*[1,3]thiazolo[4,5-*d*]azepine (E13) was prepared from 6-cyclobutyl-2-(4-piperidinyl)-5,6,7,8-tetrahydro-4*H*-[1,3]thiazolo[4,5-*d*]azepine (may be prepared as described in Example 7) and 5-methyl-3-pyridinecarboxylic acid using an analogous process to that described in example 8; MS (ES+) m/e 411 [M+H]⁺. ¹H-NMR (CDCl₃) δ 1.54-2.09 (6H, m), 2.11-2.16 (4H, m), 2.38 (3H, s), 2.64 (4H, m), 2.89 (2H, m), 2.97-3.07 (4H, m), 3.10-3.23 (2H, m), 3.85 (1 H, br s), 4.73 (1 H, br s), 7.57 (1 H, dd), 8.46 (1 H, d), 8.49 (1 H, d).

### Example 14

### 6-Cyclobutyl-2-{1-[(4-methylphenyl)carbonyl]-4-piperidinyl}-5,6,7,8-tetrahydro-4H-[1,3]thiazolo[4,5-d]azepine (E14)

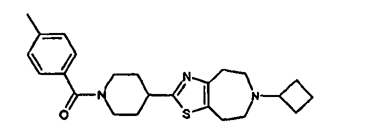

6-Cyclobutyl-2-{1-[(4-methylphenyl)carbonyl]-4-piperidinyl}-5,6,7,8-tetrahydro-4*H-*[1,3]thiazolo[4,5-*d*]azepine (E14) was prepared from 6-cyclobutyl-2-(4-piperidinyl)-5,6,7,8-tetrahydro-4*H*-[1,3]thiazolo[4,5-*d*]azepine (may be prepared as described in Example 7) and 4-methylbenzoic acid using an analogous process to that described in example 8; MS (ES+) m/e 410 [M+H]⁺.

### Example 15

### 6-Cyclobutyl-2-{1-[(5-methyl-3-isoxazolyl)carbonyl]-4-piperidinyl}-5,6,7,8-tetrahydro-4H-[1,3]thiazolo[4,5-d]azepine (E15)

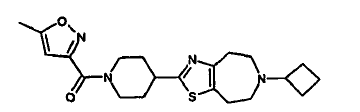

6-Cyclobutyl-2-{1-[(5-methyl-3-isoxazolyl)carbonyl]-4-piperidinyl}-5,6,7,8-tetrahydro-4*H-*[1,3]thiazolo[4,5-*d*]azepine (E15) was prepared from 6-cyclobutyl-2-(4-piperidinyl)-5,6,7,8-tetrahydro-4*H*-[1,3]thiazolo[4,5-*d*]azepine (may be prepared as described in Example 7) and 5-methyl-3-isoxazolecarboxylic acid using an analogous process to that described in example 8; MS (ES+) m/e 401 [M+H]⁺.

### Example 16

### 6-Cyclobutyl-2-[1-(1,2,3-thiadiazol-4-ylcarbonyl)-4-piperidinyl]-5,6,7,8-tetrahydro-4H-[1,3]thiazolo[4,5-d]azepine (E16)

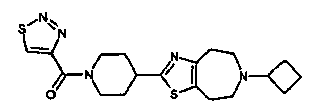

6-Cyclobutyl-2-[1-(1,2,3-thiadiazol-4-ylcarbonyl)-4-piperidinyl]-5,6,7,8-tetrahydro-4*H-*[1,3]thiazolo[4,5-*d*]azepine (E16) was prepared from 6-cyclobutyl-2-(4-piperidinyl)-5,6,7,8-tetrahydro-4*H*-[1,3]thiazolo[4,5-*d*]azepine (may be prepared as described in Example 7) and 1,2,3-thiadiazole-4-carboxylic acid using an analogous process to that described in example 8; MS (ES+) m/e 404 [M+H]⁺.

### Example 17

### 6-Cyclobutyl-2-{1-[(1-methyl-1H-pyrazol-3-yl)carbonyl]-4-piperidinyl}-5,6,7,8-tetrahydro-4H-[1,3]thiazolo[4,5-d]azepine (E17)

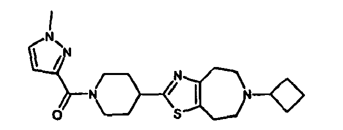

6-Cyclobutyl-2-{1-[(1-methyl-1*H*-pyrazol-3-yl)carbonyl]-4-piperidinyl}-5,6,7,8-tetrahydro-4*H*-[1,3]thiazolo[4,5-*d*]azepine (E17) was prepared from 6-cyclobutyl-2-(4-piperidinyl)-5,6,7,8-tetrahydro-4*H*-[1,3]thiazolo[4,5-*d*]azepine (may be prepared as described in Example 7) and 1-methyl-1*H*-pyrazole-3-carboxylic acid using an analogous process to that described in example 8; MS (ES+) m/e 400 [M+H]⁺.

### Example 18

### 6-Cyclobutyl-2-{1-[(3-methyl-5-isoxazolyl)carbonyl]-4-piperidinyl}-5,6,7,8-tetrahydro-4H-[1,3]thiazolo[4,5-d]azepine (E18)

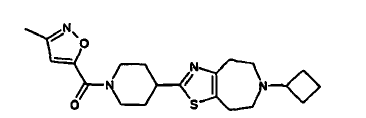

6-Cyclobutyl-2-{1-[(3-methyl-5-isoxazolyl)carbonyl]-4-piperidinyl}-5,6,7,8-tetrahydro-4*H-*[1,3]thiazolo[4,5-*d*]azepine (E18) was prepared from 6-cyclobutyl-2-(4-piperidinyl)-5,6,7,8-tetrahydro-4*H*-[1,3]thiazolo[4,5-*d*]azepine (may be prepared as described in Example 7) and 3-methyl-5-isoxazolecarboxylic acid using an analogous process to that described in example 8; MS (ES+) m/e 401 [M+H]⁺.

### Example 19

### 6-Cyclobutyl-2-[1-(6-methyl-3-pyridinyl)-4-piperidinyl]-5,6,7,8-tetrahydro-4H-[1,3]thiazolo[4,5-d]azepine (E19)

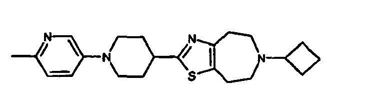

A mixture of 6-cyclobutyl-2-(4-piperidinyl)-5,6,7,8-tetrahydro-4*H*-[1,3]thiazolo[4,5-*d*]azepine (may be prepared as described in Example 7) (0.07g, 0.24mmol), 5-bromo-2-methylpyridine (0.124g, 0.72mmol), sodium *tert*-butoxide (9.092g, 0.96mmol), palladium acetate (0.010g, 0.04mmol), and 1,1'-binaphthalene-2,2'-diylbis(diphenylphosphane) (BINAP) (0.062g, 0.10mmol) in 1,4-dioxane (3ml) was heated under argon at reflux for 18 hours. The reaction was then applied to an ion exchange cartridge (SCX) and washed with methanol and then a 2M ammonia in methanol solution. The basic fractions were then reduced and purified using silica gel chromatography to afford the product (E19); MS (ES+) m/e 383 [M+H]⁺.

### Example 20

### 5-[4-(6-Cyclobutyl-5,6,7,8-tetrahydro-4H-[1,3]thiazolo[4,5-d]azepin-2-yl)-1-piperidinyl]-2-pyridinecarbonitrile (E20)

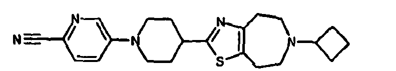

5-[4-(6-Cyclobutyl-5,6,7,8-tetrahydro-4*H*-[1,3]thiazolo[4,5-*d*]azepin-2-yl)-1-piperidinyl]-2-pyridinecarbonitrile (E20) was prepared from 6-cyclobutyl-2-(4-piperidinyl)-5,6,7,8-tetrahydro-4*H*-[1,3]thiazolo[4,5-*d*]azepine (may be prepared as described in Example 7) and 5-bromo-2-pyridinecarbonitrile using an analogous process to that described in example 19; MS (ES+) m/e 394 [M+H]⁺. ¹H-NMR (CDCl₃) δ 1.55-2.24 (10H, m), 2.71 (4H, m), 2.95-3.21 (8H, m), 3.94 (2H, m), 7.10 (1H, dd), 7.50 (1H, d), 8.25 (1H, d).

### Example 21

### 5-[4-(6-Cyclobutyl-5,6,7,8-tetrahydro-4H-[1,3]thiazolo[4,5-d]azepin-2-yl)-1-piperidinyl]-2-pyridinecarboxylic acid (E21)

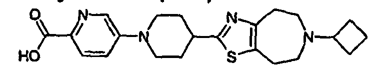

5-[4-(6-Cyclobutyl-5,6,7,8-tetrahydro-4*H*-[1,3]thiazolo[4,5-*d*]azepin-2-yl)-1-piperidinyl]-2-pyridinecarbonitrile (141mg, 0.36mmol; may be prepared as described in Example 20) was dissolved in formic acid (2ml) and treated with concentrated hydrochloric acid (2ml). The mixture was heated at reflux overnight. The solvent was evaporated and the residue azeotroped with toluene to give the title product (E21); MS (ES+) m/e 413 [M+H]⁺.

### Example 22

### 5-[4-(6-Cyclobutyl-5,6,7,8-tetrahydro-4H-[1,3]thiazolo[4,5-d]azepin-2-yl)-1-piperidinyl]-2-pyridinecarboxamide (E22)

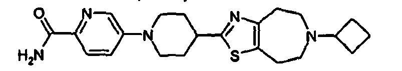

A mixture of 5-[4-(6-cyclobutyl-5,6,7,8-tetrahydro-4*H*-[1,3]thiazolo[4,5-*d*]azepin-2-yl)-1-piperidinyl]-2-pyridinecarboxylic acid (60mg, 0.15mmol; may be prepared as described in Example 21), 1-hydroxybenzotriazole (61 mg, 0.45mmol) and N-cyclohexylcarbodiimide,N'-methyl polystyrene (2.1 mmol/g, 214mg, 0.45mmol) in N,N-dimethylformamide (4ml) was stirred at room temperature for 1 hour. Ammonia (0.88 solution) (9µl) was added and the mixture stirred at room temperature during the weekend. The mixture was diluted with methanol and passed down an ion exchange cartridge (SCX), eluting with methanol and then a 2M ammonia in methanol solution. The basic fractions were combined and evaporated. The residue (50mg) was dissolved in dichloromethane (3ml), treated with N,N'-carbonyldiimidazole (40mg, 0.24mmol) and stirred at room temperature overnight. Ammonia (0,88 solution) was added and the mixture was stirred at room temperature for 5 hours. The solvent was evaporated and the product was purified by column chromatography (Biotage) with a mixture of 2M ammonia in methanol and dichloromethane (5:95). Fractions containing the product were combined and evaporated to give the title product (E22); MS (ES+) m/e 412 [M+H]⁺.

### Example 23

### 6-Cyclobutyl-2-{1-[6-(1H-imidazol-1-ylcarbonyl)-3-pyridinyl]-4-piperidinyl}-5,6,7,8-tetrahydro-4H-[1,3]thiazolo[4,5-d]azepine (E23)

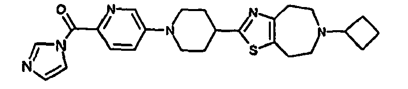

A solution of 5-[4-(6-cyclobutyl-5,6,7,8-tetrahydro-4*H*-[1,3]thiazolo[4,5-*d*]azepin-2-yl)-1-piperidinyl]-2-pyridinecarboxylic acid (may be prepared as described in Example 21) (140mg, 0.34mmol) in dichloromethane (5ml) was treated with 1,1'-carbonyldiimidazole (110mg, 0.68mmol) and heated at 40°C for 2 hours. The mixture was evaporated to dryness to afford the product (E23), which may be used without further purification.

### Example 24

### 5-[4-(6-Cyclobutyl-5,6,7,8-tetrahydro-4H-[1,3]thiazolo[4,5-d]azepin-2-yl)-1-piperidinyl]-N,N-dimethyl-2-pyridinecarboxamide (E24)

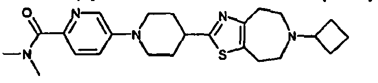

A solution of 6-cyclobutyl-2-{1-[6-(1*H*-imidazol-1-ylcarbonyl)-3-pyridinyl]-4-piperidinyl}-5,6,7,8-tetrahydro-4*H*-[1,3]thiazolo[4,5-*d*]azepine (may be prepared as described in Example 23) (119mg, 0.25mmol) in dichloromethane (3ml) was treated with dimethylamine (0.50ml, 1.00mmol) and stirred at room temperature for 4 hours and allowed to stand at room temperature overnight. The crude mixture was then purified using silica gel chromatography and re-purified using reverse phase chromatography to afford the title product (E24); MS (ES+) m/e 440 [M+H]⁺.

### Example 25

### 6-Cyclobutyl-2-{1-[(2-methyl-3-pyridinyl)carbonyl]-4-piperidinyl}-5,6,7,8-tetrahydro-4H-[1,3]thiazolo[4,5-d]azepine (E25)

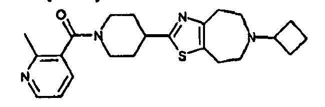

A suspension of 2-methyl-3-pyridinecarboxylic acid (47.0mg, 0.34mmol), 1*H*-1,2,3-benzotriazol-1-ol (46mg, 0.34mmol), and N-cyclohexylcarbodiimide, N'-methyl polystyrene (2.1 mmol/g) (162mg, 0.34mmol) in dimethylformamide (3ml) was stirred at room temperature for 1.5 hours. After this time a solution of 6-cyclobutyl-2-(4-piperidinyl)-5,6,7,8-tetrahydro-4*H*-[1,3]thiazolo[4,5-d]azepine (may be prepared as described in Example 7) (50.0mg, 0.17mmol) in dimethylformamide was added and the resulting mixture stirred at room temperature for 1 hour. The reaction was then diluted with methanol and applied to an ion exchange cartridge (SCX) and washed with methanol and then a 2M ammonia in methanol solution. The basic fractions were then reduced and purified using silica gel chromatography to afford the product (E25); MS (ES+) m/e 411 [M+H]⁺.

### Example 26

### 1-[4-(6-Cyclobutyl-5,6,7,8-tetrahydro-4H-[1,3]thiazolo[4,5-d]azepin-2-yl)phenyl]-3-methyl-2-imidazolidinone (E26)

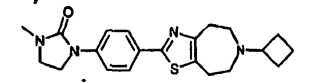

2-(4-Bromophenyl)-6-cyclobutyl-5,6,7,8-tetrahydro-4*H*-[1,3]thiazolo[4,5-*d*]azepine (may be prepared as described in Example 2) (45 mg, 0.12 mmol), 1-methyl-2-imidazolidinone (24 mg, 0.24 mmol), tris(dibenzylideneacetone)dipalladium (0) (6 mg, 0.006 mmol), 9,9-dimethyl-4,5-bis(diphenylphosphino)xanthene (10 mg, 0.018 mmol) and caesium carbonate (59 mg, 0.18 mmol) were added together in dioxane (2 ml) and the resulting mixture was heated under reflux under argon for 6 hours. The reaction mixture was allowed to cool to room temperature, diluted with methanol and passed down an SCX column eluting with methanol and 2M ammonia/methanol. The basic fractions were combined and evaporated *in vacuo* to afford the title compound (E26). MS (AP+) m/e 383 [M+H]⁺.

### Example 27

### 1-[4-(6-Cyclobutyl-5,6,7,8-tetrahydro-4H-[1,3]thiazolo[4,5-d]azepin-2-yl)phenyl]-2-imidazolidinone (E27)

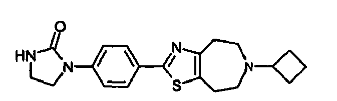

2-(4-Bromophenyl)-6-cyclobutyl-5,6,7,8-tetrahydro-4*H*-[1,3]thiazolo[4,5-*d*]azepine (may be prepared as described in Example 2) (130 mg, 0.36 mmol), 2-imidazolidinone (185 mg, 2.15 mmol), tris(dibenzylideneacetone)dipalladium (0) (16.5 mg, 0.018 mmol), 9,9-dimethyl-4,5-bis(diphenylphosphino)xanthene (31 mg, 0.054 mmol) and caesium carbonate (176 mg, 0.54 mmol) were added together in dioxane (5 ml) and the resulting mixture was heated under reflux under argon for 18 hours. The reaction mixture was allowed to cool to room temperature, diluted with methanol and passed down an SCX column eluting with methanol and 2M ammonia/methanol. The basic fractions were combined and evaporated *in vacuo.* The residue was purified using the mass directed autoprep to afford the title compound (E27). MS (AP+) m/e 369 [M+H]⁺.

### Example 28

### 1-[4-(6-Cyclopentyl-5,6,7,8-tetrahydro-4H-[1,3]thiazolo[4,5-d]azepin-2-yl)phenyl]-3-methyl-2-imidazolidinone (E28)

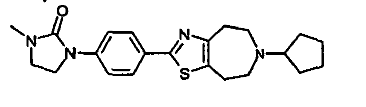

1-Methyl-3-[4-(5,6,7,8-tetrahydro-4*H*-[1,3]thiazolo[4,5-*d*]azepin-2-yl)phenyl]-2-imidazolidinone (may be prepared as described in Description 8) (21 mg, 0.06 mmol) was dissolved in dichloromethane (1 ml), treated with cyclopentanone (0.01 ml) and acetic acid (1 drop). The resulting mixture was stirred at room temperature under argon for 20 minutes. Sodium triacetoxyborohydride (25 mg, 0.12 mmol) was added and the mixture stirred for 18 hours. The reaction mixture was diluted with methanol and passed down an SCX column eluting with methanol and 2M ammonia/methanol. The basic fractions were combined and evaporated *in vacuo* to afford the title compound (E28). MS (AP+) m/e 397 [M+H]⁺.

### Examples 29-32 (E29-E32)

Examples 29 to 32 (E29-E32) may be prepared from 1-methyl-3-[4-(5,6,7,8-tetrahydro-4*H*-[1,3]thiazolo[4,5-d]azepin-2-yl)phenyl]-2-imidazolidinone (may be prepared as described in Description 8) and the appropriate carbonyl compound by an analogous process to that described in Example 28.

| Example | Carbonyl Compound | MS (AP+) |
|---|---|---|
| 1-Methyl-3-{4-[6-(1-methylethyl)-5,6,7,8-tetrahydro-4*H*-[1,3]thiazolo[4,5-*d*]azepin-2-yl]phenyl}-2-imidazolidinone (E29) | Acetone | m/e 371 [M+H]⁺. |
| 1-[4-(6-Cyclohexyl-5,6,7,8-tetrahydro-4*H*-[1,3]thiazolo[4,5-d]azepin-2-yl)phenyl]-3-methyl-2-imidazolidinone (E30) | Cyclohexanone | m/e 411 [M+H]⁺. |
| 1-{4-[6-(Cyclopropylmethyl)-5,6,7,8-tetrahydro-4*H*-[1,3]thiazolo[4,5-*d*]azepin-2-yl]phenyl}-3-methyl-2-imidazolidinone (E31) | Cyclopropanecarboxaldehyde | m/e 383 [M+H]⁺. |
| 1-Methyl-3-{4-[6-(2-methylpropyl)-5,6,7,8-tetrahydro-4*H*-[1,3]thiazolo[4,5-*d*]azepin-2-yl]phenyl}-2-imidazolidinone (E32) | 2-Methylpropanal | m/e 385 [M+H]⁺. |

### Example 33

### 1,1-Dimethylethyl 3-(6-cyclobutyl-5,6,7,8-tetrahydro-4H-[1,3]thiazolo[4,5-d]azepin-2-yl)-1-pyrrolidinecarboxylate (E33)

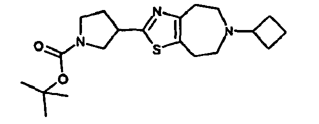

### Method A

A crude mixture of 5-bromo-1-cyclobutylhexahydro-4*H*-azepin-4-one (may be prepared as described in Description 2) (7.0mmol) and 1,1-dimethylethyl 3-(aminocarbonothioyl)-1-pyrrolidinecarboxylate (may be prepared as described in Description 9) (1.93g, 8.40mmol) in ethanol was heated under reflux for 18 hours. The solvent was removed *in vacuo* and the crude material purified using silica gel chromatography and an ion exchange cartridge (SCX) to afford the product (E33); MS (ES+) m/e 378 [M+H]⁺.

### Method B

Bromine (0.36ml, 7.00mmol) was added dropwise to a solution of 1-cyclobutylhexahydro-4*H*-azepin-4-one (may be prepared as described in Description 1) (1.17g, 7.00mmol) in dichloromethane (17.5ml) at 0°C. On completion of addition the mixture was allowed to warm to room temperature and was stirred for 1 h. The solvent was then evaporated and redissolved in ethanol (17.5ml), treated with 1,1-dimethylethyl 3-(aminocarbonothioyl)-1-pyrrolidinecarboxylate (may be prepared as described in Description 9) (1.93g, 8.40mmol) and heated at reflux for 18 hours. The solvent was removed *in vacuo* and the crude material purified using silica gel chromatography and an ion exchange cartridge (SCX) to afford the product; MS (ES+) m/e 378 [M+H]⁺.

### Example 34

### 6-Cyclobutyl-2-(3-pyrrolidinyl)-5,6,7,8-tetrahydro-4H-[1,3]thiazolo[4,5-d]azepine (E34)

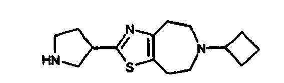

A solution of 1,1-dimethylethyl 3-(6-cyclobutyl-5,6,7,8-tetrahydro-4*H*-[1,3]thiazolo[4,5-*d*]azepin-2-yl)-1-pyrrolidinecarboxylate (may be prepared as described in Example 33) (930mg, 2.47mmol) in dichloromethane (20ml) was treated with trifluoroacetic acid (10ml). The resulting mixture was allowed to stir at room temperature for 45 minutes. The reaction was diluted with methanol and applied to an ion exchange cartridge (SCX), washed with methanol and then a 2M ammonia in methanol solution. The basic fractions were then reduced and purified using silica gel chromatography to afford the product (E34); ¹H NMR (CDCl₃) δ 1.55-1.75 (2H, m), 1.83-1.93 (2H,m), 1.96-2.03 (1H,m), 2.05-2.14 (2H,m), 2.22-2.31 (1H,m), 2.55-2.70 (5H,m), 2.82-2.87 (2H,m), 2.95-3.00 (3H,m), 3.02-3.11 (1 H,m), 3.13-3.20 (1 H,m), 3.26-3.32 (1 H,m), 3.50-3.58 (1 H,m).

### Example 35

### (±)-5-[3-(6-Cyclobutyl-5,6,7,8-tetrahydro-4H-[1,3]thiazolo[4,5-d]azepin-2-yl)-1-pyrrolidinyl]-2-pyridinecarbonitrile (E35)

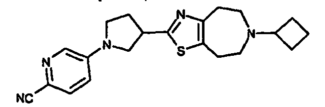

A mixture of 6-cyclobutyl-2-(3-pyrrolidinyl)-5,6,7,8-tetrahydro-4*H*-[1,3]thiazolo[4,5-*d*]azepine (may be prepared as described in Example 34) (70.0mg, 0.25mmol), 5-bromo-2-cyanopyridine (54.0mg, 0.30mmol), cesium carbonate (118mg, 0.35mmol), tris(dibenzylideneacetone)dipalladium(0) (12.0mg, 0.01mmol), and 4,5-bis(diphenylphosphino)-9,9-dimethylxanthene (24.0mg, 0.04mmol) in 1,4-dioxane (4ml) was heated under argon at 100°C for 16 hours. The reaction was then acidified and applied to an ion exchange cartridge (SCX) and washed with methanol and then a 2M ammonia in methanol solution. The basic fractions were then reduced and purified reverse phase chromatography to afford the product (E35); MS (ES+) m/e 380 [M+H]⁺.

### Example 36

### (-)-5-[3-(6-Cyclobutyl-5,6,7,8-tetrahydro-4H-[1,3]thiazolo[4,5-d]azepin-2-yl)-1-pyrrolidinyl]-2-pyridinecarbonitrile (E36)

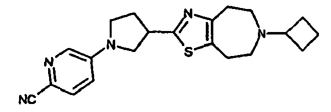

(-)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile (E36) was prepared by separating the enantiomers of (±)-5-[3-(6-cyclobutyl-5,6,7,8-tetrahydro-4*H*-[1,3]thiazolo[4,5-*d*]azepin-2-yl)-1-pyrrolidinyl]-2-pyridinecarbonitrile (may be prepared as described in Example 35) using chiral HPLC; MS (ES+) m/e 380 [M+H]⁺.

### Example 37

### (+)-5-[3-(6-Cyclobutyl-5,6,7,8-tetrahydro-4H-[1,3]thiazolo[4,5-d]azepin-2-yl)-1-pyrrolidinyl]-2-pyridinecarbonitrile (E37)

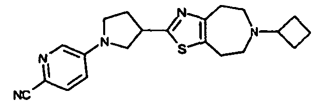

(+)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile (E37) was prepared by separating the enantiomers of (±)-5-[3-(6-cyclobutyl-5,6,7,8-tetrahydro-4*H*-[1,3]thiazolo[4,5-*d*]azepin-2-yl)-1-pyrrolidinyl]-2-pyridinecarbonitrile (may be prepared as described in Example 35) using chiral HPLC; MS (ES+) m/e 380 [M+H]⁺.

### Example 38

### (±)-6-Cyclobutyl-2-[1-(6-methyl-3-pyridinyl)-3-pyrrolidinyl]-5,6,7,8-tetrahydro-4H-[1,3]thiazolo[4,5-d]azepine (E38)

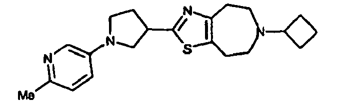

A mixture of 6-cyclobutyl-2-(3-pyrrolidinyl)-5,6,7,8-tetrahydro-4*H*-[1,3]thiazolo[4,5-*d*]azepine (may be prepared as described in Example 34) (48.0mg, 0.17mmol), 5-bromo-2-methylpyridine (44.0mg, 0.26mmol), sodium *tert*-butoxide (33.0g, 0.34mmol), palladium acetate (4.00mg, 0.02mmol), and 1,1'-binaphthalene-2,2'-diylbis(diphenylphosphane) (BINAP) (22.0mg, 0.03mmol) in 1,4-dioxane (3ml) was heated under argon at reflux for 16 hours. The reaction was then acidified and applied to an ion exchange cartridge (SCX) and washed with methanol and then a 2M ammonia in methanol solution. The basic fractions were then reduced and purified using reverse phase chromatography to afford the product (E38); MS (ES+) m/e 369 [M+H]⁺.

### Example 39

### 5-[3-(6-Cyclobutyl-5,6,7,8-tetrahydro-4H-[1,3]thiazolo[4,5-d]azepin-2-yl)-1-pyrrolidinyl]-3-pyridinecarbonitrile (E39)

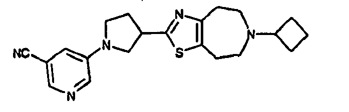

5-[3-(6-Cyclobutyl-5,6,7,8-tetrahydro-4*H*-[1,3]thiazolo[4,5-*d*]azepin-2-yl)-1-pyrrolidinyl]-3-pyridinecarbonitrile (E39) was prepared from 6-cyclobutyl-2-(3-pyrrolidinyl)-5,6,7,8-tetrahydro-4*H*-[1,3]thiazolo[4,5-*d*]azepine (maybe prepared as described in Example 34) and 5-bromo-3-pyridinecarbonitrile using an analogous process to that described in example 38; MS (ES+) m/e 380 [M+H]⁺.

### Example 40

### Methyl 4-(6-cyclobutyl-5,6,7,8-tetrahydro-4H-[1,3]thiazolo[4,5-d]azepin-2-yl)benzoate (E40)

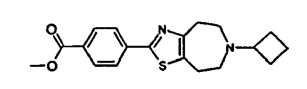

### Method A

1-Cyclobutylhexahydro-4*H*-azepin-4-one (may be prepared as described in Description 1, method B) (300mg, 1.80mmol) was dissolved in acetic acid (3ml), treated with bromine (0.09ml, 1.80mmol) and heated at 60°C under argon for 1.5 hours. The mixture was allowed to cool to room temperature, evaporated under reduced pressure and azeotroped with toluene. The crude product was dissolved in ethanol (3ml), treated with methyl 4-(aminocarbonothioyl)benzoate (may be prepared as described in WO 2005011685) (280mg, 1.44mmol) and heated under reflux under argon for 3 hours. The mixture was allowed to cool to room temperature and left to stand overnight. The resulting solid was collected by filtration and discarded. The filtrate was diluted with methanol and applied to an ion exchange cartridge (SCX) and washed with methanol and then a 2M ammonia in methanol solution. The basic fractions were combined and evaporated and the residue purified by column chromatography eluting with a mixture of 2M ammonia/methanol and dichloromethane (2:98) to afford the product (E40); MS (ES+) m/e 343 [M+H]⁺.

### Method B

1-Cyclobutylhexahydro-4*H*-azepin-4-one (may be prepared as described in Description 1, method B) (400mg, 2.40mmol) was dissolved in acetic acid (3ml), treated with bromine (0.12ml, 2.40mmol) and heated at 60°C under argon for 1 hour. The mixture was allowed to cool to room temperature, evaporated under reduced pressure and azeotroped with toluene (x2). The crude product was dissolved in ethanol (3ml), treated with methyl 4-(aminocarbonothioyl)benzoate (may be prepared as described in WO 2005011685) (561 mg, 2.90mmol) and heated under reflux under argon for 18 hours. The mixture was allowed to cool to room temperature, diluted with methanol and applied to an ion exchange cartridge (SCX) and washed with methanol and then a 2M ammonia in methanol solution. The basic fractions were combined and evaporated and the residue purified by column chromatography eluting with a mixture of 2M ammonia/methanol and dichloromethane (2:98) to afford the product (E40); MS (ES+) m/e 343 [M+H]⁺.

### Method C

1-Cyclobutylhexahydro-4*H*-azepin-4-one (may be prepared as described in Description 1, method B) (346mg, 2.07mmol) was dissolved in dichloromethane (3ml), treated with bromine (0.11 ml, 2.07mmol) and stirred at room temperature under argon for 2.5 hours. The solvent was evaporated under reduced pressure to give a light brown solid. The crude product was suspended in ethanol (3ml), treated with methyl 4-(aminocarbonothioyl)benzoate (may be prepared as described in WO 2005011685) (485mg, 2.49mmol) and heated under reflux for 18 hours. The mixture was allowed to cool to room temperature and the resulting solid was collected by filtration and discarded. The filtrate was diluted with methanol and applied to an ion exchange cartridge (SCX) and washed with methanol and then a 2M ammonia in methanol solution. The basic fractions were combined and evaporated and the residue purified by column chromatography eluting with a mixture of 2M ammonia/methanol and dichloromethane (2:98) to afford the product (E40); MS (ES+) m/e 343 [M+H]⁺.

### Example 41

### 4-(6-Cyclobutyl-5,6,7,8 tetrahydro-4H-[1,3]thiazolo[4,5-d]azepin-2-yl)benzoic acid (E41)

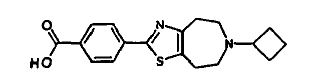

Methyl 4-(6-cyclobutyl-5,6,7,8-tetrahydro-4*H*-[1,3]thiazolo[4,5-*d*]azepin-2-yl)benzoate (may be prepared as described in Example 40) (67mg, 0.20mmol) was dissolved in ethanol (1 ml), treated with 2M sodium hydroxide solution (0.3ml, 0.60mmol) and stirred at room temperature for 3 hours. The mixture was diluted with methanol and applied to an ion exchange cartridge (SCX) and washed with methanol and then a 2M ammonia in methanol solution. The basic fractions were combined and evaporated to afford the product (E41); MS (ES+) m/e 329 [M+H]⁺.

### Example 42

### 6-Cyclobutyl-2-phenyl-5,6,7,8-tetrahydro-4H-[1,3]thiazolo[4,5-d]azepine (E42)

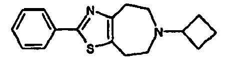

2-(4-Bromophenyl)-6-cyclobutyl-5,6,7,8-tetrahydro-4*H*-[1,3]thiazolo[4,5-*d*]azepine (may be prepared as described in Example 2) (43mg, 0.12mmol), (3,5-dimethyl-4-isoxazolyl)boronic acid (20mg, 0.14mmol), tetrakis(triphenylphosphine)palladium (0) (7mg, 0.006mmol) and 0.5M aqueous sodium carbonate solution (1ml) were added together in ethanol (1 ml) and the resulting mixture was heated under reflux under argon for 18 hours. The mixture was allowed to cool to room temperature, acidified with 2M hydrochloric acid solution, applied to an ion exchange cartridge (SCX) and washed with methanol and then a 2M ammonia in methanol solution. The basic fractions were combined and evaporated and the residue purified using reverse phase chromatography to afford the product (E42); MS (ES+) m/e 285 [M+H]⁺.

### Example 43

### 4-(6-Cyclobutyl-5,6,7,8-tetrahydro-4H-[1,3]thiazolo[4,5-d]azepin-2-yl)-N methylbenzamide (E43)

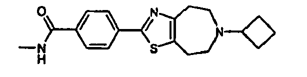

1-Cyclobutylhexahydro-4*H*-azepin-4-one (may be prepared as described in Description 1, method B) (220mg, 1.32mmol) was dissolved in acetic acid (2ml), treated with bromine (0.07ml, 1.32mmol) and stirred at room temperature under argon for 6 hours. The mixture was evaporated and azeotroped with toluene. The crude product was dissolved in ethanol (2ml), treated with 4-(aminocarbonothioyl)-N-methylbenzamide (may be prepared as described in WO 2002046186) (384mg, 1.98mmol) and heated under reflux for 18 hours. The mixture was allowed to cool to room temperature, the resulting solid removed by filtration and the filtrate applied to an ion exchange cartridge (SCX) and washed with methanol and then a 2M ammonia in methanol solution. The basic fractions were combined and evaporated and the residue purified by column chromatography eluting with a mixture of 2M ammonia/methanol and dichloromethane (4:96). The product was further purified using reverse phase chromatography to afford the product (E43); MS (ES+) m/e 342 [M+H]⁺.

### Example 44

### 3-[4-(6-Cyclobutyl-5,6,7,8-tetrahydro-4H-[1,3]thiazolo[4,5-d]azepin-2-yl)phenyl]-1,3-oxazolidin-2-one (E44)

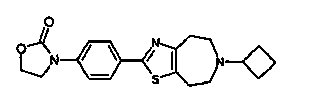

3-[4-(6-Cyclobutyl-5,6,7,8-tetrahydro-4*H*-[1,3]thiazolo[4,5-*d*]azepin-2-yl)phenyl]-1,3-oxazolidin-2-one(E44) was prepared from 2-(4-bromophenyl)-6-cyclobutyl-5,6,7,8-tetrahydro-4*H*-[1,3]thiazolo[4,5-*d*]azepine (may be prepared as described in Example 2, method B) and 1,3-oxazolidin-2-one using an analogous process to that described in example 26; MS (ES+) m/e 370 [M+H]⁺.

### Example 45

### 6-Cyclobutyl-2-[4-(1-pyrrolidinylcarbonyl)phenyl]-5,6,7,8-tetrahydro-4H-[1,3]thiazolo[4,5-d]azepine (E45)

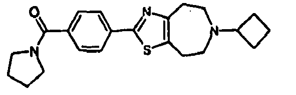

4-(6-Cyclobutyl-5,6,7,8-tetrahydro-4*H*-[1,3]thiazolo[4,5-*d*]azepin-2-yl)benzoic acid (may be prepared as described in Example 41) (52mg, 0.16mmol) was dissolved in dichloromethane (2ml), treated with N,N'-carbonyldiimidazole (78mg, 0.48mmol) and heated at 40°C under argon for 2 hours. The mixture was allowed to cool to room temperature. Pyrrolidine (0.08ml, 0.96mmol) was added and the mixture stirred at room temperature under argon for 1.5 hours. The mixture was applied directly to a silica column and purified by column chromatography eluting with a mixture of 2M ammonia/methanol and dichloromethane (3:97) to afford the product (E45); MS (ES+) m/e 382 [M+H]⁺.

### Example 46

### 4-(6-Cyclobutyl-5,6,7,8-tetrahydro-4H-[1,3]thiazolo[4,5-d]azepin-2-yl)benzamide (E46)

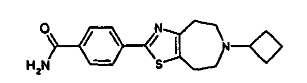

4-(6-Cyclobutyl-5,6,7,8-tetrahydro-4*H*-[1,3]thiazolo[4,5-*d*]azepin-2-yl)benzoic acid (may be prepared as described in Example 41) (74mg, 0.23mmol) was dissolved in dichloromethane (3ml), treated with N,N'-carbonyldiimidazole (118mg, 0.69mmol) and heated at 40°C under argon for 2 hours. The mixture was allowed to cool to room temperature. 0.88 Ammonia solution (0.1ml) was added and the mixture stirred at room temperature under argon for 18 hours. The solid was filtered and dissolved in a mixture of dichloromethane/methanol. The mixture was purified by column chromatography eluting with a mixture of 2M ammonia/methanol and dichloromethane (7:93). The product was dissolved in methanol and applied to an ion exchange cartridge (SCX) and washed with methanol and then a 2M ammonia in methanol solution. The basic fractions were combined and evaporated to afford the product (E46); MS (ES+) m/e 328 [M+H]⁺.

### Example 47

### 4-(6-Cyclobutyl-5,6,7,8-tetrahydro-4H-[1,3]thiazolo[4,5-d]azepin-2-yl)-N-ethylbenzamide (E47)

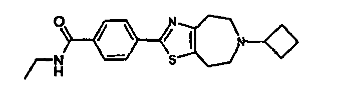

4-(6-Cyclobutyl-5,6,7,8-tetrahydro-4*H*-[1,3]thiazolo[4,5-*d*]azepin-2-yl)benzoic acid (may be prepared as described in Example 41) (146mg, 0.45mmol) was dissolved in dichloromethane (3ml), treated with N,N'-carbonyldiimidazole (219mg, 1.35mmol) and heated under reflux for 2 hours. The mixture was allowed to cool to room temperature. Ethylamine hydrochloride (220mg, 2.70mmol) and triethylamine (0.38ml, 2.70mmol) were added and the mixture stirred at room temperature under argon for 18 hours. The mixture was applied directly to a silica column and purified by column chromatography eluting with a mixture of 2M ammonia/methanol and dichloromethane (2:98) to afford the product (E47); MS (ES+) m/e 356 [M+H]⁺.

### Example 48

### 4-(6-Cyclobutyl-5,6,7,8-tetrahydro-4H-[1,3]thiazolo[4,5-d]azepin-2-yl)benzonitrile (E48)

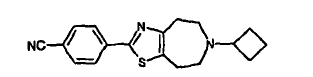

4-(5,6,7,8-Tetrahydro-4*H*-[1,3]thiazolo[4,5-*d*]azepin-2-yl)benzonitrile (may be prepared as described in Description 10) (29mg, 0.11mmol) was dissolved in dichloromethane (2ml), treated with cyclobutanone (0.02ml, 0.22mmol), sodium triacetoxyborohydride (47mg, 0.22mmol) and acetic acid (1 drop) and the resulting mixture was stirred at room temperature under argon for 2 hours. The mixture was diluted with methanol and applied to an ion exchange cartridge (SCX) and washed with methanol and then a 2M ammonia in methanol solution. The basic fractions were combined and evaporated to afford the product (E48); MS (ES+) m/e 310 [M+H]⁺.

### Example 49

### 5-(6-Cyclobutyl-5,6,7,8-tetrahydro-4H-[1,3]thiazolo[4,5-d]azepin-2-yl)-2-pyridinecarbonitrile (E49)

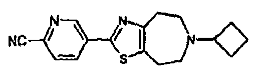

### Method A

6-Cyclobutyl-2-iodo-5,6,7,8-tetrahydro-4*H*-[1,3]thiazolo[4,5-*d*]azepine (may be prepared as described in Description 12) (117mg, 0.35mmol), 5-(trimethylstannanyl)-2-pyridinecarbonitrile (may be prepared as described in Description 13) (112mg, 0.42mmol) and bis(triphenylphosphine)palladium (II) chloride (37mg, 0.05mmol) were added together in dioxane (3ml) and the resulting mixture was heated under reflux under argon for 54 hours. The mixture was allowed to cool to room temperature, diluted with methanol and applied to an ion exchange cartridge (SCX) and washed with methanol and then a 2M ammonia in methanol solution. The basic fractions were combined and evaporated and the residue was purified by column chromatography eluting with a mixture of 2M ammonia/methanol and dichloromethane (3:97) to afford the product (E49); MS (ES+) m/e 311 [M+H]⁺.

### Method B

6-Cyclobutyl-2-iodo-5,6,7,8-tetrahydro-4*H*-[1,3]thiazolo[4,5-*d*]azepine (may be prepared as described in Description 12) (100mg, 0.30mmol), 5-(trimethylstannanyl)-2-pyridinecarbonitrile (may be prepared as described in Description 13) (96mg, 0.36mmol) and bis(triphenylphosphine)palladium (II) chloride (21mg, 0.03mmol) were added together in dioxane (2ml) and the resulting mixture was heated under reflux under argon for 18 hours. The mixture was allowed to cool to room temperature, bis(triphenylphosphine)palladium (II) chloride (21 mg, 0.03mmol) was added and the resulting mixture was heated under reflux under argon for 18 hours. The mixture was allowed to cool to room temperature, diluted with methanol and applied to an ion exchange cartridge (SCX) and washed with methanol and then a 2M ammonia in methanol solution. The basic fractions were combined and evaporated and the residue was purified by column chromatography eluting with a mixture of 2M ammonia/methanol and dichloromethane (3:97) to afford the product (E49); MS (ES+) m/e 311 [M+H]⁺.

### Example 50

### 5-(6-Cyclobutyl-5,6,7,8-tetrahydro-4H-[1,3]thiazolo[4,5-d]azepin-2-yl)-2-pyridinecarboxylic acid (E50)

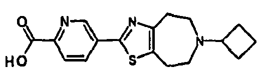

5-(6-Cyclobutyl-5,6,7,8-tetrahydro-4*H*-[1,3]thiazolo[4,5-*d*]azepin-2-yl)-2-pyridinecarbonitrile (may be prepared as described in Example 49, method B) (39mg, 0.13mmol) was dissolved in ethanol (1ml), treated with 10% sodium hydroxide solution (1 ml) and heated under reflux for 1 hour. The mixture was allowed to cool to room temperature, diluted with methanol and applied to an ion exchange cartridge (SCX) and washed with methanol and then a 2M ammonia in methanol solution. The basic fractions were combined and evaporated to afford the product (E50); MS (ES+) m/e 330 [M+H]⁺.

### Example 51

### 5-(6-Cyclobutyl-5,6,7,8-tetrahydro-4H-[1,3]thiazolo[4,5-d]azepin-2-yl)-N-methyl-2-pyridinecarboxamide (E51)

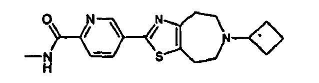

5-(6-Cyclobutyl-5,6,7,8-tetrahydro-4*H*-[1,3]thiazolo[4,5-*d*]azepin-2-yl)-2-pyridinecarboxylic acid (may be prepared as described in Example 50) (40mg, 0.12mmol) was dissolved in dichloromethane (1 ml), treated with N,N'-carbonyldiimidazole (39mg, 0.24mmol) and heated under reflux under argon for 2 hours. The mixture was allowed to cool to room temperature and stirred for 54 hours. 2M methylamine solution in tetrahydrofuran (0.24ml, 0.48mmol) was added and the mixture stirred at room temperature under argon for 1 hour. The mixture was applied directly to a silica column and purified by column chromatography eluting with a mixture of 2M ammonia/methanol and dichloromethane (2:98) to afford the product (E51); MS (ES+) m/e 343 [M+H]⁺.

### Example 52

### 1-[5-(6-Cyclobutyl-5,6,7,8-tetrahydro-4H-[1,3]thiazolo[4,5-d]azepin-2-yl)-2-pyridinyl]-2-pyrrolidinone (E52)

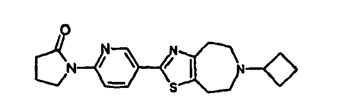

1-[5-(6-Cyclobutyl-5,6,7,8-tetrahydro-4*H*-[1,3]thiazolo[4,5-*d*]azepin-2-yl)-2-pyridinyl]-2-pyrrolidinone (E52) was prepared from 1-[5-(5,6,7,8-tetrahydro-4*H*-[1,3]thiazolo[4,5-*d*]azepin-2-yl)-2-pyridinyl]-2-pyrrolidinone (may be prepared as described in Description 18) and cyclobutanone using an analogous process to that described in Description 12; MS (ES+) m/e 369 [M+H]⁺.

### Example 53

### 1-[5-(6-Cyclobutyl-5,6,7,8-tetrahydro-4H-[1,3]thiazolo[4,5-d]azepin-2-yl)-2-pyridinyl]-3-methyl-2-imidazolidinone (E53)

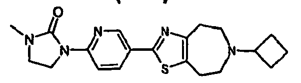

1-[5-(6-Cyclobutyl-5,6,7,8-tetrahydro-4*H*-[1,3]thiazolo[4,5-*d*]azepin-2-yl)-2-pyridinyl]-3-methyl-2-imidazolidinone (E53) was prepared from 1-methyl-3-[5-(5,6,7,8-tetrahydro-4*H-*[1,3]thiazolo[4,5-*d*]azepin-2-yl)-2-pyridinyl]-2-imidazolidinone (may be prepared as described in Description 20) and cyclobutanone using an analogous process to that described in Description 12; MS (ES+) m/e 384 [M+H]⁺.

### Example 54

### 2-(6-Chloro-3-pyridinyl)-6-cyclobutyl-5,6,7,8-tetrahydro-4H-[1,3]thiazolo[4,5-d]azepine (E54)

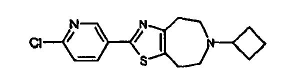

### Method A

2-(6-Chloro-3-pyridinyl)-6-cyclobutyl-5,6,7,8-tetrahydro-4*H*-[1,3]thiazolo[4,5-*d*]azepine (E54) was prepared from 2-(6-chloro-3-pyridinyl)-5,6,7,8-tetrahydro-4*H*-[1,3]thiazolo[4,5-*d*]azepine (may be prepared as described in Description 21, method A) and cyclobutanone using an analogous process to that described in Description 12; MS (ES+) m/e 320 [M+H]⁺.

### Method B

2-(6-Chloro-3-pyridinyl)-5,6,7,8-tetrahydro-4*H*-[1,3]thiazolo[4,5-*d*]azepine (may be prepared as described in Description 21, method B) (60.0mg, 0.23mmol) was dissolved in dichloromethane (3ml) and treated with acetic acid (1 drop) and cyclobutanone (0.034ml, 0.46mmol). The mixture was stirred at room temperature under argon for 10 minutes and sodium triacetoxyborohydride (98.0mg, 0.46mmol) was added. After stirring at room temperature for 1 hour the mixture was diluted with methanol and passed down an ion exchange cartridge (SCX) and washed with methanol and then a 2M ammonia in methanol solution. The basic fractions were then reduced to afford the product (E54); MS (ES+) m/e 320 [M+H]⁺.

### Method C

To a suspension of 2-(6-chloro-3-pyridinyl)-5,6,7,8-tetrahydro-4H-[1,3]thiazolo[4,5-d]azepine (maybe prepared as described in Description 21, method C) (125mg, 0.472mmol) in dichloromethane (5mL) was added 3 drops of acetic acid, cyclobutanone (53 uL, 0.71 mmol), Sodium triacetoxyborohydride (150mg, 0.71 mmol). The resulting mixture was allowed to stir at room temperature for 1 hour. Reaction mixture was acidified with 2M Hydrochloric acid and applied to an ion exchange cartridge (SCX), washed with methanol and then a 2M ammonia in methanol solution. The basic fractions were then evaporated *in vacuo* to afford the product (E54); MS (ES+) m/e 320 [M+H]⁺.

### Example 55

### 6-Cyclobutyl-2-[6-(1-piperidinyl)-3-pyridinyl]-5,6,7,8-tetrahydro-4H-[1,3]thiazolo[4,5-d]azepine (E55)

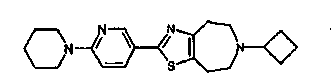

2-(6-Chloro-3-pyridinyl)-6-cyclobutyl-5,6,7,8-tetrahydro-4*H*-[1,3]thiazolo[4,5-*d*]azepine (may be prepared as described in Example 54) (26mg, 0.08mmol) was dissolved in toluene (2ml), treated with piperidine (0.02ml, 0.16mmol) and heated under reflux under argon for 18 hours. Piperidine (0.02ml, 0.16mmol) was added and the mixture was heated under reflux under argon for 18 hours. The mixture was allowed to cool to room temperature and the solvent removed under reduced pressure. The residue was dissolved in toluene, treated with piperidine (0.02ml, 0.16mmol) and heated in a microwave reactor at 150°C for 20 minutes. The mixture was heated in a microwave reactor for a further 20 minutes at 120°C. The solvent was removed under reduced pressure and the residue dissolved in piperidine (2ml) and heated under reflux for 18 hours. The mixture was allowed to cool to room temperature and the solvent removed under reduced pressure. The residue was purified by column chromatography eluting with a mixture of 2M ammonia/methanol and dichloromethane (2:98) to afford the product (E55); MS (ES+) m/e 369 [M+H]⁺.

### Examples 56 and 57

### 5-(6-Cyclobutyl-5,6,7,8-tetrahydro-4H-[1,3]thiazolo[4,5-d]azepin-2-yl)-2-pyrimidinecarbonitrile (E56) and 6-cyclobutyl-2-[2-(methyloxy)-5-pyrimidinyl]-5,6,7,8-tetrahydro-4H-[1,3]thiazolo[4,5-d]azepine (E57)

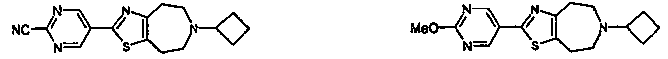

6-Cyclobutyl-2-iodo-5,6,7,8-tetrahydro-4*H*-[1,3]thiazolo[4,5-*d*]azepine (may be prepared as described in Description 12) (167mg, 0.50mmol), 5-(trimethylstannanyl)-2-pyrimidinecarbonitrile (may be prepared as described in WO 20040434358) (174mg, 0.65mmol) and bis(triphenylphosphine)palladium (II) chloride (53mg, 0.075mmol) were added together in dioxane (5ml) and the resulting mixture was heated under reflux under argon for 7 hours. Bis(triphenylphosphine)palladium (II) chloride (18mg, 0.026mmol) was added and the mixture was heated under reflux under argon for 18 hours. The mixture was allowed to cool to room temperature, diluted with methanol and applied to an ion exchange cartridge (SCX) and washed with methanol and then a 2M ammonia in methanol solution. The basic fractions were combined and evaporated. The residue was purified by column chromatography eluting with a mixture of 2M ammonia/methanol and dichloromethane (3:97). The product was further purified by column chromatography eluting with a mixture of 2M ammonia/methanol and dichloromethane (2:98) to afford the products E56; MS (ES+) m/e 312 [M+H]⁺ and E57; MS (ES+) m/e 317 [M+H]⁺.

### Example 58

### 6-Cyclobutyl-2-(5-pyrimidinyl)-5,6,7,8-tetrahydro-4H-[1,3]thiazolo[4,5-d]azepine (E58)

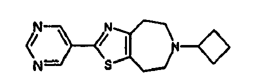

5-(6-Cyclobutyl-5,6,7,8-tetrahydro-4*H*-[1,3]thiazolo[4,5-*d*]azepin-2-yl)-2-pyrimidinecarbonitrile (may be prepared as described in Example 56) (35mg, 0.11 mmol) was dissolved in formic acid (0.7ml), treated dropwise with concentrated sulfuric acid (0.7ml) and stirred at room temperature for 30 minutes. The mixture was heated under reflux for 1 hour and allowed to cool to room temperature. The mixture was diluted with methanol and applied to an ion exchange cartridge (SCX) and washed with methanol and then a 2M ammonia in methanol solution. The basic fractions were combined and evaporated. The residue was purified by column chromatography eluting with a mixture of 2M ammonia/methanol and dichloromethane (3:97) to afford the product (E58); MS (ES+) m/e 287 [M+H]⁺.

### Example 59

### 5-(6-Cyclobutyl-5,6,7,8-tetrahydro-4H-[1,3]thiazolo[4,5-d]azepin-2-yl)-2(1H)-pyrimidinone (E59)

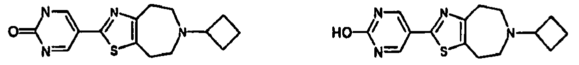

6-Cyclobutyl-2-[2-(methyloxy)-5-pyrimidinyl]-5,6,7,8-tetrahydro-4*H*-[1,3]thiazolo[4,5-*d*]azepine (may be prepared as described in Example 57) was dissolved in dichloromethane (1ml), cooled in an ice batch and treated with a 1M solution of boron tribromide in dichloromethane (0.40ml, 0.40mmol) dropwise. The mixture was stirred for 20 minutes, allowed to warm to room temperature and stirred for 1 hour. The mixture was heated under reflux for 2 hours, allowed to cool to room temperature and a 1 M solution of boron tribromide in dichloromethane (0.40ml, 0.40mmol) was added and the mixture heated under reflux for 2 hours. The mixture was left to stand overnight at room temperature. The mixture was diluted with methanol and applied to an ion exchange cartridge (SCX) and washed with methanol and then a 2M ammonia in methanol solution. The basic fractions were combined and evaporated. The residue was dissolved in 5M hydrochloric acid solution (2ml) and stirred at room temperature for 2 hours. The mixture was heated at 50°C for 1 hour, allowed to cool to room temperature, diluted with methanol and applied to an ion exchange cartridge (SCX) and washed with methanol and then a 2M ammonia in methanol solution. The basic fractions were combined and evaporated to afford the product (E59); MS (ES+) m/e 303 [M+H]⁺.

### Example 60

### 2-(2-Chloro-5-pyrimidinyl)-6-cyclobutyl-5,6,7,8-tetrahydro-4H-[1,3]thiazolo[4,5-d]azepine (E60)

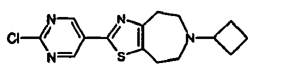

5-(6-Cyclobutyl-5,6,7,8-tetrahydro-4*H*-[1,3]thiazolo[4,5-*d*]azepin-2-yl)-2(1*H*)-pyrimidinone (may be prepared as described in Example 59) (30mg, 0.1mmol) was suspended in phosphorus oxychloride (0.5ml) and heated under reflux under argon for 7 hours. The mixture was allowed to cool to room temperature and left to stand overnight. The mixture was evaporated to afford the product (E60); MS (ES+) m/e 321 [M+H]⁺.

### Example 61

### 6-Cyclobutyl-2-[2-(1-piperidinyl)-5-pyrimidinyl]-5,6,7,8-tetrahydro-4H-[1,3]thiazolo[4,5-d]azepine (E61)

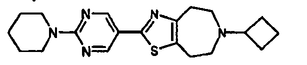

2-(2-Chloro-5-pyrimidinyl)-6-cyclobutyl-5,6,7,8-tetrahydro-4*H*-[1,3]thiazolo[4,5-*d*]azepine (may be prepared as described in Example 60) (31mg, 0.1 mmol) was dissolved in piperidine (2ml) and heated under reflux for 18 hours. The mixture was allowed to cool to room temperature and the solvent removed under reduced pressure. The residue was purified by column chromatography eluting with a mixture of 2M ammonia/methanol and dichloromethane (2:98) to afford the product (E61); MS (ES+) m/e 370 [M+H]⁺.

### Example 62

### 1-{4-[6-(Cyclohexylmethyl)-5,6,7,8-tetrahydro-4H-[1,3]thiazolo[4,5-d]azepin-2-yl]phenyl}-3-methyl-2-imidazolidinone (E62)

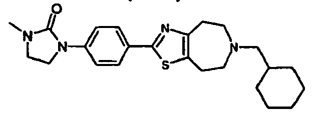

1-{4-[6-(Cyclohexylmethyl)-5,6,7,8-tetrahydro-4*H*-[1,3]thiazolo[4,5-*d*]azepin-2-yl]phenyl}-3-methyl-2-imidazolidinone (E62) may be prepared from 1-methyl-3-[4-(5,6,7,8-tetrahydro-4*H*-[1,3]thiazolo[4,5-*d*]azepin-2-yl)phenyl]-2-imidazolidinone (may be prepared as described in Description 8) and cyclohexanecarboxaldehyde by an analogous process to that described in Example 28; MS (ES+) m/e 425 [M+H]⁺.

### Example 63

### 1-[4-(6-Ethyl-5,6,7,8-tetrahydro-4H-[1,3]thiazolo[4,5-d]azepin-2-yl)phenyl]-3-methyl-2-imidazolidinone (E63)

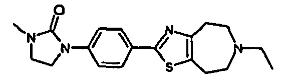

1-Methyl-3-[4-(5,6,7,8-tetrahydro-4*H*-[1,3]thiazolo[4,5-*d*]azepin-2-yl)phenyl]-2-imidazolidinone (may be prepared as described in Description 8) (20mg, 0.06mmol) was dissolved in ethanol (2ml), treated with potassium carbonate (12mg, 0.09mmol) and iodoethane (0.005ml, 0.06mmol) and heated under reflux under argon for 6 hours. Iodoethane (0.001ml, 0.012mmol) and potassium carbonate (1 2mg, 0.09mmol) were added and the mixture heated under reflux for 18 hours. Iodoethane (0.005ml, 0.06mmol) and potassium carbonate (12mg, 0.09mmol) were added and the mixture heated under reflux for 5 hours. The mixture was allowed to cool to room temperature and left to stand overnight. The mixture was diluted with methanol and applied to an ion exchange cartridge (SCX) and washed with methanol and then a 2M ammonia in methanol solution. The basic fractions were combined and evaporated to afford the product (E63); MS (ES+) m/e 357 [M+H]⁺.

### Example 64

### 6-Cyclobutyl-2-{1-[(6-methyl-3-pyridazinyl)carbonyl]-4-piperidinyl}-5,6,7,8-tetrahydro-4H-[1,3]thiazolo[4,5-d]-azepine (E64)

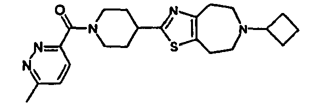

6-Methyl-3-pyridazinecarboxylic acid (may be prepared as described in J. Heterocyclic Chem, 1992, 29, 1, 93-5) (47mg, 0.34mmol), N-cyclohexylcarbodiimide, N'-methyl polystyrene (2.1 mmol/g) (162mg, 0.34mmol) and 1-hydroxybenzotriazole (46mg, 0.34mmol) were suspended in dimethylformamide (5ml) and stirred at room temperature under argon for 30 minutes. 6-Cyclobutyl-2-(4-piperidinyl)-5,6,7,8-tetrahydro-4*H-*[1,3]thiazolo[4,5-*d*]azepine (may be prepared as described in Example 7) (50mg, 0.17mmol) was added and the resulting mixture stirred at room temperature under argon for 1.5 hours. The reaction was then diluted with methanol and applied to an ion exchange cartridge (SCX) and washed with methanol and then a 2M ammonia in methanol solution. The basic fractions were then reduced and purified by column chromatography eluting with a mixture of 2M ammonia/methanol and dichloromethane (3:97 to 5:95) to afford the product (E64); MS (ES+) m/e 412 [M+H]⁺.

### Example 65

### 6-Cyclobutyl-2-{1-[(6-methyl-3-pyridinyl)carbonyl]-3-pyrrolidinyl}-5,6,7,8-tetrahydro-4H-[1,3]thiazolo[4,5-d]azepine (E65)

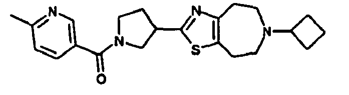

A suspension of 6-methyl-3-pyridinecarboxylic acid (33.0mg, 0.24mmol), 1*H*-1,2,3-benzotriazol-1-ol (33.0mg, 0.24mmol), and N-cyclohexylcarbodiimide, N'-methyl polystyrene (2.1 mmol/g) (114mg, 0.24mmol) in dimethylformamide (3ml) was stirred at room temperature for approximately 30 minutes. After this time a solution of 6-cyclobutyl-2-(3-pyrrolidinyl)-5,6,7,8-tetrahydro-4*H*-[1,3]thiazolo[4,5-*d*]azepine (may be prepared as described in Example 34) (33.0mg, 0.12mmol) in dimethylformamide (1ml) was added and the reaction mixture stirred at room temperature overnight. The reaction mixture was passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-4%) to afford the product (E65); MS (ES+) m/e 397 [M+H]⁺.

### Example 66

### 5-[4-(6-Cyclobutyl-5,6,7,8-tetrahydro-4H-[1,3]thiazolo[4,5-d]azepin-2-yl)-1-piperidinyl]-3-pyridinecarbonitrile (E66)

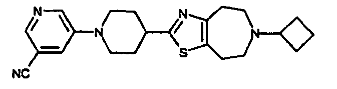

A mixture of 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol), 5-bromo-3-pyridinecarbonitrile (37.0mg, 0.20mmol), cesium carbonate (78.0mg, 0.24mmol), tris(dibenzylideneacetone)dipalladium(0) (8.00mg, 0.009mmol) and 9,9-dimethyl-4,5-bis(diphenylphosphino)xanthene (17.0 mg, 0.03 mmol) in dioxan (2ml) was heated at 100 °C overnight. The mixture was passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-5%) to afford the product (E66); MS (ES+) m/e 394 [M+H]⁺.

### Example 67

### 4-[4-(6-Cyclobutyl-5,6,7,8-tetrahydro-4H-[1,3]thiazolo[4,5-d]azepin-2-yl)-1-piperidinyl]benzonitrile (E67)

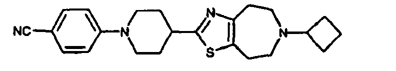

4-[4-(6-Cyclobutyl-5,6,7,8-tetrahydro-4*H*-[1,3]thiazolo[4,5-*d*]azepin-2-yl)-1-piperidinyl]benzonitrile (E67) was prepared from 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) and 4-bromobenzonitrile (38.0mg, 0.20mmol) using an analogous process to that described in Example 66; MS (ES+) m/e 393 [M+H]⁺.

### Example 68

### 4-(6-Cyclobutyl-5,6,7,8-tetrahydro-4H-[1,3]thiazolo[4,5-d]azepin-2-yl)-N-methyl-1-piperidinecarboxamide (E68)

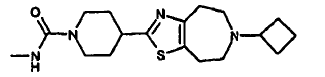

A solution of 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) in dichloromethane (1ml) was added dropwise to phosgene (20% solution in toluene) (0.27ml, 0.51 mmol). The resulting suspension was allowed to stir at room temperature for 90 minutes. After this time the mixture was evaporated, redissolved in dichloromethane (1ml) and treated with triethylamine (24.0µl, 0.17mmol), then added to methylamine (40% solution in water) (29µl, 0.34mmol). The mixture was allowed to stir at room temperature over the weekend, then passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-6%), and repurified using MDAP to afford the product (E68); MS (ES+) m/e 349 [M+H]⁺.

### Example 69

### 4-(6-Cyclobutyl-5,6,7,8-tetrahydro-4H-[1,3]thiazolo[4,5-d]azepin-2-yl)-N-(1-methylethyl)-1-piperidinecarboxamide (E69)

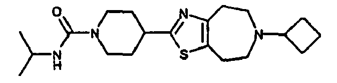

4-(6-Cyclobutyl-5,6,7,8-tetrahydro-4*H*-[1,3]thiazolo[4,5-*d*]azepin-2-yl)-*N*-(1-methylethyl)-1-piperidinecarboxamide (E69) was prepared from 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) and isopropylamine (29µl, 0.34mmol) using an analogous process to that described in Example 68; MS (ES+) m/e 377 [M+H]⁺.

### Example 70

### 4-(6-Cyclobutyl-5,6,7,8-tetrahydro-4H-[1,3]thiazolo[4,5-d]azepin-2-yl)-N-(2-methylphenyl)-1-piperidinecarboxamide (E70)

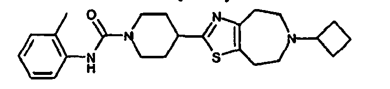

4-(6-Cyclobutyl-5,6,7,8-tetrahydro-4*H*-[1,3]thiazolo[4,5-*d*]azepin-2-yl)-N-(2-methylphenyl)-1-piperidinecarboxamide (E70) was prepared from 6-cyclobutyl-2-(4-piperidinyl)-5,6,7,8-tetrahydro-4*H-*[1,3]thiazolo[4,5-*d*]azepine (may be prepared as described in Example 7) (50.0mg, 0.17mmol) and 2-methylaniline (36µl, 0.34mmol) using an analogous process to that described in Example 68; MS (ES+) m/e 425 [M+H]⁺.

### Example 71

### 4-(6-Cyclobutyl-5,6,7,8-tetrahydro-4H-[1,3]thiazolo[4,5-d]azepin-2-yl)-N-(3-methylphenyl)-1-piperidinecarboxamide (E71)

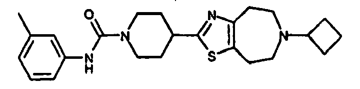

4-(6-Cyclobutyl-5,6,7,8-tetrahydro-4*H*-[1,3]thiazolo[4,5-*d*]azepin-2-yl)-*N*-(3-methylphenyl)-1-piperidinecarboxamide (E71) was prepared from 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) and 3-methylaniline (36µl, 0.34mmol) using an analogous process to that described in Example 68; MS (ES+) m/e 425 [M+H]⁺.

### Example 72

### 4-(6-Cyclobutyl-5,6,7,8-tetrahydro-4H-[1,3]thiazolo[4,5-d]azepin-2-yl)-N-(4-methylphenyl)-1-piperidinecarboxamide (E72)

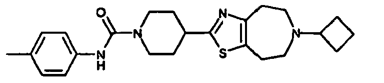

4-(6-Cyclobutyl-5,6,7,8-tetrahydro-4*H*[1,3]thiazolo[4,5-*d*]azepin-2-yl)-*N*-(4-methylphenyl)-1-piperidinecarboxamide (E72) was prepared from 6-cyclobutyl-2-(4-piperidinyl)-5,6,7,8-tetrahydro-4H-[1,3]thiazolo[4,5-*d*]azepine (may be prepared as described in Example 7) (50.0mg, 0.17mmol) and 4-methylaniline (36µl, 0.34mmol) using an analogous process to that described in Example 68; MS (ES+) m/e 425 [M+H]⁺.

### Example 73

### 6-(6-Cyclobutyl-5,6,7,8-tetrahydro-4H-[1,3]thiazolo[4,5-d]azepin-2-yl)-3-pyridinecarbonitrile (E73)

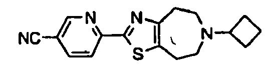

To a suspension of 6-(5,6,7,8-tetrahydro-4*H*-[1,3]thiazolo[4,5-*d*]azepin-2-yl)-3-pyridinecarbonitrile (may be prepared as described in Description 24) (33.0mg, 0.13mml) in dichloromethane (2ml) and acetic acid (2 drops) was added cyclobutanone (14.0µl, 0.19mmol) and the resulting mixture allowed to stir at room temperature for approximately 10 minutes. After this time sodium triacetoxyborohydride (40.0mg, 0.19mmol) was added and the reaction mixture stirred at room temperature for 2.5 hours. The mixture was diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-5%), and repurified using MDAP to afford the product (E73); MS (ES+) m/e 311 [M+H]⁺.

### Example 74.

### 6-(6-Cyclopentyl-5,6,7,8-tetrahydro-4H-[1,3]thiazolo[4,5-d]azepin-2-yl)-3-pyridinecarbonitrile (E74)

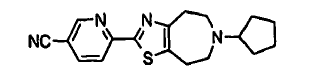

To a suspension of 6-(5,6,7,8-tetrahydro-4*H-*[1,3]thiazolo[4,5-*d*]azepin-2-yl)-3-pyridinecarbonitrile (may be prepared as described in Description 24) (33.0mg, 0.13mml) in dichloromethane (2ml) and acetic acid (2 drops) was added cyclopentanone (17.0µl, 0.19mmol) and the resulting mixture allowed to stir at room temperature for approximately 10 minutes. After this time sodium triacetoxyborohydride (40.0mg, 0.19mmol) was added and the reaction mixture stirred at room temperature for 2.5 hours. A further portion of cyclopentanone (17.0µl, 0.19mmol) and sodium triacetoxyborohydride (40.0mg, 0.19mmol) was added and the reaction mixture stirred at room temperature for 30 minutes. The mixture was diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-5%), and repurified using MDAP to afford the product (E74); MS (ES+) m/e 325 [M+H]⁺.

### Example 75

### 6-(6-Cyclobutyl-5,6,7,8-tetrahydro-4H-[1,3]thiazolo[4,5-d]azepin-2-yl)-3-pyridinecarboxylic acid (E75)

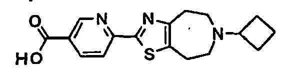

To a solution of 6-[6-(trifluoroacetyl)-5,6,7,8-tetrahydro-4*H*-[1,3]thiazolo[4,5-*d*]azepin-2-yl]-3-pyridinecarbonitrile (may be prepared as described in Description 23) (27.0mg, 0.08mmol) in methanol (2ml) and water (1ml) was added potassium carbonate (52.0mg, 0.38mmol) and the resulting mixture allowed to stir at room temperature for 1 hour. The reaction mixture was acidified with 2M HCl and passed down an ion exchange cartridge (SCX) and washed with methanol and then a 2M ammonia in methanol solution. The basic fractions were then combined and evaporated to afford a 2:1 mixture of methyl 6-(5,6,7,8-tetrahydro-4*H*-[1,3]thiazolo[4,5-*d*]azepin-2-yl)-3-pyridinecarboxylate and 6-(5,6,7,8-tetrahydro-4*H*-[1,3]thiazolo[4,5-*d*]azepin-2-yl)-3-pyridinecarbonitrile. The mixture was suspended in dichloromethane (2ml) and acetic acid (1 drop). To this suspension was added cyclobutanone (16µl, 0.22mmol), followed by sodium triacetoxyborohydride (46.0mg, 0.22mmol). The resulting mixture was allowed to stir at room temperature overnight. The mixture was quenched with methanol and passed down an ion exchange cartridge (SCX) and washed with methanol and then a 2M ammonia in methanol solution. The basic fractions were then combined and evaporated to afford a 2:1 mixture of methyl 6-(6-cyclobutyl-5,6,7,8-tetrahydro-4*H-*[1,3]thiazolo[4,5-*d*]azepin-2-yl)-3-pyridinecarboxylate and 6-(6-cyclobutyl-5,6,7,8-tetrahydro-4*H-*[1,3]thiazolo[4,5-*d*]azepin-2-yl)-3-pyridinecarbonitrile. The mixture was dissolved in formic acid (1 ml), treated with concentrated hydrochloric acid (1 ml) and heated at reflux for 1 hour. The reaction mixture was evaporated and azeotroped with toluene and ethanol to afford the product (E75); MS (ES+) m/e 330 [M+H]⁺.

### Example 76

### 6-(6-Cyclobutyl-5,6,7,8-tetrahydro-4H-[1,3]thiazolo[4,5-d]azepin-2-yl)-N-methyl-3-pyridinecarboxamide (E76)

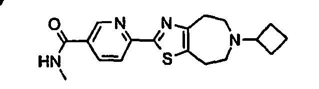

A mixture of 6-(6-cyclobutyl-5,6,7,8-tetrahydro-4*H*-[1,3]thiazolo[4,5-*d*]azepin-2-yl)-3-pyridinecarboxylic acid (may be prepared as described in Example 75) (20.0mg, 0.06mmol), 1*H-*1,2,3-benzotriazol-1-ol (16.0mg, 0.12mmol), and N-cyclohexylcarbodiimide, N'-methyl polystyrene (2.1 mmol/g) (57.0mg, 0.12mmol) in dimethylformamide (2ml) was stirred at room temperature for approximately 30 minutes. After this time a 40% solution of methylamine in water (10µl, 0.12mmol) was added and the reaction mixture stirred at room temperature overnight. A further portion of a 40% solution of methylamine in water (10µl, 0.12mmol) was added and the reaction mixture stirred at room temperature for 30 minutes. The reaction mixture was passed down an ion exchange cartridge (SCX) and washed with methanol and then a 2M ammonia in methanol solution. The basic fractions were then reduced and purified using silica gel chromatography, eluting with a mixture of 2M ammonia/methanol in dichloromethane (0-5%) to afford the product (E76); MS (ES+) m/e 343 [M+H]⁺.

### Example 77

### 5-[4-(6-Cyclobutyl-5,6,7,8-tetrahydro-4H-[1,3]thiazolo[4,5-d]azepin-2-yl)-1-piperidinyl]-N-methyl-2-pyridinecarboxamide (E77)

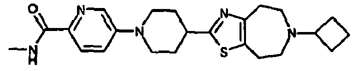

6-Cyclobutyl-2-{1-[6-(1*H*-imidazol-1-ylcarbonyl)-3-pyridinyl]-4-piperidinyl}-5,6,7,8-tetrahydro-4H-[1,3]thiazolo[4,5-*d*]azepine (may be prepared as described in Example 23) (49.0mg, 0.25mmol) was dissolved in dichloromethane (3ml) and treated with a 2M solution of methylamine in THF (0.50ml, 1.00mmol) and stirred at room temperature for 2.5 hours. The mixture was purified directly using silica gel chromatography, eluting with a mixture of 2M ammonia/methanol in dichloromethane (0-7%). The product was repurified using silica gel chromatography, eluting with a mixture of 2M ammonia/methanol in dichloromethane (3%) to afford the product (E77); MS (ES+) m/e 426 [M+H]⁺.

### Examples 78-93 (E78-E93)

Examples 78 to 93 (E78-E93) may be prepared from 6-cyclobutyl-2-(4-piperidinyl)-5,6,7,8-tetrahydro-4*H*-[1,3]thiazolo[4,5-*d*]azepine (may be prepared as described in Example 7) and the appropriate carboxylic acid by an analogous process to that described in Example 8.

| Example | Carboxylic Acid | MS (ES+) |
|---|---|---|
| 6-Cyclobutyl-2-{1-[(2-methylphenyl)carbonyl]-4-piperidinyl}-5,6,7,8-tetrahydro-4*H* [1,3]thiazolo[4,5-*d*]azepine (E78) | 2-Methylbenzoic acid | m/e 410 [M+H]⁺. |
| 6-Cyclobutyl-2-{1-[(3-methyl-1*H*-pyrazol-5-yl)carbonyl]-4-piperidinyl}-5,6,7,8-tetrahydro-4*H*-1,3]thiazolo[4.5-*d*]azepine (E79) | 3-Methyl-1*H*-pyrazole-5-carboxylic acid | m/e 400 [M+H]⁺. |
| 2-{1-[(4-Chlorophenyl)carbonyl]-4-piperidinyl}-6-cyclobutyl-5,6,7,8-tetrahydro-4*H*-[1,3]thiazolo[4,5-*d*]azepine (E80) | 4-Chlorobenzoic acid | m/e 430 [M+H]⁺. |
| 2-{1-[(3-Chlorophenyl)carbonyl]-4-piperidinyl}-6-cyclobutyl-5,6,7,8-tetrahydro-4*H*-[1,3]thiazolo[4,5-*d*]azepine (E81) | 3-Chlorobenzoic acid | m/e 430.[M+H]⁺. |
| 6-Cyclobutyl-2-{1-[(3-methylphenyl)carbonyl]-4-piperidinyl}-5,6,7,8-tetrahydro-4*H*-[1,3]thiazolo[4,5-*d*]azepine (E82) | 3-Methylbenzoic acid | m/e 410 [M+H]⁺. |
| 6-Cyclobutyl-2-{1-[(3-methyl-2-pyridinyl)carbonyl]-4-piperidinyl}-5,6,7,8-tetrahydro-4*H-*[1,3]thiazolo[4,5-*d*]azepine (E83) | 3-Methyl-2-pyridinecarboxylic acid | m/e 411 [M+H]⁺. |
| 6-Cyclobutyl-2-[1-(1*H*-pyrazol-3-ylcarbonyl)-4-piperidinyl]-5,6,7,8-tetrahydro-4*H*-[1,3]thiazolo[4,5-*d*]azepine (E84) | 1*H*-Pyrazole-3-carboxylic acid | m/e 386 [M+H]⁺. |
| 6-Cyclobutyl-2-{1-[(1,4-dimethyl-1*H*-pyrazol-3-yl)carbonyl]-4-piperidinyl}-5,6,7,8-tetrahydro-4*H*-[1,3]thiazolo[4,5-*d*]azepine (E85) | 1,4-Dimethyl-1*H*-pyrazole-3-carboxylic acid | m/e 414 [M+H]⁺. |
| 6-Cyclobutyl-2-{1-[(1-methyl-1*H*-pyrrol-2-yl)carbonyl]-4-piperidinyl}-5,6,7,8-tetrahydro-4H-[1,3]thiazolo[4,5-*d*]azepine (E86) | 1-Methyl-1*H*-pyrrole-2-carboxylic acid | m/e 399 [M+H]⁺. |
| 6-Cyclobutyl-2-{1-[(1-methyl-1*H* imidazol-5-yl)carbonyl]-4-piperidinyl}-5,6,7,8-tetrahydro-4*H*-[1,3]thiazolo[4,5-*d*]azepine (E87) | 1-Methyl-1*H-*imidazole-5-carboxylic acid | m/e 400 [M+H]⁺. |
| 6-Cyclobutyl-2-{1-[(1,5-dimethyl-1*H*-pyrazol-3-yl)carbonyl]-4-piperidinyl}-5,6,7,8-tetrahydro-4*H*-[1,3]thiazolo[4,5-*d*]azepine (E88) | 1,5-Dimethyl-1*H*-pyrazole-3-carboxylic acid | m/e 414 [M+H]⁺. |
| 6-Cyclobutyl-2-{1-[(1-methyl-1*H* imidazol-4-yl)carbonyl]-4-piperidinyl}-5,6,7,8-tetrahydro-4*H*-[1,3]thiazolo[4,5-*d*]azepine (E89) | 1-Methyl-1 H-imidazole-4-carboxylic acid | m/e 400 [M+H]⁺. |
| 6-Cyclobutyl-2-[1-(2-pyridinylcarbonyl)-4-piperidinyl]-5,6,7,8-tetrahydro-4*H*-[1,3]thiazolo[4.5-*d*]azepine (E90) | 2-Pyridinecarboxylic acid | m/e 397 [M+H]⁺. |
| 6-Cyclobutyl-2-[1-(3-pyridinylcarbonyl)-4-piperidinyl]-5,6,7,8-tetrahydro-4*H*-[1,3]thiazolo[4,5-*d*]azepine (E91) | 3-Pyridinecarboxylic acid | m/e 397 [M+H]⁺. |
| 6-Cyclobutyl-2-{1-[(6-methyl-2-pyrazinyl)carbonyl]-4-piperidinyl}-5,6,7,8-tetrahydro-4*H*-[1,3]thiazolo[4,5-*d*]azepine (E92) | 6-Methyl-2-pyrazinecarboxylic acid (may be prepared as described in Description 25) | m/e 412 [M+H]⁺. |
| 6-Cyclobutyl-2-{1-[(2-methyl-4-pyridinyl)carbonyl]-4-piperidinyl}-5,6,7,8-tetrahydro-4*H*-[1,3]thiazolo[4,5-*d*]azepine (E93) | 2-Methyl-4-pyridinecarboxylic acid (may be prepared as described in Description 26) | m/e 411 [M+H]⁺. |

### Example 94

### 6-Cyclobutyl-2-[1-(4-pyridinylcarbonyl)-4-piperidinyl]-5,6,7,8-tetrahydro-4H-[1,3]thiazolo[4,5-d]azepine (E94)

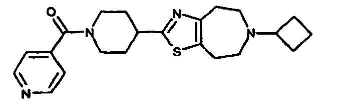

2-[1-(4-Pyidinylcarbonyl)-4-piperidinyl]-5,6,7,8-tetrahydro-4*H*-[1,3]thiazolo[4,5-*d*]azepine (may be prepared as described in Description 28) (12.0mg, 0.035mmol) was dissolved in dichloromethane (4ml) and treated with cyclobutanone (5.00µl, 0.07mmol) and acetic acid (1 drop) and stirred at room temperature. Sodium triacetoxyborohydride (15.0mg, 0.07mmol) was added and the mixture stirred for 2 hours. The mixture was diluted with methanol and passed down an ion exchange cartridge (SCX) and washed with methanol and then a 2M ammonia in methanol solution. The product containing fractions were then reduced to afford the product (E94); MS (ES+) m/e 397 [M+H]⁺.

### Example 95

### 6-Cyclobutyl-2-{1-[(2-methyl-4-pyrimidinyl)carbonyl]-4-piperidinyl}-5,6,7,8-tetrahydro-4H-[1,3]thiazolo[4,5-d]azepine (E95)

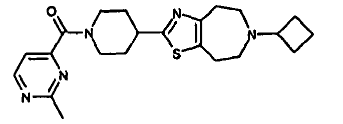

A mixture of 2-methyl-4-pyrimidinecarboxylic acid (may be prepared as described in Description 31) (30.0mg, 0.22mmol), 1*H*-1,2,3-benzotriazol-1-ol (29.0mg, 0.22mmol), and N-cyclohexylcarbodiimide, N'-methyl polystyrene (2.1 mmol/g) (105mg, 0.22mmol) in dimethylformamide (3ml) was stirred at room temperature for 30 minutes. After this time a solution of 6-cyclobutyl-2-(4-piperidinyl)-5,6,7,8-tetrahydro-4*H*-[1,3]thiazolo[4,5-*d*]azepine (may be prepared as described in Example 7) (43.0mg, 0.15mmol) in dimethylformamide (3ml) was added and the resulting mixture stirred at room temperature for 5 hours. The mixture was diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia in methanol and dichloromethane (5%) to afford the product (E95); MS (ES+) m/e 412 [M+H]⁺.

### Example 96

### 6-Cyclobutyl-2-{1-[(2-methyl-5-pyrimidinyl)carbonyl]-4-piperidinyl}-5,6,7,8-tetrahydro-4H-[1,3]thiazolo[4,5-d]azepine (E96)

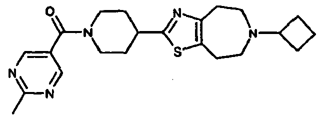

6-Cyclobutyl-2-{1-[(2-methyl-5-pyrimidinyl)carbonyl]-4-piperidinyl}-5,6,7,8-tetrahydro-4H [1,3]thiazolo[4,5-*d*]azepine (E96) was prepared from 6-cyclobutyl-2-(4-piperidinyl)-5,6,7,8-tetrahydro-4*H*-[1,3]thiazolo[4,5-*d*]azepine (may be prepared as described in Example 7) (30.0mg, 0.10mmol) and 2-methyl-5-pyrimidinecarboxylic acid (may be prepared as described in Description 34) (20.0mg, 0.15mmol) using an analogous process to that described in Example 95; MS (ES+) m/e 412 [M+H]⁺.

### Example 97

### 6-Cyclobutyl-2-[1-(4-morpholinylcarbonyl)-4-piperidinyl]-5,6,7,8-tetrahydro-4H-[1,3]thiazolo[4,5-d]azepine (E97)

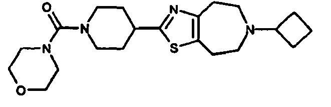

6-Cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) was dissolved in dichloromethane (6ml). 4-morpholinecarbonyl chloride (40.0µl, 0.34mmol) and triethylamine (47.0µl, 0.34mmol) were added and the mixture stirred at room temperature overnight. The mixture was diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia in methanol and dichloromethane (0-5%) to afford the product (E97); MS (ES+) m/e 405 [M+H]⁺.

### Example 98

### 6-Cyclobutyl-2-[1-(1-pyrrolidinylcarbonyl)-4-piperidinyl]-5,6,7,8-tetrahydro-4H-[1,3]thiazolo[4,5-d]azepine (E98)

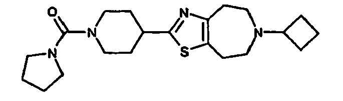

6-Cyclobutyl-2-[1-(1-pyrrolidinylcarbonyl)-4-piperidinyl]-5,6,7,8-tetrahydro-4*H* [1,3]thiazolo[4,5-*d*]azepine (E98) was prepared from 6-cyclobutyl-2-(4-piperidinyl)-5,6,7,8-tetrahydro-4*H*-[1,3]thiazolo[4,5-*d*]azepine (may be prepared as described in Example 7) (50.0mg, 0.17mmol) and 1-pyrrolidinecarbonyl chloride (38.0µl, 0.34mmol) using an analogous process to that described in Example 97; MS (ES+) m/e 389 [M+H]⁺.

### Example 99

### 6-Cyclobutyl-2-[4-(1,1-dioxido-2-isothiazolidinyl)phenyl]-5,6,7,8-tetrahydro-4H-[1,3]thiazolo[4,5-d]azepine (E99)

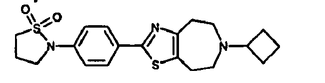

A mixture of 2-(4-bromophenyl)-6-cyclobutyl-5,6,7,8-tetrahydro-4*H*[1,3]thiazolo[4,5-*d*]azepine (may be prepared as described in Example 2) (57.0mg, 0.16mmol), isothiazolidine 1,1-dioxide (38.0mg, 0.31mmol), cesium carbonate (78.0mg, 0.24mmol), tris(dibenzylideneacetone)dipalladium(0) (7.00mg, 0.008mmol), and 4,5-bis(diphenylphosphino)-9,9-dimethyl-xanthene (Xantphos) (14.0mg, 0.024mmol) in 1,4-dioxane (3ml) was heated under argon at 106°C for 3 hours. The reaction was then cooled to room temperature, diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia in methanol and dichloromethane (1-2.5%) to afford the product (E99); MS (ES+) m/e 404 [M+H]⁺.

### Example 100

### 5-(6-Cyclobutyl-5,6,7,8-tetrahydro-4H-[1,3]thiazolo[4,5-d]azepin-2-yl)-2-pyridinecarboxamide (E100)

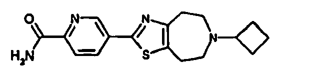

5-(6-Cyclobutyl-5,6,7,8-tetrahydro-4*H*-[1,3]thiazolo[4,5-*d*]azepin-2-yl)-2-pyridinecarboxylic acid (may be prepared as described in Example 50) (130mg, 0.40mmol) was dissolved in dichloromethane (3ml), treated with N,N'-carbonyldiimidazole (128mg, 0.79mmol) and stirred at room temperature for 5 hours, then for 2 days. A further portion of N,N'-carbonyldiimidazole (64.0mg, 0.40mmol) was added and the mixture was heated at 40 °C for 1 hour. The mixture was evaporated, and a third of the residue redissoved in dichloromethane (3ml) and treated with 0.880 ammonia (0.023ml, 1.20mmol). The mixture was stirred at room temperature for 2 hours. The mixture was then purified using silica gel chromatography, eluting with a mixture of 2M ammonia in methanol and dichloromethane (3%) to afford the product (E100); MS (ES+) m/e 329 [M+H]⁺.

### Example 101

### 6-Cyclobutyl-2-[6-(1-pyrrolidinylcarbonyl)-3-pyridinyl]-5,6,7,8-tetrahydro-4H-[1,3]thiazolo[4,5-d]azepine (E101)

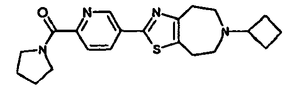

6-Cyclobutyl-2-[6-(1-pyrrolidinylcarbonyl)-3-pyridinyl]-5,6,7,8-tetrahydro-4*H-*[1,3]thiazolo[4,5-*d*]azepine (E101) was prepared from 5-(6-cyclobutyl-5,6,7,8-tetrahydro-4*H*-[1,3]thiazolo[4,5-*d*]azepin-2-yl)-2-pyridinecarboxylic acid (may be prepared as described in Example 50) and pyrrolidine using using an analogous process to that described in Example 100; MS (ES+) m/e 383 [M+H]⁺.

### Example 102

### 6-Cyclobutyl-2-[6-(3-methyl-1,2,4-oxadiazol-5-yl)-3-pyridinyl]-5,6,7,8-tetrahydro-4H-[1,3]thiazolo[4,5-d]azepine (E102)

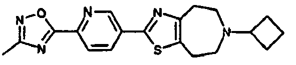

5-(6-Cyclobutyl-5,6,7,8-tetrahydro-4*H*-[1,3]thiazolo[4,5-*d*]azepin-2-yl)-2-pyridinecarboxylic acid (may be prepared as described in Example 50) (130mg, 0.40mmol) was dissolved in dichloromethane (3ml), treated with N,N'-carbonyldiimidazole (128mg, 0.79mmol) and stirred at room temperature for 5 hours, then for 2 days. A further portion of N,N'-carbonyldiimidazole (64.0mg, 0.40mmol) was added and the mixture was heated at 40 °C for 1 hour. The mixture was evaporated, a third redissolved in toluene (3ml) and treated with (1Z)-*N*-hydroxyethanimidamide (may be prepared as described in Description 35) (88.0mg, 1.20mmol). The mixture was stirred and heated at reflux for 4 hours. Allowed to cool to room temperature and purified using silica gel chromatography, eluting with a mixture of 2M ammonia in methanol and dichloromethane (4%) to afford the product (E102); MS (ES+) m/e 368 [M+H]⁺.

### Example 103

### 1-(5-(6-Cyclobutyl-5,6,7,8-tetrahydro-4H-[1,3]thiazolo[4,5-d]azepin-2-yl)-2-pyridinyl]-2-imidazolidinone (E103)

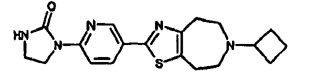

2-(6-Chloro-3-pyridinyl)-6-cyclobutyl-5,6,7,8-tetrahydro-4*H-*[1,3]thiazolo[4,5-*d*]azepine (may be prepared as described in Example 54, method B) (54.0mg, 0.17mmol), 2-imidazolidinone (88.0mg, 1.02mmol), cesium carbonate (83.0mg, 0.26mmol), tris(dibenzylideneacetone)dipalladium(0) (7.80mg, 0.009mmol), and 4,5-bis(diphenylphosphino)-9,9-dimethyl-xanthene (Xantphos) (15.0mg, 0.026mmol) in 1,4-dioxane (3ml) was heated at reflux under argon for 4 hours. The reaction was then cooled to room temperature, evaporated and diluted with methanol and passed down an ion exchange cartridge (SCX) and washed with methanol and then a 2M ammonia in methanol solution. The basic fractions were then reduced and purified using silica gel chromatography, eluting with a mixture of 2M ammonia in methanol and dichloromethane (3%), the re-purified using MDAP to afford the product (E103); MS (ES+) m/e 370 [M+H]⁺.

### Example 104

### Methyl 5-(6-cyclobutyl-5,6,7,8-tetrahydro-4H-[1,3]thiazolo[4,5-d]azepin-2-yl)-2-pyrazinecarboxylate (E104)

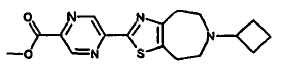

Methyl 5-(trimethylstannanyl)-2-pyrazinecarboxylate (may be prepared as described in Description 36) (227mg, 0.753mmol), 6-cyclobutyl-2-iodo-5,6,7,8-tetrahydro-4H-[1,3]thiazolo[4,5-d]azepine (maybe prepared as described in Description 12, method B) (210mg 0.628mmol) and bis(triphenylphosphine)palladium (II) chloride (25 mg, 5%mol) were dissolved in 10ml of dioxan. The resulting mixture was heated under reflux under argon overnight. A further quantity of bis(triphenylphosphine)palladium (II) chloride (25 mg, 5%mol) was added and the reaction was heated under reflux under argon for another 8 hours. After this time the reaction mixture was applied to an ion exchange cartridge (SCX), washed with methanol and then a 2M ammonia in methanol solution. The basic fractions were then evaporated *in vacuo.* The crude mixture was purified using silica gel chromatography and preparative reverse phase HPLC to afford the product (E104); MS (ES+) m/e 345 [M+H]⁺.

### Example 105

### 4-{[3-(6-Cyclobutyl-5,6,7,8-tetrahydro-4H-[1,3]thiazolo[4,5-d]azepin-2-yl)-1-pyrrolidinyl]carbonyl}benzonitrile (E105)

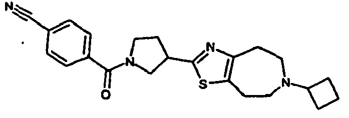

4-{[3-(6-Cyclobutyl-5,6,7,8-tetrahydro-4H-[1,3]thiazolo[4,5-*d*]azepin-2-yl)-1-pyrrolidinyl]carbonyl}benzonitrile (E105) was prepared from 6-cyclobutyl-2-(3-pyrrolidinyl)-5,6,7,8-tetrahydro-4*H-*[1,3]thiazolo[4,5-*d*]azepine (maybe prepared as described in Example 34) and 4-cyanobenzoic acid using an analogous process to that described in example 8; MS (ES+) m/e 407 [M+H]⁺.

### Example 106

### 6-Cyclobutyl-2-{1-[(6-methyl-2-pyridinyl)carbonyl]-3-pyrrolidinyl}-5,6,7,8-tetrahydro-4H-[1,3]thiazolo[4,5-d]azepine) (E106)

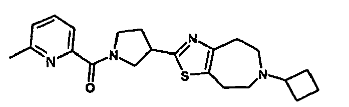

6-Cyclobutyl-2-{1-[(6-methyl-2-pyridinyl)carbonyl]-3-pyrrolidinyl}-5,6,7,8-tetrahydro-4H-[1,3]thiazolo[4,5-d]azepine) (E106) was prepared from 6-cyclobutyl-2-(3-pyrrolidinyl)-5,6,7,8-tetrahydro-4*H*-[1,3]thiazolo[4,5-*d*]azepine (maybe prepared as described in Example 34) and 6-methyl-2-pyridinecarboxylic acid using an analogous process to that described in example 8; MS (ES+) m/e 397 [M+H]+.

### Example 107

### 6-Cyciobutyl-2-[6-(1-pyrrolidinyl)-3-pyridinyl]-5,6,7,8-tetrahydro-4H-[1,3]thiazolo[4,5-d]azepine (E107)

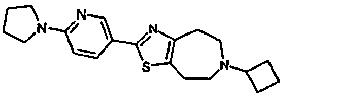

2-(6-Chloro-3-pyridinyl)-6-cyclobutyl-5,6,7,8-tetrahydro-4H-[1,3]thiazolo[4,5-d]azepine (maybe prepared as described in Example 54, method C) (37.8mg, 0.118mmol), pyrrolidine (50 uL, 0.59mmol) and potassium carbonate (82mg, 0.59mmol) were dissolved in 1 ml of dimethylformamide. The resulting mixture was stirred at room temperature for 2 hours. After this time the reaction was warmed to 60 °C overnight. The reaction was allowed to cool down, acidified with 2M hydrochloric acid and applied to an ion exchange cartridge (SCX), washed with methanol and then a 2M ammonia in methanol solution. The methanolic fractions were combined, evaporated *in vacuo* and applied to another ion exchange cartridge (SCX), washed with methanol and then a 2M ammonia in methanol solution. The basic fractions from both cartridges were then evaporated *in vacuo* and the crude material purified using silica gel chromatography, preparative reverse phase HPLC, ion exchange cartridge (SCX) and distillation with dichloromethane to afford the product (E107); MS (ES+) m/e 355 [M+H]⁺.

### Example 108

### 6-Cyclobutyl-2-[6-(4-morpholinyl)-3-pyridinyl]-5,6,7,8-tetrahydro-4H-[1,3]thiazolo[4,5-d]azepine (E108)

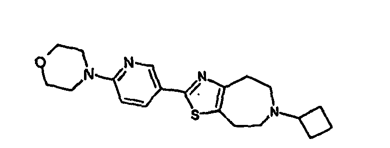

2-(6-Chloro-3-pyridinyl)-6-cyclobutyl-5,6,7,8-tetrahydro-4H-[1,3]thiazolo[4,5-d]azepine (may be prepared as described in Example 54, method C) (60mg, 0.189mmol) was dissolved in neat morpholine (1 mL), The resulting mixture was stirred at 100 °C overnight. The reaction was allowed to cool down, azeotroped with toluene and applied to an ion exchange cartridge (SCX), washed with methanol and then a 2M ammonia in methanol solution. The basic fractions were then evaporated *in vacuo* and crude material purified using silica gel chromatography to afford the product (E108); MS (ES+) m/e 371 [M+H]⁺.

### Example 109

### 5-(6-Cyclobutyl-5,6,7,8-tetrahydro-4H-[1,3]thiazolo[4,5-d]azepin-2-yl)-N-methyl-2-pyrazinecarboxamide (E109)

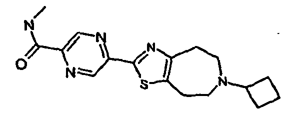

Methyl 5-(6-cyclobutyl-5,6,7,8-tetrahydro-4H-[1,3]thiazolo[4,5-d]azepin-2-yl)-2-pyrazinecarboxylate (may be prepared as described in Example 104) (30mg, 0.087mmol) was dissolved in 1 ml of dry tetrahydrofuran and magnesium chloride (4.15mg, 0.044 mmol) and a 2M solution of methylamine in tetrahydrofuran (131 µl, 0.261 mmol) were added. The resulting mixture was stirred at room temperature for 6 hours. After this time a further portion of a 2M solution of methylamine in tetrahydrofuran (131µl, 0.261 mmol) was added and the mixture was left stirring overnight. The volatiles were removed under reduce pressure and the reaction was dissolved in methanol and applied to an ion exchange cartridge (SCX), washed with methanol and then a 2M ammonia in methanol solution. The basic fractions were then evaporated *in vacuo* and crude material purified using silica gel chromatography, preparative reverse phase HPLC ans ion exchange cartridge (SCX) to afford the product (E109); MS (ES+) m/e 344 [M+H]⁺.

### Example 110

### 6-Cyclobutyl-2-[4-(4-morpholinyl)phenyl]-5,6,7,8-tetrahydro-4H-[1,3]thiazolo[4,5-d]azepine (E110)

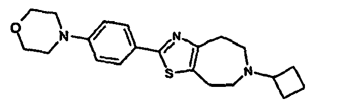

6-Cyclobutyl-2-iodo-5,6,7,8-tetrahydro-4H-[1,3]thiazolo[4,5-d]azepine (may be prepared as described in Description 12) (90mg, 0.27mmol), 4-[4-(4,4,5,5-tetramethyl-1,3,2-dioxaborolan-2-yl)phenyllmorpholine (94mg,0.324 mmol), bis(triphenylphosphine)palladium (II) chloride (19 mg, 10%mol) and sodium carbonate (106 mg, 1.0 mmol) were added together in dioxan (2 ml) and water (0.5 ml) and the resulting mixture was heated under reflux under argon for 2 hours. After this time the reaction mixture was allowed to cool down, acidified to pH=1 with 2N hydrochloric acid, applied to an ion exchange cartridge (SCX), washed with methanol and then a 2M ammonia in methanol solution. The basic fractions were then evaporated *in vacuo* and the crude material purified using silica gel chromatography to afford the product (E110); MS (ES+) m/e 370 [M+H]⁺.

### Example 111

### 6-Cyclobutyl-2-(4-methyl-3,4-dihydro-2H-1,4-benzoxazin-7-yl)-5,6,7,8-tetrahydro-4H-[1,3]thiazolo[4,5-d]azepine (E111)

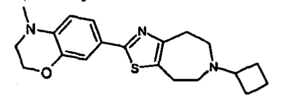

6-Cyclobutyl-2-iodo-5,6,7,8-tetrahydro-4H-[1,3]thiazolo[4,5-d]azepine (maybe prepared as described in Description 12) (89mg, 0.266mmol), 4-methyl-7-(4,4,5,5-tetramethyl-1,3,2-dioxaborolan-2-yl)-3,4-dihydro-2H-1,4-benzoxazine (89mg,0.32 mmol), bis(triphenylphosphine)palladium (II) chloride (19 mg, 10%mol) and sodium carbonate (106 mg, 1.0 mmol) were added together in dioxan (2 ml) and water (0.5 ml) and the resulting mixture was heated under reflux under argon overnight. After this time the reaction mixture was allowed to cool down, acidified with 2N hydrochloric acid, applied to an ion exchange cartridge (SCX), washed with methanol and then a 2M ammonia in methanol solution. The basic fractions were then evaporated *in vacuo* and crude material purified using silica gel chromatography and preparative reverse phase HPLC and ion exchange cartridge (SCX) to afford the product (E111); MS (ES+) m/e 356 [M+H]⁺.

### Example 112

### 3-(6-Cyclobutyl-5,6,7,8-tetrahydro-4H-[1,3]thiazolo[4,5-d]azepin-2-yl)benzonitrile (E112)

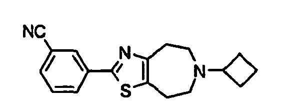

A mixture of 3-(5,6,7,8-tetrahydro-4*H*-[1,3]thiazolo[4,5-*d*]azepin-2-yl)benzonitrile (may be prepared as described in Description 37) (50 mg, 0.2 mmol), cyclobutanone (14 µl, 0.4 mmol) and sodium triacetoxyborohydride (86 mg, 0.4 mmol) in dichloromethane (2 ml) was treated with acetic acid (1 drop) and stirred at room temperature for 2 hours. The mixture was purified on a 5g SCX ion exchange cartridge eluting with methanol and then 2M ammonia in methanol. The basic fractions were combined and evaporated. The residue was purified by chromatography on silica eluting with 5-95 2M ammonia in methanol in dichloromethane. Fractions containing the product were combined and evaporated to afford the title compound (E112); MS (ES+) m/e 310 [M+H]⁺.

### Example 113

### 3-(6-Cyclopentyl-5,6,7,8-tetrahydro-4H-[1,3]thiazolo[4,5-d]azepin-2-yl)benzonitrile (E113)

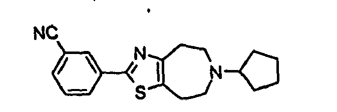

3-(6-Cyclopentyl-5,6,7,8-tetrahydro-4*H*-[1,3]thiazolo[4,5-*d*]azepin-2-yl)benzonitrile (E113) may be prepared from 3-(5,6,7,8-tetrahydro-4*H-*[1,3]thiazolo[4,5-*d*]azepin-2-yl)benzonitrile (may be prepared as described in Description 37) in the same manner as 3-(6-cyclobutyl-5,6,7,8-tetrahydro-4*H*-[1,3]thiazolo[4,5-*d*]azepin-2-yl)benzonitrile (E112) substituting cyclopentanone for cyclobutanone. MS (ES+) m/e 324 [M+H]⁺.

### Example 114

### (±)-6-Cyclobutyl-2-[1-(6-methyl-2-pyridinyl)-3-pyrrolidinyl]-5,6,7,8-tetrahydro-4H-[1,3]thiazolo[4,5-d]azepine (E114)

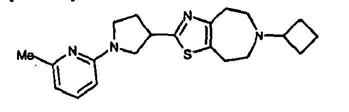

A mixture of 6-cyclobutyl-2-(3-pyrrolidinyl)-5,6,7,8-tetrahydro-4*H*-[1,3]thiazolo[4,5-*d*]azepine (may be prepared as described in Example 34) (48.0mg, 0.17mmol), 2-bromo-6-methylpyridine (44.0mg, 0.26mmol), sodium *tert*-butoxide (33.0mg, 0.34mmol), palladium acetate (4.00mg, 0.02mmol), and 1,1'-binaphthalene-2,2'-diylbis(diphenylphosphane) (BINAP) (22.0mg. 0.04mmol) in 1,4-dioxane (3ml) was heated under argon at reflux for 16 hours. The reaction was then acidified and applied to an ion exchange cartridge (SCX), washed with methanol and then a 2M ammonia in methanol solution. The basic fractions were then reduced and purified using reverse phase chromatography to afford the product (E114); MS (ES+) m/e 369 [M+H]⁺.

### Example 115

### 4-{[4-(6-Cyclobutyl-5,6,7,8-tetrahydro-4H-[1,3]thiazolo[4,5-d]azepin-2-yl)-1-piperidinyl]carbonyl}benzonitrile (E115)

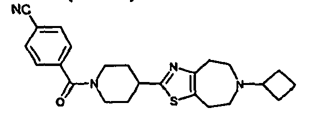

A suspension of 4-cyanobenzoic acid (0.073g, 0.5mmol), 1*H*-1,2,3-benzotriazol-1-ol (0.067g, 0.5mmol), and N-cyclohexylcarbodiimide, N'-methyl polystyrene (2.1 mmol/g) (0.47g, 1 mmol) in dimethylformamide (2ml) was stirred at room temperature for 20 minutes. After this time a solution of 6-cyclobutyl-2-(4-piperidinyl)-5,6,7,8-tetrahydro-4*H* [1,3]thiazolo[4,5-*d*]azepine (may be prepared as described in Example 7) (0.05g, 0.5mmol) in dimethylformamide (0.5ml) was added and the resulting mixture stirred at room temperature for 2 hours. The reaction was then applied to an ion exchange cartridge (SCX) and washed with methanol and then a 2M ammonia in methanol solution. The basic fractions were then reduced and purified using silica gel chromatography to afford the product (E115) (0.03g); MS (ES+) m/e 421 [M+H]⁺.

### Example 116

### 4-(6-Cyclobutyl-5,6,7,8-tetrahydro-4H-[1,3]thiazolo[4,5-d]azepin-2-yl)-N,N-dimethylaniline (E116)

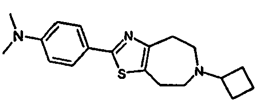

4-(6-Cyclobutyl-5,6,7,8-tetrahydro-4*H*-[1,3]thiazolo[4,5-*d*]azepin-2-yl)-*N*,*N-*dimethylaniline (E116) was prepared from N,N-dimethyl-4-(5,6,7,8-tetrahydro-4H-[1,3]thiazolo[4,5-d]azepin-2-yl)aniline (may be prepared as described in Description 38) using an analogous process to that described in Example 54, method C; MS (ES+) m/e 328 [M+H]+.

### Abbreviations

| | |
|---|---|
| SCX | strong cation exchange |
| Dioxane/dioxan | 1,4-dioxane |

### Biological Data

A membrane preparation containing histamine H3 receptors may be prepared in accordance with the following procedures:

### (i) Generation of histamine H3 cell line

DNA encoding the human histamine H3 gene (Huvar, A. et al. (1999) Mol. Pharmacol. 55(6),1101-1107) was cloned into a holding vector, pCDNA3.1 TOPO (InVitrogen) and its cDNA was isolated from this vector by restriction digestion of plasmid DNA with the enzymes BamH1 and Not-1 and ligated into the inducible expression vector pGene (InVitrogen) digested with the same enzymes. The GeneSwitch™ system (a system where in transgene expression is switched off in the absence of an inducer and switched on in the presence of an inducer) was performed as described in US Patent nos: 5,364,791; 5,874,534; and 5,935,934. Ligated DNA was transformed into competent DH5α E. coli host bacterial cells and plated onto Luria Broth (LB) agar containing Zeocin^{™} (an antibiotic which allows the selection of cells expressing the sh ble gene which is present on pGene and pSwitch) at 50µg ml⁻¹. Colonies containing the re-ligated plasmid were identified by restriction analysis. DNA for transfection into mammalian cells was prepared from 250ml cultures of the host bacterium containing the pGeneH3 plasmid and isolated using a DNA preparation kit (Qiagen Midi-Prep) as per manufacturers guidelines (Qiagen).
CHO K1 cells previously transfected with the pSwitch regulatory plasmid (InVitrogen) were seeded at 2x10e6 cells per T75 flask in Complete Medium, containing Hams F12 (GIBCOBRL, Life Technologies) medium supplemented with 10% v/v dialysed foetal bovine serum, L-glutamine, and hygromycin (100µg ml⁻¹), 24 hours prior to use. Plasmid DNA was transfected into the cells using Lipofectamine plus according to the manufacturers guidelines (InVitrogen). 48 hours post transfection cells were placed into complete medium supplemented with 500µg ml⁻¹ Zeocin^{™}.
10-14 days post selection 10nM Mifepristone (InVitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1x 10e7 cells were examined for receptor expression by staining with a rabbit polyclonal antibody, 4a, raised against the N-terminal domain of the histamine H3 receptor, incubated on ice for 60 minutes, followed by two washes in sorting medium. Receptor bound antibody was detected by incubation of the cells for 60 minutes on ice with a goat anti rabbit antibody, conjugated with Alexa 488 fluorescence marker (Molecular Probes). Following two further washes with Sorting Medium, cells were filtered through a 50µm Filcon^{™} (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500µg ml⁻¹ Zeocin^{™} and allowed to expand before reanalysis for receptor expression via antibody and ligand binding studies. One clone, 3H3, was selected for membrane preparation.

### (ii) Membrane preparation from cultured cells

All steps of the protocol are carried out at 4°C and with pre-cooled reagents. The cell pellet is resuspended in 10 volumes of homogenisation buffer (50mM N-2-hydroxyethylpiperazine-N'-2-ethanesulfonic acid (HEPES), 1 mM ethylenediamine tetra-acetic acid (EDTA), pH 7.4 with KOH, supplemented with 10e-6M leupeptin (acetyl-leucyl-leucyl-arginal; Sigma L2884), 25µg/ml bacitracin (Sigma B0125), , 1mM phenylmethylsulfonyl fluoride (PMSF) and 2x10e-6M pepstain A (Sigma)). The cells are then homogenised by 2 x 15 second bursts in a 1 litre glass Waring blender, followed by centrifugation at 500g for 20 minutes. The supernatant is then spun at 48,000g for 30 minutes. The pellet is resuspended in homogenisation buffer (4X the volume of the original cell pellet) by vortexing for 5 seconds, followed by homogenisation in a Dounce homogeniser (10-15 strokes). At this point the preparation is aliquoted into polypropylene tubes and stored at -80°C.

### (iii) Generation of histamine H1 cell line

The human H1 receptor was cloned using known procedures described in the literature [Biochem. Biophys. Res. Commun. 1994, 201(2), 894]. Chinese hamster ovary cells stably expressing the human H1 receptor were generated according to known procedures described in the literature [Br. J. Pharmacol. 1996, 117(6), 1071].

Compounds of the invention may be tested for in vitro biological activity in accordance with the following assays:

### (I) Histamine H3 functional antagonist assay

For each compound being assayed, in a solid white 384 well plate, is added:-
(a) 0.5µl of test compound diluted to the required concentration in DMSO (or 0.5µl DMSO as a control);
(b) 30µl bead/membrane/GDP mix prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20mM N-2-Hydroxyethylpiperazine-N'-2-ethanesulfonic acid (HEPES) + 100mM NaCl + 10mM MgCl₂, pH7.4 NaOH) to give a final volume of 30µl which contains 5µg protein and 0.25mg bead per well, incubating at room temperature for 60 minutes on a roller and, just prior to addition to the plate, adding 10µM final concentration of guanosine 5' diphosphate (GDP) (Sigma; diluted in assay buffer);
(c) 15µl 0.38nM [³⁵S]-GTPγS (Amersham; Radioactivity concentration=37MBq/ml; Specific activity=1160Ci/mmol), histamine (at a concentration that results in the final assay concentration of histamine being EC₈₀).
After 2-6 hours, the plate is centrifuged for 5 min at 1500 rpm and counted on a Viewlux counter using a 613/55 filter for 5 min/plate. Data is analysed using a 4-parameter logistical equation. Basal activity used as minimum i.e. histamine not added to well.

### (11) Histamine H1 functional antagonist assay

The histamine H1 cell line was seeded into non-coated black-walled clear bottom 384-well tissue culture plates in alpha minimum essential medium (Gibco /Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and maintained overnight at 5% CO₂. 37°C.

Excess medium was removed from each well to leave 10µl). 30µl loading dye (250µM Brilliant Black, 22µM Fluo-4 diluted in Tyrodes buffer + probenecid (145 mM NaCl, 2.5 mM KCI, 10mM HEPES, 10mM D-glucose, 1.2 mM MgCl₂, 1.5 mM CaCl₂, 2.5 mM probenecid, pH adjusted to 7.40 with NaOH 1.0 M)) was added to each well and the plates were incubated for 60 minutes at 5% CO₂, 37°C.

10µl of test compound, diluted to the required concentration in Tyrodes buffer + probenecid (or 10µl Tyrodes buffer + probenecid as a control) was added to each well and the plate incubated for 30 min at 37°C, 5% CO₂. The plates were then placed into a FLIPR^{™} (Molecular Devices, UK) to monitor cell fluorescence (λₑₓ= 488 nm, λ_{EM}= 540 nm) in the manner described in Sullivan et al. (In: Lambert DG (ed.), Calcium Signaling Protocols, New Jersey: Humana Press, 1999, 125-136) before and after the addition of 10µl histamine at a concentration that results in the final assay concentration of histamine being EC₈₀.

Functional antagonism is indicated by a suppression of histamine induced increase in fluorescence, as measured by the FLIPR^{™} system (Molecular Devices). By means of concentration effect curves, functional affinities are determined using standard pharmacological mathematical analysis.

### Results

The hydrochloride salts of Examples E1-E5, E8-E20, E22, E24-E32, E35-E39, E42-E49, E51-E53, E55-E58, E61-E74, E76-E103, E105-E106, E108-E110 and E112-E116 were tested in the histamine H3 functional antagonist assay. The results are expressed as functional pKᵢ (fpKᵢ) values. A functional pKi is the negative logarithm of the antagonist equilibrium dissociation constant as determined in the H3 functional antagonist assay using membrane prepared from cultured H3 cells. The results given are averages of a number of experiments. These compounds exhibited fpKᵢ values > 7.5. More particularly, the hydrochloride salts of E3, E5, E8, E11-E20, E22, E24-E32, E35-E39, E43-E44, E48, E51-E53, E55, E66-E67, E70, E77-E83, E85, E88, E90, E92-E93, E95, E99-E100, E103, E105, E108, E110, E114 and E116 exhibited fpKᵢ values > 8.9. Most particularly, the hydrochloride salts of E3, E11, E13, E20, E22, E26, E28, E32, E36, E44, E52-E53, E67, E78, E81 and E114 exhibited fpKᵢ values ≥ 9.5.

The hydrochloride salts of Examples E107 and E110 were also tested in the histamine H3 functional antagonist assay and exhibited fpkᵢ values of < 8.1 and < 7.81 respectively.

The hydrochloride salts of Examples E1-E5, E8-E20, E22, E24-E32, E35-E39, E42-E49, E51-E53, E55-E58, E61-E74, E76-E103 and E105-E115 were tested in the histamine H1 functional antagonist assay. The results are expressed as functional pKᵢ (fpKᵢ)) values and are averages of a number of experiments. The functional pKi may be derived from the negative logarithm of the plC50 (concentration producing 50% inhibition) in the H1 functional antagonist assay according to the Cheng-Prusoff equation (Cheng, Y.C. and Prusoff, W. H., 1973, Biochem. Pharmacol. 22, 3099-3108.). All compounds tested exhibited fpKᵢ values <5.6.

## Claims

1. A compound of formula (I) or a pharmaceutically acceptable salt or solvate thereof: 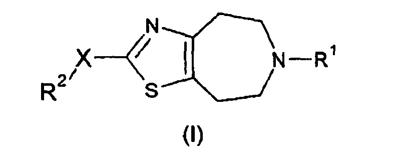 wherein:
R¹ represents -C₂₋₆ alkyl, -C₁₋₃ alkyl-C₃₋₈ cycloalkyl or -C₃₋₇ cycloalkyl, wherein the cycloalkyl groups may be optionally substituted by C₁₋₃ alkyl;
X represents aryl, heteroaryl or heterocyclyl;
R² represents hydrogen, halogen, hydroxy, cyano, nitro, =O, -NR⁸R⁹, -Y-H, -Y-C₁₋₅ alkyl, -Y-C₃₋₈ cycloalkyl, -Y-C₁₋₆alkylC₃₋₈cycloalkyl, -Y-aryl, -Y-heterocyclyl, -Y-heteroaryl, -Y-C₁₋₆ alkyl-aryl, -C₁₋₆ alkyl-Y-H, -C₁₋₆ alkyl-Y-C₁₋₆ alkyl, -C₁₋₆ alkyl-Y-C₃₋₈ cycloalkyl, -C₁₋₆ alkyl-Y-C₁₋₆alkylC₃₋₈cycloalkyl, -C₁₋₆ alkyl-Y-aryl, -C₁₋₆ alkyl-Y-heterocyclyl or -C₁₋₆ alkyl-Y-heteroaryl, wherein R⁸ and R⁹ are independently selected from the group consisting of hydrogen and methyl;
Y represents a bond, C₁₋₆ alkyl, CO, CO₂, CONR³, NR³CO, O, S, SO, SO₂, -SO₂-0-, SO₂NR³, NR³SO₂, OCONR³, NR³CO₂ or NR³CONR⁴ (wherein R³ and R⁴ independently represent hydrogen, -C₁₋₆ alkyl, -C₃₋₈ cycloalkyl, -C₁₋₆ alkylC₃₋₈ cycloalkyl, -aryl, -heterocyclyl or -heteroaryl);
wherein said aryl, heteroaryl and heterocyclyl groups of X may optionally be substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁₋₆ alkyl;
wherein said alkyl, cycloalkyl, aryl, heteroaryl and heterocyclyl groups of R² may be optionally substituted by 1, 2 or 3 substituents which may be the same or different, and which are halogen, cyano, nitro, =O, or a group -R⁵, -OR⁵, -OC₁₋₆ alkyl-R⁶, -C₁₋₆ alkyl-OR⁶, -CO₂R⁵, -COR⁵, COR⁵R⁶, -C₁₋₆ alkyl-COR⁵, -SR⁵, -SO₂R⁵, -SOR⁵, -OSO₂R⁵, -C₁₋₆ alkyl-SO₂R⁵, -C₁₋₆ alkyl-NR⁵SO₂R⁶, -C₁₋₆ alkyl-SO₂NR⁵R⁶, -NR⁵R⁶, -C₁₋₆ alkyl-NR⁵R⁶, -C₃₋₈ cycloalkyl-NR⁵R⁶, -CONR⁵R⁶, -NR⁵COR⁶, -C₁₋₆ alkyl-NR⁵COR⁶, -C₁₋₆ alkyl-CONR⁵R⁶, -NR⁵SO₂R⁶, -OCONR⁵R⁶, -NR⁵CO₂R⁶, -NR⁷CONR⁵R⁶ or -SO₂NR⁵R⁶ (wherein R⁵, R⁶ and R⁷ independently represent hydrogen, C₁₋₆ alkyl, -C₃₋₈ cycloalkyl, -C₁₋₆ alkyl-C₃₋₈ cycloalkyl, aryl, heterocyclyl or heteroaryl or wherein -NR⁵R⁶ may represent a nitrogen containing heterocyclyl group), provided that where R¹ represents -C₂₋₆ alkyl or -C₁₋₃ alkyl-C₃₋₈ cycloalkyl, the alkyl, cycloalkyl, aryl, heteroaryl and heterocyclyl groups of R² may not be substituted with -CO₂R⁵;
wherein R³, R⁴, R⁵, R⁶ and R⁷ may be optionally substituted by 1, 2 or 3 substituents which may be the same or different, and which are selected from the group consisting of halogen, hydroxy, cyano, amino, nitro, =O, C₁₋₆ alkyl, C₁₋₆ alkoxy and haloC₁₋₆ alkyl; and provided that the aryl and heteroaryl groups of X, R², R³, R⁴, R⁵, R⁶ and R⁷ may only be substituted by =O if the substituted group is aromatic;
or solvates thereof.

2. A compound according to claim 1 wherein R¹ represents unsubstituted -C₃₋₇ cycloalkyl.

3. A compound according to claim 2, wherein R¹ represents unsubstituted cyclobutyl.

4. A compound according to any preceding claim, wherein R² represents
-hydrogen;
-halogen;
-cyano;
=O;
-Y-H;
-Y-C₁₋₆6alkyl;
-Y-aryl;
-Y-heterocyclyl; or
-Y-heteroaryl.

5. A compound according to any preceding claim, wherein Y is a bond, O, CO, CO₂ or CONR³, where R³ represents hydrogen.

6. A compound according to any preceding claim, wherein said alkyl, aryl, heteroaryl and heterocyclyl groups of R² may be optionally substituted by 1, 2 or 3 substituents which may be the same or different, and which are selected from the group consisting of cyano, halogen, =O, R⁵, COR⁵, CO₂R⁵, and -CONR⁵R⁶ (wherein R⁵ and R⁶ independently represent hydrogen, -C₁₋₆alkyl or heterocyclyl, and wherein R⁵ and R⁶ may optionally be further substituted by 1, 2 or 3 substituents selected from the group consisting of halogen or -C₁₋₆alkyl), provided that where R¹ represents -C₂₋₆ alkyl or -C₁₋₃ alkyl-C₃₋₈ cycloalkyl, the alkyl, aryl, heteroaryl and heterocyclyl groups of R² may not be substituted with -CO₂R⁵, and provided that the aryl and heteroaryl groups of R² may only be substituted by =O if the substituted group is aromatic.

7. A compound of formula (I) or a pharmaceutically acceptable salt or solvate thereof according to claim 1, 2 or 3, wherein:
R¹ represents -C₂₋₆ alkyl, -C₁₋₃ alkyl-C₃₋₈ cycloalkyl or -C₃₋₇ cycloalkyl, wherein the cycloalkyl groups may be optionally substituted by C₁₋₃ alkyl;
X represents aryl, heteroaryl or heterocyclyl;
R² represents hydrogen, halogen, cyano, =O, -Y-H, -Y-C₁₋₆alkyl, -Y-aryl, -Y-heterocyclyl, -Y-heteroaryl or -NR⁸R⁹ wherein R⁸ and R⁹ are independently selected from the group consisting of hydrogen and methyl;
Y represents a bond, O, CO, CO₂ or CONR³, where R³ represents hydrogen;
wherein said aryl, heteroaryl and heterocyclyl groups of X may optionally be substituted by 1, 2 or 3 substituents which may be the same or different, and which are selected from the group consisting of halogen, C₁₋₆ alkyl, C₁₋₆ alkoxy and haloC₁₋₆ alkyl;
wherein said alkyl, aryl, heteroaryl and heterocyclyl groups of R² may be optionally substituted by 1, 2 or 3 substituents which may be the same or different, and which are selected from the group consisting of cyano, halogen, =O, R⁵, COR⁵, CO₂R⁵, and -CONR⁵R⁶ (wherein R⁵ and R⁶ independently represent hydrogen, -C₁₋₆alkyl or heterocyclyl, and wherein R⁵ and R⁶ may optionally be further substituted by 1, 2 or 3 substituents selected from the group consisting of halogen and -C₁₋₆alkyl);
provided that where R¹ represents -C₂₋₆ alkyl or -C₁₋₃ alkyl-C₃₋₈ cycloalkyl, the alkyl, aryl, heteroaryl and heterocyclyl groups of R² may not be substituted with -CO₂R⁵, and
provided that the aryl and heteroaryl groups of X and R² may only be substituted by =O if the substituted group is aromatic.

8. A compound of formula (I) or a pharmaceutically acceptable salt or solvate thereof according to claim 7, wherein:
R¹ represents -C₁₋₃ alkyl-C₃₋₈ cycloalkyl or -C₃₋₇ cycloalkyl;
X represents aryl, heteroaryl or heterocyclyl;
R² represents hydrogen, halogen, -Y-C₁₋₆alkyl, Y-aryl, -Y-heterocyclyl, or -Y-heteroaryl; Y represents a bond or CO;
wherein said alkyl, aryl, heteroaryl and heterocyclyl groups of R² may be optionally substituted by 1, 2 or 3 substituents which may be the same or different, and which are selected from the group consisting of cyano, =O, R⁵, -COR⁵, -CO₂R⁵, and -CONR⁵R⁶ (wherein R⁵ and R⁶ independently represent hydrogen or -C₁₋₆alkyl, and wherein R⁵ and R⁶ may optionally be further substituted by 1, 2 or 3 halogen atoms);
provided that where R¹ represents -C₁₋₃ alkyl-C₃₋₈ cycloalkyl, the alkyl, aryl, heteroaryl and heterocyclyl groups of R² may not be substituted with -CO₂R⁵, and
provided that the aryl and heteroaryl groups of R² may only be substituted by =O if the substituted group is aromatic.

9. A compound of formula (I) or a pharmaceutically acceptable salt or solvate thereof according to claim 8, wherein:
R¹ represents -C₁₋₃ alkyl-C₃₋₈ cycloalkyl or -C₃₋₇ cycloalkyl;
X represents aryl, heteroaryl or heterocyclyl;
R² represents hydrogen, halogen, -Y-C₁₋₆alkyl, Y-aryl, -Y-heterocyclyl, or -Y-heteroaryl; Y represents a bond or CO;
wherein said alkyl, aryl, heteroaryl and heterocyclyl groups of R² may be optionally substituted by 1, 2 or 3 substituents which may be the same or different, and which are selected from the group consisting of cyano, =O, R⁵, -COR and -CONR⁵R⁶ (wherein R⁵ and R⁶ independently represent hydrogen or -C₁₋₆alkyl, and wherein R⁵ and R⁶ may optionally be further substituted by 1, 2 or 3 halogen atoms);
provided that the aryl and heteroaryl groups of R² may only be substituted by =O if the substituted group is aromatic.

10. A compound according to claim 1, which is:
6-Cyclobutyl-2-[4-(trifluoromethyl)phenyl]-5,6,7,8-tetrahydro-4*H*-[1,3]thiazolo[4,5-d]azepine;
2-(4-Bromophenyl)-6-cyclobutyl-5,6,7,8-tetrahydro-4*H*-[1,3]thiazolo[4,5-*d*]azepine;
1-[4-(6-Cyclobutyl-5,6,7,8-tetrahydro-4*H*-[1,3]thiazolo[4,5-*d*]azepin-2-yl)phenyl]-2-pyrrolidinone;
6-Cyclobutyl-2-[6-(trifluoromethyl)-3-pyridinyl]-5,6,7,8-tetrahydro-4*H*-[1,3]thiazolo[4,5-*d*]azepine;
6-Cyclobutyl-2-[4-(1,2,3-thiadiazol-4-yl)phenyl]-5,6,7,8-tetrahydro-4*H*-[1,3]thiazolo[4,5-*d*]azepine;
1,1-Dimethylethyl 4-(6-cyctobutyl-5,6,7,8-tetrahydro-4*H*-[1,3]thiazolo[4,5-d]azepin-2-yl)-1-piperidinecarboxylate;
6-Cyclobutyl-2-(4-piperidinyl)-5,6,7,8-tetrahydro-4*H*-[1,3]thiazolo[4,5-*d*]azepine;
6-Cyclobutyl-2-{1-[(6-methyl-3-pyridinyl)carbonyl]-4-piperidinyl}-5,6,7,8-tetrahydro-4*H-*[1,3]thiazolo[4,5-*d*]azepine;
5-{[4-(6-Cyclobutyl-5,6,7,8-tetrahydro-4*H*-[1,3]thiazolo[4,5-*d*]azepin-2-yl)-1-piperidinyl]carbonyl}-2-pyridinecarbonitrile;
6-Cyclobutyl-2-(1-{[6-(trifluoromethyl)-3-pyridinyl]carbonyl}-4-piperidinyl)-5,6,7,8-tetrahydro-4*H*-[1,3]thiazolo[4,5-*d*]azepine;
6-Cyclobutyl-2-{1-[(6-methyl-2-pyridinyl)carbonyl]-4-piperidinyl}-5,6,7,8-tetrahydro-4*H-*[1,3]thiazolo[4,5-*d*]azepine;
6-Cyclobutyl-2-{1-[(5-methyl-2-pyrazinyl)carbonyl]-4-piperidinyl}-5,6,7,8-tetrahydro-4*H-*[1,3]thiazolo[4,5-*d*]azepine;
6-Cyclobutyl-2-{1-[(5-methyl-3-pyridinyl)carbonyl]-4-piperidinyl}-5,6,7,8-tetrahydro-4*H-*[1,3]thiazolo[4,5-*d*]azepine;
6-Cyclobutyl-2-{1-[(4-methylphenyl)carbonyl]-4-piperidinyl}-5,6,7,8-tetrahydro-4*H-*[1,3]thiazolo[4,5-*d*]azepine;
6-Cyclobutyl-2-{1-[(5-methyl-3-isoxazolyl)carbonyl]-4-piperidinyl}-5,6,7,8-tetrahydro-4*H-*[1,3]thiazolo[4,5-*d*]azepine;
6-Cyclobutyl-2-[1-(1,2,3-thiadiazol-4-ylcarbonyl)-4-piperidinyl]-5,6,7,8-tetrahydro-4*H-*[1,3]thiazolo[4,5-*d*]azepine;
6-Cyclobutyl-2-{1-[(1-methyl-1*H*-pyrazol-3-yl)carbonyl]-4-piperidinyl}-5,6,7,8-tetrahydro-4*H*-[1,3]thiazolo[4,5-*d*]azepine;
6-Cyclobutyl-2-{1-[(3-methyl-5-isoxazolyl)carbonyl]-4-piperidinyl}-5,6,7,8-tetrahydro-4*H-*[1,3]thiazolo[4,5-*d*]azepine;
6-Cyclobutyl-2-[1-(6-methyl-3-pyridinyl)-4-piperidinyl]-5,6,7,8-tetrahydro-4*H-*[1,3]thiazolo[4,5-*d*]azepine;
5-[4-(6-Cyclobutyl-5,6,7,8-tetrahydro-4*H*-[1,3]thiazolo[4,5-*d*]azepin-2-yl)-1-piperidinyl]-2-pyridinecarbonitrile;
5-[4-(6-Cyclobutyl-5,6,7,8-tetrahydro-4*H*-[1,3]thiazolo[4,5-*d*]azepin-2-yl)-1-piperidinyl]-2-pyridinecarboxylic acid;
5-[4-(6-Cyclobutyl-5,6,7,8-tetrahydro-4*H*-[1,3]thiazolo[4,5-*d*]azepin-2-yl)-1-piperidinyl]-2-pyridinecarboxamide;
6-Cyclobutyl-2-{1-[6-(1*H*-imidazol-1-ylcarbonyl)-3-pyridinyl]-4-piperidinyl}-5,6,7,8-tetrahydro-4*H*-[1,3]thiazolo[4,5-*d*]azepine;
5-[4-(6-Cyclobutyl-5,6,7,8-tetrahydro-4*H*-[1,3]thiazolo[4,5-*d*]azepin-2-yl)-1-piperidinyl]-*N*,*N*-dimethyl-2-pyridinecarboxamide;
6-Cyclobutyl-2-{1-[(2-methyl-3-pyridinyl)carbonyl]-4-piperidinyl}-5,6,7,8-tetrahydro-4*H-*[1,3]thiazolo[4,5-*d*]azepine;
1-[4-(6-Cyclobutyl-5,6,7,8-tetrahydro-4*H*-[1,3]thiazolo[4,5-*d*]azepin-2-yl)phenyl]-3-methyl-2-imidazolidinone;
1-[4-(6-Cyclobutyl-5,6,7,8-tetrahydro-4*H*-[1,3]thiazolo[4,5-*d*]azepin-2-yl)phenyl]-2-imidazolidinone;
1-[4-(6-Cyclopentyl-5,6,7,8-tetrahydro-4*H*-[1,3]thiazolo[4,5-*d*]azepin-2-yl)phenyl]-3-methyl-2-imidazolidinone;
1-Methyl-3-{4-[6-(1-methylethyl)-5,6,7,8-tetrahydro-4*H*-[1,3]thiazolo[4,5-*d*]azepin-2-yl]phenyl}-2-imidazolidinone;
1-[4-(6-Cyclohexyl-5,6,7,8-tetrahydro-4*H*-[1,3]thiazolo[4,5-*d*]azepin-2-yl)phenyl]-3-methyl-2-imidazolidinone;
1-{4-[6-(Cyclopropylmethyl)-5,6,7,8-tetrahydro-4*H*-[1,3]thiazolo[4,5-*d*]azepin-2-yl]phenyl}-3-methyl-2-imidazolidinone;
1-Methyl-3-{4-[6-(2-methylpropyl)-5,6,7,8-tetrahydro-4*H*-[1,3]thiazolo[4,5-*d*]azepin-2-yl]phenyl}-2-imidazolidinone;
1,1-Dimethylethyl 3-(6-cyclobutyl-5,6,7,8-tetrahydro-4*H*-[1,3]thiazolo[4,5-*d*]azepin-2-yl)-1-pyrrolidinecarboxylate;
6-Cyclobutyl-2-(3-pyrrolidinyl)-5,6,7,8-tetrahydro-4*H*-[1,3]thiazolo[4,5-*d*]azepine;
(±)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile;
(-)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile;
(+)-5-[3-(6-Cyclobutyl-5,6,7,8-tetrahydro-4*H*-[1,3]thiazolo[4,5-*d*]azepin-2-yl)-1-pyrrolidinyl]-2-pyridinecarbonitrile;
(±)-6-Cyclobutyl-2-[1-(6-methyl-3-pyridinyl)-3-pyrrolidinyl]-5,6,7,8-tetrahydro-4*H-*[1,3]thiazolo[4,5-*d*]azepine;
5-[3-(6-Cyclobutyl-5,6,7,8-tetrahydro-4*H*-[1,3]thiazolo[4,5-*d*]azepin-2-yl)-1-pyrrolidinyl]-3-pyridinecarbonitrile;
Methyl 4-(6-cyclobutyl-5,6,7,8-tetrahydro-4*H*-[1,3]thiazolo[4,5-*d*]azepin-2-yl)benzoate;
4-(6-Cyclobutyl-5,6,7,8-tetrahydro-4*H*-[1,3]thiazolo[4,5-*d*]azepin-2-yl)benzoic acid;
6-Cyclobutyl-2-phenyl-5,6,7,8-tetrahydro-4*H-*[1,3]thiazolo[4,5-*d*]azepine;
4-(6-Cyclobutyl-5,6,7,8-tetrahydro-4*H*-[1,3]thiazolo[4,5-*d*]azepin-2-yl)-*N-*methylbenzamide;
3-[4-(6-Cyclobutyl-5,6,7,8-tetrahydro-4*H*-[1,3]thiazolo[4,5-*d*]azepin-2-yl)phenyl]-1,3-oxazolidin-2-one;
6-Cyclobutyl-2-[4-(1-pyrrolidinylcarbonyl)phenyl]-5,6,7,8-tetrahydro-4*H*-[1,3]thiazofo[4,5-*d*]azepine;
4-(6-Cyclobutyl-5,6,7,8-tetrahydro-4*H*-[1,3]thiazolo[4,5-*d*]azepin-2-yl)benzamide;
4-(6-Cyclobutyl-5,6,7,8-tetrahydro-4*H*-[1,3]thiazolo[4,5-*d*]azepin-2-yl)-*N*-ethylbenzamide;
4-(6-Cyclobutyl-5,6,7,8-tetrahydro-4*H*-[1,3]thiazolo[4,5-*d*]azepin-2-yl)benzonitrile;
5-(6-Cyclobutyl-5,6,7,8-tetrahydro-4*H*-[1,3]thiazofo[4,5-*d*]azepin-2-yl)-2-pyridinecarbonitrile;
5-(6-Cyclobutyl-5,6,7,8-tetrahydro-4*H*-[1,3]thiazolo[4,5-*d*]azepin-2-yl)-2-pyridinecarboxylic acid;
5-(6-Cyclobutyl-5,6,7,8-tetrahydro-4*H*-[1,3]thiazolo[4,5-*d*]azepin-2-yl)-*N*-methyl-2-pyridinecarboxamide;
1-[5-(6-Cyclobutyl-5,6,7,8-tetrahydro-4*H*-[1,3]thiazolo[4,5-*d*]azepin-2-yl)-2-pyridinyl]-2-pyrrolidinone;
1-[5-(6-Cyclobutyl-5,6,7,8-tetrahydro-4*H*-[1,3]thiazolo[4,5-*d*]azepin-2-yl)-2-pyridinyl]-3-methyl-2-imidazolidinone;
2-(6-Chloro-3-pyridinyl)-6-cyclobutyl-5,6,7,8-tetrahydro-4*H*-[1,3]thiazolo[4,5-*d*]azepine;
6-Cyclobutyl-2-[6-(1-piperidinyl)-3-pyridinyl]-5,6,7,8-tetrahydro-4*H*-[1,3]thiazolo[4,5-*d*]azepine;
5-(6-Cyclobutyl-5,6,7,8-tetrahydro-4*H*-[1,3]thiazolo[4,5-*d*]azepin-2-yl)-2-pyrimidinecarbonitrile;
6-Cyclobutyl-2-[2-(methyloxy)-5-pyrimidinyl]-5,6,7,8-tetrahydro-4*H*-[1,3]thiazolo[4,5-*d*]azepine;
6-Cyclobutyl-2-(5-pyrimidinyl)-5,6,7,8-tetrahydro-4*H*-[1,3]thiazolo[4,5-*d*]azepine;
5-(6-Cyclobutyl-5,6,7,8-tetrahydro-4*H*-[1,3]thiazolo[4,5-*d*]azepin-2-yl)-2(1*H*)-pyrimidinone;
2-(2-Chloro-5-pyrimidinyl)-6-cyclobutyl-5,6,7,8-tetrahydro-4*H*-[1,3]thiazolo[4,5-*d*]azepine;
6-Cyclobutyl-2-[2-(1-piperidinyl)-5-pyrimidinyl]-5,6,7,8-tetrahydro-4*H*-[1,3]thiazolo[4,5-*d*]azepine;
1-{4-[6-(Cyclohexylmethyl)-5,6,7,8-tetrahydro-4*H*-[1,3]thiazolo[4,5-*d*]azepin-2-yl]phenyl} 3-methyl-2-imidazolidinone;
1-[4-(6-Ethyl-5,6,7,8-tetrahydro-4*H*-[1,3]thiazolo[4,5-*d*]azepin-2-yl)phenyl]-3-methyl-2-imidazolidinone;
6-Cyclobutyl-2-{1-[(6-methyl-3-pyridazinyl)carbonyl]-4-piperidinyl}-5,6,7,8-tetrahydro-4*H-*[1,3]thiazolo[4,5-*d*]azepine;
6-Cyclobutyl-2-{1-[(6-methyl-3-pyridinyl)carbonyl]-3-pyrrolidinyl}-5,6,7,8-tetrahydro-4*H-*[1,3]thiazolo[4,5-*d*]azepine;
5-[4-(6-Cyclobutyl-5,6,7,8-tetrahydro-4*H*-[1,3]thiazolo[4,5-*d*]azepin-2-yl)-1-piperidinyl]-3-pyridinecarbonitrile;
4-[4-(6-Cyclobutyl-5,6,7,8-tetrahydro-4*H*-[1,3]thiazolo[4,5-*d*]azepin-2-yl)-1-piperidinyl]benzonitrile;
4-(6-Cyclobutyl-5,6,7,8-tetrahydro-4*H*-[1,3]thiazolo[4,5-*d*]azepin-2-yl)-*N*-methyl-1-piperidinecarboxamide;
4-(6-Cyclobutyl-5,6,7,8-tetrahydro-4*H*-[1,3]thiazolo[4,5-*d*]azepin-2-yl)-*N*-(1-methylethyl)-1-piperidinecarboxamide;
4-(6-Cyclobutyl-5,6,7,8-tetrahydro-4*H*-[1,3]thiazolo[4,5-*d*]azepin-2-yl)-*N*-(2-methylphenyl)-1-piperidinecarboxamide;
4-(6-Cyclobutyl-5,6,7,8-tetrahydro-4*H*-[1,3]thiazolo[4,5-*d*]azepin-2-yl)-*N*-(3-methylphenyl)-1-piperidinecarboxamide;
4-(6-Cyclobutyl-5,6,7,8-tetrahydro-4*H*-[1,3]thiazolo[4,5-*d*]azepin-2-yl)-*N*-(4-methylphenyl)-1-piperidinecarboxamide;
6-(6-Cyclobutyl-5,6,7,8-tetrahydro-4*H*-[1,3]thiazolo[4,5-*d*]azepin-2-yl)-3-pyridinecarbonitrile;
6-(6-Cyclopentyl-5,6,7,8-tetrahydro-4*H*-[1,3]thiazolo[4,5-*d*]azepin-2-yl)-3-pyridinecarbonitrile;
6-(6-Cyclobutyl-5,6,7,8-tetrahydro-4*H*-[1,3]thiazolo[4,5-*d*]azepin-2-yl)-3-pyridinecarboxylic acid;
6-(6-Cyclobutyl-5,6,7,8-tetrahydro-4*H*-[1,3]thiazolo[4,5-*d*]aiepin-2-yl)-*N*-methyl-3-pyridinecarboxamide;
5-[4-(6-Cyclobutyl-5,6,7,8-tetrahydro-4*H*-[1,3]thiazolo[4,5-*d*]azepin-2-yl)-1-piperidinyl]-*N-*methyl-2-pyridinecarboxamide;
6-Cyclobutyl-2-{1-[(2-methylphenyl)carbonyl]-4-piperidinyl}-5,6,7,8-tetrahydro-4*H-*[1,3]thiazolo[4,5-*d*]azepine;
6-Cyclobutyl-2-{1-[(3-methyl-1*H*-pyrazol-5-yl)carbonyl]-4-piperidinyl}-5,6,7,8-tetrahydro-4*H*-[1,3]thiazolo[4,5-*d*]azepine;
2-{1-[(4-Chlorophenyl)carbonyl]-4-piperidinyl}-6-cyclobutyl-5,6,7,8-tetrahydro-4*H-*[1,3]thiazolo[4,5-*d* ]azepine;
2-{1-[(3-Chlorophenyl)carbonyl]-4-piperidinyl}-6-cyclobutyl-5,6,7,8-tetrahydro-4*H-*[1,3]thiazolo[4,5-*d*]azepine;
6-Cyclobutyl-2-{1-[(3-methylphenyl)carbonyl]-4-piperidinyl}-5,6,7,8-tetrahydro-4*H-*[1,3]thiazolo[4,5-*d*]azepine;
6-Cyclobutyl-2-{1-[(3-methyl-2-pyridinyl)carbonyl]-4-piperidinyl}-5,6,7,8-tetrahydro-4*H-*[1,3]thiazolo[4,5-*d*]azepine;
6-Cyclobutyl-2-[1-(1*H*-pyrazol-3-ylcarbonyl)-4-piperidinyl]-5,6,7,8-tetrahydro-4*H-*[1,3]thiazolo[4,5-*d*]azepine;
6-Cyclobutyl-2-{1-[(1,4-dimethyl-1*H*-pyrazol-3-yl)carbonyl]-4-piperidinyl}-5,6,7,8-tetrahydro-4*H*-[1,3]thiazolo[4,5-*d*]azepine;
6-Cyclobutyl-2-{1-[(1-methyl-1*H*-pyrrol-2-yl)carbonyl]-4-piperidinyl}-5,6,7,8-tetrahydro-4*H*-[1,3]thiazolo[4,5-*d*]azepine;
6-Cyclobutyl-2-{1-[(1-methyl-1*H*-imidazol-5-yl)carbonyl]-4-piperidinyl}-5,6,7,8-tetrahydro-4*H*-[1,3]thiazolo[4,5-*d*]azepine;
6-Cyclobutyl-2-{1-[(1,5-dimethyl-1*H*-pyrazol-3-yl)carbonyl]-4-piperidinyl}-5,6,7,8-tetrahydro-4*H*-[1,3]thiazolo[4,5-*d*]azepine;
6-Cyclobutyl-2-{1-[(1-methyl-1*H*-imidazol-4-yl)carbonyl]-4-piperidinyl}-5,6,7,8-tetrahydro-4*H*-[1,3]thiazolo[4,5-*d*]azepine;
6-Cyclobutyl-2-[1-(2-pyridinylcarbonyl)-4-piperidinyl]-5,6,7,8-tetrahydro-4*H-*[1,3]thiazolo[4,5-*d*]azepine;
6-Cyclobutyl-2-[1-(3-pyridinylcarbonyl)-4-piperidinyl]-5,6,7,8-tetrahydro-4*H-*[1,3]thiazolo[4,5-*d*]azepine;
6-Cyclobutyl-2-{1-[(6-methyl-2-pyrazinyl)carbonyl]-4-piperidinyl}-5,6,7,8-tetrahydro-4*H-*[1,3]thiazolo[4,5-*d*]azepine;
6-Cyclobutyl-2-{1-[(2-methyl-4-pyridinyl)carbonyl]-4-piperidinyl}-5,6,7,8-tetrahydro-4*H-*[1,3]thiazolo[4,5-*d*]azepine;
6-Cyclobutyl-2-[1-(4-pyridinylcarbonyl)-4-piperidinyl]-5,6,7,8-tetrahydro-4H-[1,3]thiazolo[4,5-d]azepine;
6-Cyclobutyl-2-{1-[(2-methyl-4-pyrimidinyl)carbonyl]-4-piperidinyl}-5,6,7,8-tetrahydro-4H-[1,3]thiazolo[4,5-d]azepine;
6-Cyclobutyl-2-{1-[(2-methyl-5-pyrimidinyl)carbonyl]-4-piperidinyl}-5,6,7,8-tetrahydro-4H-[1,3]thiazolo[4,5-d]azepine;
6-Cyclobutyl-2-[1-(4-morpholinylcarbonyl)-4-piperidinyl]-5,6,7,8-tetrahydro-4H-[1,3]thiazolo[4,5-d]azepine;
6-Cyclobutyl-2-[1-(1-pyrrolidinylcarbonyl)-4-piperidinyl]-5,6,7,8-tetrahydro-4H-[1,3]thiazolo[4,5-d]azepine;
6-Cyclobutyl-2-[4-(1,1-dioxido-2-isothiazolidinyl)phenyl]-5,6,7,8-tetrahydro-4H-[1,3]thiazolo[4,5-d]azepine;
5-(6-Cyclobutyl-5,6,7,8-tetrahydro-4H-[1,3]thiazolo[4,5-d]azepin-2-yl)-2-pyridinecarboxamide;
6-Cyclobutyl-2-[6-(1-pyrrolidinylcarbonyl)-3-pyridinyl]-5,6,7,8-tetrahydro-4H-[1,3]thiazolo[4,5-d]azepine;
6-Cyclobutyl-2-[6-(3-methyl-1,2,4-oxadiazol-5-yl)-3-pyridinyl]-5,6,7,8-tetrahydro-4H-[1,3]thiazolo[4,5-d]azepine;
1-[5-(6-Cyclobutyl-5,6,7,8-tetrahydro-4H-[1,3]thiazolo[4,5-d]azepin-2-yl)-2-pyridinyl]-2-imidazolidinone;
Methyl 5-(6-cyclobutyl-5,6,7,8-tetrahydro-4H-[1,3]thiazolo[4,5-d]azepin-2-yl)-2-pyrazinecarboxylate;
4-{[3-(6-Cyclobutyl-5,6,7,8-tetrahydro-4H-[1,3]thiazolo[4,5-d]azepin-2-yl)-1-pyrrolidinyl]carbonyl}benzonitrile;
6-Cyclobutyl-2-{1-[(6-methyl-2-pyridinyl)carbonyl]-3-pyrrolidinyl}-5,6,7,8-tetrahydro-4H-[1,3]thiazolo[4,5-d]azepine;
6-Cyclobutyl-2-[6-(1-pyrrolidinyl)-3-pyridinyl]-5,6,7,8-tetrahydro-4H-[1,3]thiazolo[4,5-d]azepine;
6-Cyclobutyl-2-[6-(4-morpholinyl)-3-pyridinyl]-5,6,7,8-tetrahydro-4H-[1,3]thiazolo[4,5-d]azepine;
5-(6-Cyclobutyl-5,6,7,8-tetrahydro-4H-[1,3]thiazolo[4,5-d]azepin-2-yl)-N-methyl-2-pyrazinecarboxamide;
6-Cyclobutyl-2-[4-(4-morpholinyl)phenyl]-5,6,7,8-tetrahydro-4H-[1,3]thiazolo[4,5-d]azepine;
6-Cyclobutyl-2-(4-methyl-3,4-dihydro-2H-1,4-benzoxazin-7-yl)-5,6,7,8-tetrahydro-4H-[1,3]thiazolo[4,5-d]azepine;
3-(6-Cyclobutyl-5,6,7,8-tetrahydro-4H-[1,3]thiazolo[4,5-d]azepin-2-yl)benzonitrile;
3-(6-Cyclopentyl-5,6,7,8-tetrahydro-4H-[1,3]thiazolo[4,5-d]azepin-2-yl)benzonitrile;
(±)-6-Cyclobutyl-2-[1-(6-methyl-2-pyridinyl)-3-pyrrolidinyl]-5,6,7,8-tetrahydro-4H-[1,3]thiazolo[4,5-d]azepine;
4-{[4-(6-Cyclobutyl-5,6,7,8-tetrahydro-4H-[1,3]thiazolo[4,5-d]azepin-2-yl)-1-piperidinyl]carbonyl}benzonitrile;
4-(6-Cylobutyl-5,6,7,8-tetrahydro-4H-[1,3]thiazolo[4,5-d]azepin-2-yl)-N,N-dimethylaniline;
or a pharmaceutically acceptable salt or solvate thereof.

11. A compound according to claim 10 which is:
1-[4-(6-Cyclobutyl-5,6,7,8-tetrahydro-4*H*-[1,3]thiazolo[4,5-*d*]azepin-2-yl)phenyl]-2-pyrrolidinone;
6-Cyclobutyl-2-{1-[(6-methyl-2-pyridinyl)carbonyl]-4-piperidinyl}-5,6,7,8-tetrahydro-4*H*-[1,3]thiazolo[4,5-*d*]azepine;
6-Cyclobutyl-2-{1-[(5-methyl-3-pyridinyl)carbonyl]-4-piperidinyl}-5,6,7,8-tetrahydro-4*H*-[1,3]thiazolo[4,5-*d*]azepine;
5-[4-(6-Cyclobutyl-5,6,7,8-tetrahydro-4*H*-[1,3]thiazolo[4,5-*d*]azepin-2-yl)-1-piperidinyl]-2-pyridinecarbonitrile;
5-[4-(6-Cyclobutyl-5,6,7,8-tetrahydro-4*H*-[1,3]thiazolo[4,5-*d*]azepin-2-yl)-1-piperidinyl]-*N*,*N*-dimethyl-2-pyridinecarboxamide;
1-[4-(6-Cyclobutyl-5,6,7,8-tetrahydro-4*H*-[1,3]thiazolo[4,5-*d*]azepin-2-yl)phenyl]-3-methyl-2-imidazolidinone;
1-[4-(6-Cyclobutyl-5,6,7,8-tetrahydro-4*H*-[1,3]thiazolo[4,5-*d*]azepin-2-yl)phenyl]-2-imidazolidinone;
(+)-5-[3-(6-Cyclobutyl-5,6,7,8-tetrahydro-4*H*-[1,3]thjazolo[4,5-*d*]azepin-2-yl)-1-pyrrolidinyl]-2-pyridinecarbonitrile;
(±)-6-Cyclobutyl-2-[1-(6-methyl-3-pyridinyl)-3-pyrrolidinyl]-5,6,7,8-tetrahydro-4*H-*[1,3]thiazolo[4,5-*d*]azepine;
or a pharmaceutically acceptable salt or solvate thereof.

12. A compound according to claim 10 which is
4-(6-cyclobutyl-5,6,7,8-tetrahydro-4*H*-[1,3]thiazolo[4,5-*d*]azepin-2-yl)-*N*-methyl-1-piperidinecarboxamide 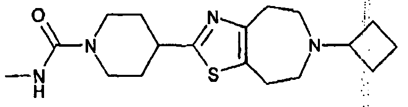 or a pharmaceutically acceptable salt or solvate thereof.

13. A pharmaceutical composition which comprises the compound of formula (I) as defined in any preceding claim or a pharmaceutically acceptable salt or solvate thereof and a pharmaceutically acceptable carrier or excipient.

14. A compound as defined in any one of claims 1 to 12 for use in therapy,

15. A compound as defined in any one of claims 1 to 12 four use in the treatment of neurological diseases.

16. A compound of formula (I) or a pharmaceutically acceptable salt or solvate thereof according to any one of claims 1 to 12, for use as a therapeutic substance in the treatment or prophylaxis of cognitive impairments in Alzheimer's disease or a related neurodegenerative disorder.

17. Use of a compound as defined in any one of claims 1 to 12 in the manufacture of a medicament for the treatment of neurological diseases.

18. A process for the preparation of a compound of formula (I) or a pharmaceutically acceptable salt or solvate thereof as defined in any one of claims 1 to 12, which process comprises:
(a) reacting a compound of formula (II) 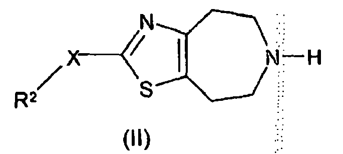 wherein X and R² are as defined in claim 1, with a compound of formula R^{1'}=O, wherein R^{1,} is =C₂₋₆ alkyl, =C₁₋₃ alkyl-C₃₋₈ cycloalkyl or =C₃₋₇ cycloalkyl, wherein the cycloalkyl groups may be optionally substituted by C₁₋₃ alkyl; or
(b) reacting a compound of formula (III) 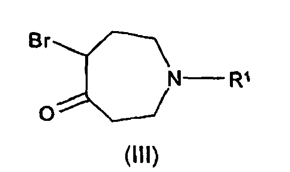 wherein R¹ is as defined in claim 1 with a compound of formula R²X-C(=S)NH₂ wherein R² and X are as defined in claim 1; or
(c) deprotecting a compound of formula (I) which is protected; or
(d) interconversion from one compound of formula (I) to another; or
(e) reacting a compound of formula (X) 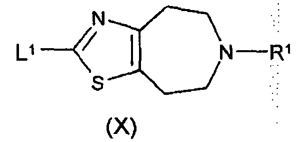 wherein R¹ is as defined in claim 1 and L¹ represents a leaving group such as a halogen, with an organometallic compound of formula (XI), R²-X-L², wherein R² and X are as defined in claim 1 and wherein L² is a leaving group; or
(f) reacting a compound of formula (II) 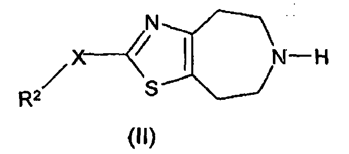
wherein X and R² are as defined in claim 1, with a compound of formula R¹-L³, wherein R¹ is as defined in claim 1, and wherein L³ is a leaving group such as a halogen.

## Patentansprüche

1. Verbindung der Formel (I) oder ein pharmazeutisch verträgliches Salz oder Solvat davon: 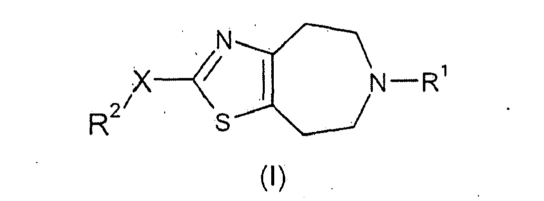 wobei:
R¹ -C₂₋₆-Alkyl, -C₁₋₃-Alkyl-C₃₋₈-cycloalkyl oder -C₃₋₇-Cycloalkyl darstellt, wobei die Cycloalkylreste gegebenenfalls mit C₁₋₃-Alkyl substituiert sein können;
X Aryl, Heteroaryl oder Heterocyclyl darstellt;
R² Wasserstoff, Halogen, Hydroxy, Cyano, Nitro, =O, NR⁸R⁹, -Y-H, -Y-C₁₋₆-Alkyl, -Y-C₃₋₈-Cycloalkyl, -Y-C₁₋₆-Alkyl-C₃₋₈-cycloalkyl, -Y-Aryl, -Y-Heterocyclyl, -Y-Heteroaryl, -Y-C₁₋₆-Alkylaryl, -C₁₋₆-Alkyl-Y-H, -C₁₋₆-Alkyl-Y-C₁₋₆-alkyl, -C₁₋₆-Alkyl-Y-C₃₋₈-cycloalkyl, -C₁₋₆-Alkyl-Y-C₁₋₆-alkyl-C₃₋₈-cycloalkyl, -C₁₋₆-Alkyl-Y-aryl, -C₁₋₆-Alkyl-Y-heterocyclyl oder -C₁₋₆-Alkyl-Y-heteroaryl darstellt, wobei R⁸ und R⁹ unabhängig ausgewählt sind aus Wasserstoff und Methyl;
Y eine Bindung, C₁₋₆-Alkyl, CO, CO₂, CONR³, NR³CO, O, S, SO, SO₂, -SO₂-O-, SO₂NR³, NR³SO₂, OCONR³, NR³CO₂ oder NR³CONR⁴ (wobei R³ und R⁴ unabhängig Wasserstoff, -C₁₋₆-Alkyl, -C₃₋₈-Cycloalkyl, -C₁₋₆-Alkyl-C₃₋₈-cycloalkyl, -Aryl, -Heterocyclyl oder -Heteroaryl darstellen) darstellt;
wobei die Aryl-, Heteroaryl- und Heterocyclylreste von X gegebenenfalls mit 1, 2 oder 3 Substituenten substituiert sein können, die gleich oder verschieden sein können und die ausgewählt sind aus Halogen, Hydroxy, Cyano, Amino, Nitro, =O, C₁₋₆-Alkyl, C₁₋₆-Alkoxy und Halogen-C₁₋₆-alkyl;
wobei die Alkyl-, Cycloalkyl-, Aryl-, Heteroaryl- und Heterocyclylreste von R² gegebenenfalls mit 1, 2 oder 3 Substituenten substituiert sein können, die gleich oder verschieden sein können und die Halogen, Cyano, Nitro, =O, oder ein Rest -R⁵, -OR⁵, -OC₁₋₆-Alkyl-R⁶, -C₁₋₆-Alkyl-OR⁶, -CO₂R⁵, -COR⁵, COR⁵R⁶, -C₁₋₆-Alkyl-COR⁵, -SR⁵, -SO₂R⁵, -SOR⁵, -OSO₂R⁵, -C₁₋₆-Alkyl-SO₂R⁶, -C₁₋₆-Alkyl-NR⁵SO₂R⁶, -C₁₋₆-Alkyl-SO₂NR⁵R⁶, -NR⁵R⁶, -C₁₋₆-Alkyl-NR⁵R⁶, -C₃₋₈-Cycloalkyl-NR⁵R⁶, -CONR⁵R⁶, -NR⁵COR⁶, -C₁₋₆-Alkyl-NR⁵COR⁶, -C₁₋₆-Alkyl-CONR⁵R⁶, -NR⁵SO₂R⁶, -OCONR⁵R⁶, -NR⁵CO₂R⁶, -NR⁷CONR⁵R⁶ oder -SO₂NR⁵R⁶ (wobei R⁵, R⁶ und R⁷ unabhängig Wasserstoff, C₁₋₆-Alkyl, -C₃₋₈-Cycloalkyl, -C₁₋₆-Alkyl-C₃₋₈-cycloalkyl, Aryl, Heterocyclyl oder Heteroaryl darstellen oder wobei -NR⁵R⁶ einen einen Stickstoff enthaltenden Heterocyclylrest darstellen kann) sind, mit der Maßgabe, dass wenn R¹ -C₂₋₆-Alkyl oder -C₁₋₃-Alkyl-C₃₋₈-cycloalkyl darstellt, die Alkyl-, Cycloalkyl-, Aryl-, Heteroaryl- und Heterocyclylreste von R² nicht mit -CO₂R⁵ substituiert sein können;
wobei R³, R⁴, R⁵, R⁶ und R⁷ gegebenenfalls mit 1, 2 oder 3 Substituenten substituiert sein können, die gleich oder verschieden sein können und die ausgewählt sind aus Halogen, Hydroxy, Cyano, Amino, Nitro, =O, C₁₋₆-Alkyl, C₁₋₆-Alkoxy und Halogen-C₁₋₆-alkyl; und mit der Maßgabe, dass die Aryl- und Heteroarylreste von X, R², R³, R⁴, R⁵, R⁶ und R⁷ nur mit =O substituiert sein können, wenn der substituierte Rest aromatisch ist; oder Solvate davon.

2. Verbindung gemäß Anspruch 1, wobei R¹ unsubstituiertes -C₃₋₇-Cycloalkyl darstellt.

3. Verbindung gemäß Anspruch 2, wobei R¹ unsubstituiertes Cyclobutyl darstellt.

4. Verbindung gemäß einem der vorstehenden Ansprüche, wobei R²
-Wasserstoff;
-Halogen;
-Cyano;
=O;
-Y-H;
-Y-C₁₋₆-Alkyl;
-Y-Aryl;
-Y-Heterocyclyl; oder
-Y-Heteroaryl
darstellt.

5. Verbindung gemäß einem der vorstehenden Ansprüche, wobei Y eine Bindung, O, CO, CO₂ oder CONR³ ist, wobei R³ Wasserstoff darstellt.

6. Verbindung gemäß einem der vorstehenden Ansprüche, wobei die Alkyl-, Aryl-, Heteroaryl- und Heterocyclylreste von R² gegebenenfalls mit 1, 2 oder 3 Substituenten substituiert sein können, die gleich oder verschieden sein können und die ausgewählt sind aus Cyano, Halogen, =O, R⁵, COR⁵, CO₂R⁵ und -CONR⁵R⁶ (wobei R⁵ und R⁶ unabhängig Wasserstoff, -C₁₋₆-Alkyl oder Heterocyclyl darstellen, und wobei R⁵ und R⁶ gegebenenfalls weiterhin mit 1, 2 oder 3 Substituenten, ausgewählt aus Halogen oder -C₁₋₆-Alkyl, substituiert sein können), mit der Maßgabe, dass, wenn R¹ -C₂₋₆-Alkyl oder -C₁₋₃-Alkyl-C₃₋₈-cycloalkyl darstellt, die Alkyl-, Aryl-, Heteroaryl- und Heterocyclylreste von R² nicht mit -CO₂R⁵ substituiert sein können, und mit der Maßgabe, dass die Aryl- und Heteroarylreste von R² nur mit =O substituiert sein können, wenn der substituierte Rest aromatisch ist.

7. Verbindung der Formel (I), oder ein pharmazeutisch verträgliches Salz oder Solvat davon, gemäß Anspruch 1, 2 oder 3, wobei:
R¹ -C₂₋₆-Alkyl, -C₁₋₃-Alkyl-C₃₋₈-cycloalkyl oder -C₃₋₇-Cycloalkyl darstellt, wobei die Cycloalkylreste gegebenenfalls mit C₁₋₃-Alkyl substituiert sein können;
X Aryl, Heteroaryl oder Heterocyclyl darstellt;
R² Wasserstoff, Halogen, Cyano, =O, -Y-H, -Y-C₁₋₆-Alkyl, -Y-Aryl, -Y-Heterocyclyl, -Y-Heteroaryl oder -NR⁸R⁹ darstellt, wobei R⁸ und R⁹ unabhängig ausgewählt sind aus Wasserstoff und Methyl;
Y eine Bindung, O, CO, CO₂ oder CONR³ darstellt, wobei R³ Wasserstoff darstellt;
wobei die Aryl-, Heteroaryl- und Heterocyclylreste von X gegebenenfalls mit 1, 2 oder 3 Substituenten substituiert sein können, die gleich oder verschieden sein können und die ausgewählt sind aus Halogen, C₁₋₆-Alkyl, C₁₋₆-Alkoxy und Halogen-C₁₋₆-alkyl;
wobei die Alkyl-, Aryl-, Heteroaryl- und Heterocyclylreste von R² gegebenenfalls mit 1, 2 oder 3 Substituenten substituiert sein können, die gleich oder verschieden sein können und die ausgewählt sind aus Cyano, Halogen, =O, R⁵, COR⁵, CO₂R⁵ und -CONR⁵R⁶ (wobei R⁵ und R⁶ unabhängig Wasserstoff, -C₁₋₆-Alkyl oder Heterocyclyl darstellen und wobei R⁵ und R⁶ gegebenenfalls weiterhin mit 1, 2 oder 3 Substituenten, ausgewählt aus Halogen und -C₁₋₆-Alkyl, substituiert sein können);
mit der Maßgabe, dass, wenn R¹ -C₂-₆-Alkyl oder -C₁₋₃-Alkyl-C₃₋₈-cycloalkyl darstellt, die Alkyl-, Aryl-, Heteroaryl- und Heterocyclylreste von R² nicht mit -CO₂R⁵ substituiert sein können, und mit der Maßgabe, dass, die Aryl- und Heteroarylreste von X und R² nur mit =O substituiert sein können, wenn der substituierte Rest aromatisch ist.

8. Verbindung der Formel (I), oder ein pharmazeutisch verträgliches Salz oder Solvat davon, gemäß Anspruch 7, wobei:
R¹ -C₁₋₃-Alkyl-C₃₋₈-cycloalkyl oder -C₃₋₇-Cycloalkyl darstellt;
X Aryl, Heteroaryl oder Heterocyclyl darstellt;
R² Wasserstoff, Halogen, -Y-C₁₋₆-Alkyl, Y-Aryl, -Y-Heterocyclyl oder -Y-Heteroaryl darstellt;
Y eine Bindung oder CO darstellt;
wobei die Alkyl-, Aryl-, Heteroaryl- und Heterocyclylreste von R² gegebenenfalls mit 1, 2 oder 3 Substituenten substituiert sein können, die gleich oder verschieden sein können und die ausgewählt sind aus Cyano, =O, R⁵, -COR⁵, -CO₂R⁵ und -CONR⁵R⁶ (wobei R⁵ und R⁶ unabhängig Wasserstoff oder -C₁₋₆-Alkyl darstellen, und wobei R⁵ und R⁶ gegebenenfalls weiterhin mit 1, 2 oder 3 Halogenatomen substituiert sein können);
mit der Maßgabe, dass, wenn R¹ -C₁₋₃-Alkyl-C₃₋₈-cycloalkyl darstellt, die Alkyl-, Aryl-, Heteroaryl- und Heterocyclylreste von R² nicht mit -CO₂R⁵ substituiert sein können, und mit der Maßgabe, dass die Aryl- und Heteroarylreste von R² nur mit =O substituiert sein können, wenn der substituierte Rest aromatisch ist.

9. Verbindung der Formel (I), oder ein pharmazeutisch verträgliches Salz oder Solvat davon, gemäß Anspruch 8, wobei:
R¹ -C₁₋₃-Alkyl-C₃₋₈-cycloalkyl oder -C₃₋₇-Cycloalkyl darstellt;
X Aryl, Heteroaryl oder Heterocyclyl darstellt;
R² Wasserstoff, Halogen, -Y-C₁₋₆-Alkyl, Y-Aryl, -Y-Heterocyclyl oder -Y-Heteroaryl darstellt;
Y eine Bindung oder CO darstellt;
wobei die Alkyl-, Aryl-, Heteroaryl-, und Heterocyclylreste von R² gegebenenfalls mit 1, 2 oder 3 Substituenten substituiert sein können, die gleich oder verschieden sein können und die ausgewählt sind aus Cyano, =O, R⁵, -COR⁵ und -CONR⁵R⁶ (wobei R⁵ und R⁶ unabhängig Wasserstoff oder -C₁₋₆-Alkyl darstellen, und wobei R⁵ und R⁶ gegebenenfalls weiterhin mit 1, 2 oder 3 Halogenatomen substituiert sein können);
mit der Maßgabe, dass die Aryl- und Heteroarylreste von R² nur mit =O substituiert sein können, wenn der substituierte Rest aromatisch ist.

10. Verbindung gemäß Anspruch 1, nämlich:
6-Cyclobutyl-2-[4-(trifluormethyl)phenyl]-5,6,7,8-tetrahydro-4*H*-[1,3]thiazol[4,5-*d*]azepin;
2-(4-Bromphenyl)-6-cyclobutyl-5,6,7,8-tetrahydro-4*H*-[1,3]thiazolo[4,5-*d*]azepin;
1-[4-(6-Cyclobutyl-5,6,7,8-tetrahydro-4*H*-[1,3]thiazolo[4,5-*d*]azepin-2-yl)phenyl]-2-pyrrolidinon;
6-Cyclobutyl-2-[6-(trifluormethyl)-3-pyridinyl]-5,6,7,8-tetrahydro-4*H*-[1,3]thiazolo[4,5-*d*]azepin;
6-Cyclobutyl-2-[4-(1,2,3-thiadiazol-4-yl)phenyl]-5,6,7,8-tetrahydro-4*H*-[1,3]thiazolo[4,5-*d*]azepin;
1,1-Dimethylethyl-4-(6-cyclobutyl-5,6,7,8-tetrahydro-4*H-*[1,3]thiazolo[4,5-*d*]azepin-2-yl)-1-piperidincarboxylat;
6-Cyclobutyl-2-(4-piperidinyl)-5,6,7,8-tetrahydro-4*H-*[1,3]thiazolo[4,5-*d*]azepin;
6-Cyclobutyl-2-{1-[(6-methyl-3-pyridinyl)carbonyl]-4-piperidinyl}-5,6,7,8-tetrahydro-4*H*-[1,3]thiazolo[4,5-*d*]azepin;
5-{[4-(6-Cyclobutyl-5,6,7,8-tetrahydro-4*H*-[1,3]thiazolo[4,5-*d*]azepin-2-yl)-1-piperidinyl]carbonyl}-2-pyridincarbonitril;
6-Cyclobutyl-2-(1-{[6-(trifluormethyl)-3-pyridinyl]carbonyl}-4-piperidinyl)-5,6,7,8-tetrahydro-4*H-*[1,3]thiazolo[4,5-*d*]azepin;
6-Cyclobutyl-2-{1-[(6-methyl-2-pyridinyl)carbonyl]4-piperidinyl}-5,6,7,8-tetrahydro-4*H-*[1,3]thiazolo[4,5-*d*]azepin;
6-Cyclobutyl-2-{1-[(5-methyl-2-pyrazinyl)carbonyl]-4-piperidinyl}-5,6,7,8-tetrahydro-4*H*-[1,3]thiazolo[4,5-*d*]azepin;
6-Cyclobutyl-2-{1-[(5-methyl-3-pyridinyl)carbonyl]-4-piperidinyl}-5,6,7,8-tetrahydro-4*H*-[1,3]thiazolo[4,5-*d*]azepin;
6-Cyclobutyl-2-{1-[(4-methylphenyl)carbonyl]-4-piperidinyl}-5,6,7,8-tetrahydro-4*H-*[1,3]thiazolo[4,5-*d*]azepin;
6-Cyclobutyl-2-{1-[(5-methyl-3-isoxazolyl)carbonyl]-4-piperidinyl}-5,6,7,8-tetrahydro-4*H*-[1,3]thiazolo[4,5-*d*]azepin;
6-Cyclobutyl-2-[1-(1,2,3-thiadiazol-4-ylcarbonyl)-4-piperidinyl]-5,6,7,8-tetrahydro-4*H-*[1,3]thiazolo[4,5-*d*]azepin;
6-Cyclobutyl-2-{1-[(1-methyl-1*H*-pyrazol-3-yl)carbonyl]-4-piperidinyl}-5,6,7,8-tetrahydro-4*H*-[1,3]thiazolo[4,5-*d*]azepin;
6-Cyclobutyl-2-{1-[(3-methyl-5-isoxazolyl)carbonyl]-4-piperidinyl}-5,6,7,8-tetrahydro-4*H*-[1,3]thiazolo[4,5-*d*]azepin;
6-Cyclobutyl-2-[1-(6-methyl-3-pyridinyl)-4-piperidinyl]-5,6,7,8-tetrahydro-4*H-*[1,3]thiazolo[4,5-*d*]azepin;
5-[4-(6-Cyclobutyl-5,6,7,8-tetrahydro-4*H*-[1,3]thiazolo[4,5-*d*]azepin-2-yl)-1-piperidinyl]-2-pyridincarbonitril;
5-[4-(6-Cyclobutyl-5,6,7,8-tetrahydro-4*H*-[1,3]thiazolo[4,5-*d*]azepin-2-yl)-1-piperidinyl]-2-pyridincarbonsäure;
5-[4-(6-Cyclobutyl-5,6,7,8-tetrahydro-4*H*-[1,3]thiazolo[4,5-*d*]azepin-2-yl)-1-piperidinyl]-2-pyridincarboxamid;
6-Cyclobutyl-2-{1-[6-(1*H*-imidazol-1-ylcarbonyl)-3-pyridinyl]-4-piperidinyl}-5,6,7,8-tetrahydro-4*H*-[1,3]thiazolo[4,5-*d*]azepin;
5-[4-(6-Cyclobutyl-5,6,7,8-tetrahydro-4*H*-[1,3]thiazolo[4,5-*d*]azepin-2-yl)-1-piperidinyl]-*N,N*-dimethyl-2-pyridincarboxamid;
6-Cyclobutyl-2-{1-[(2-methyl-3-pyridinyl)carbonyl]-4-piperidinyl}-5,6,7,8-tetrahydro-4*H*-[1,3]thiazolo[4,5-*d*]azepin;
1-[4-(6-Cyclobutyl-5,6,7,8-tetrahydro-4*H*-[1,3]thiazolo[4,5-*d*]azepin-2-yl)phenyl]-3-methyl-2-imidazolidinon;
1-[4-(6-Cyclobutyl-5,6,7,8-tetrahydro-4*H-*[1,3]thiazolo[4,5-*d*]azepin-2-yl)phenyl]-2-imidazolidinon;
1-[4-(6-Cyclopentyl-5,6,7,8-tetrahydro-4*H*-[1,3]thiazolo[4,5-*d*]azepin-2-yl)phenyl]-3-methyl-2-imidazolidinon;
1-Methyl-3-{4-[6-(1-methylethyl)-5,6,7,8-tetrahydro-4*H*-[1,3]thiazolo[4,5-*d*]azepin-2-yl]phenyl}-2-imidazolidinon;
1-[4-(6-Cyclohexyl-5,6,7,8-tetrahydro-4*H*-[1,3]thiazolo[4,5-*d*]azepin-2-yl)phenyl]-3-methyl-2-imidazolidinon;
1-{4-[6-(Cyclopropylmethyl)-5,6,7,8-tetrahydro-4*H*-[1,3]thiazolo[4,5-*d*]azepin-2-yl]phenyl}-3-methyl-2-imidazolidinon;
1-Methyl-3-{4-[6-(2-methylpropyl)-5,6,7,8-tetrahydro-4*H*[1,3]thiazolo[4,5-*d*]azepin-2-yl]phenyl}-2-imidazolidinon;
1,1-Dimethylethyl-3-(6-cyclobutyl-5,6,7,8-tetrahydro-4*H*-[1,3]thiazolo[4,5-*d*]azepin-2-yl)-1-pyrrolidincarboxylat;
6-Cyclobutyl-2-(3-pyrrolidinyl)-5,6,7,8-tetrahydro-4*H*[1,3]thiazolo[4,5-*d*]azepin;
(±)-5-[3-(6-Cyclobutyl-5,6,7,8-tetrahydro-4*H*-[1,3]thiazolo[4,5-*d*]azepin-2-yl)-1-pyrrolidinyl]-2-pyridincarbonitril;
(-)-5-[3-(6-Cyclobutyl-5,6,7,8-tetrahydro-4*H*-[1,3]thiazolo[4,5-*d*]azepin-2-yl)-1-pyrrolidinyl]-2-pyridincarbonitril;
(+)-5-[3-(6-Cyclobutyl-5,6,7,8-tetrahydro-4*H*-[1,3]thiazolo[4,5-*d*]azepin-2-yl)-1-pyrrolidinyl]-2-pyridincarbonitril;
(±)-6-Cyclobutyl-2-[1-(6-methyl-3-pyridinyl)-3-pyrrolidinyl]-5,6,7,8-tetrahydro-4*H-*[1,3]thiazolo[4,5-*d*]azepin;
5-[3-(6-Cyclobutyl-5,6,7,8-tetrahydro-4*H*-[1,3]thiazolo[4,5-*d*]azepin-2-yl)-1-pyrrolidinyl]-3-pyridincarbonitril;
Methyl-4-(6-cyclobutyl-5,6,7,8-tetrahydro-4*H-*[1,3]thiazolo[4,5-*d*]azepin-2-yl)benzoat;
4-(6-Cyclobutyl-5,6,7,8-tetrahydro-4*H*-[1,3]thiazolo[4,5-*d*]azepin-2-yl)benzoesäure;
6-Cyclobutyl-2-phenyl-5,6,7,8-tetrahydro-4*H*-[1,3]thiazolo[4,5-*d*]azepin;
4-(6-Cyclobutyl-5,6,7,8-tetrahydro-4*H*-[1,3]thiazolo[4,5-*d*]azepin-2-yl)-*N-*methylbenzamid;
3-[4-(6-Cyclobutyl-5,6,7,8-tetrahydro-4*H*-[1,3]thiazolo[4,5-*d*]azepin-2-yl)phenyl]-1,3-oxazolidin-2-on;
6-Cyclobutyl-2-[4-(1-pyrrolidinylcarbonyl)phenyl]-5,6,7,8-tetrahydro-4*H-*[1,3]thiazolo[4,5-*d*]azepin;
4-(6-Cyclobutyl-5,6,7,8-tetrahydro-4*H*-[1,3]thiazolo[4,5-*d*]azepin-2-yl)benzamid;
4-(6-Cyclobutyl-5,6,7,8-tetrahydro-4*H*-[1,3]thiazolo[4,5-*d*]azepin-2-yl)-*N-*ethylbenzamid;
4-(6-Cyclobutyl-5,6,7,8-tetrahydro-4*H*-[1,3]thiazolo[4,5-*d*]azepin-2-yl)benzonitril;
5-(6-Cyclobutyl-5,6,7,8-tetrahydro-4*H*-[1,3]thiazolo[4,5-*d*]azepin-2-yl)-2-pyridincarbonitril;
5-(6-Cyclobutyl-5,6,7,8-tetrahydro-4*H-*[1,3]thiazolo[4,5-*d*]azepin-2-yl)-2-pyridincarbonsäure;
5-(6-Cyclobutyl-5,6,7,8-tetrahydro-4*H*-[1,3]thiazolo[4,5-*d*]azepin-2-yl)-*N*-methyl-2-pyridincarboxamid;
1-[5-(6-Cyclobutyl-5,6,7,8-tetrahydro-4*H*-[1,3]thiazolo[4,5-*d*]azepin-2-yl)-2-pyridinyl]-2-pyrrolidinon;
1-[5-(6-Cyclobutyl-5,6,7,8-tetrahydro-4*H*-[1,3]thiazolo[4,5-*d*]azepin-2-yl)-2-pyridinyl]-3-methyl-2-imidazolidinon;
2-(6-Chlor-3-pyridinyl)-6-cyclobutyl-5,6,7,8-tetrahydro-4*H-*[1,3]thiazolo[4,5-*d*]azepin;
6-Cyclobutyl-2-[6-(1-piperidinyl)-3-pyridinyl]-5,6,7,8-tetrahydro-4*H*-[1,3]thiazolo[4,5-*d*]azepin;
5-(6-Cyclobutyl-5,6,7,8-tetrahydro-4*H-*[1,3]thiazolo[4,5-*d*]azepin-2-yl)-2-pyrimidincarbonitril;
6-Cyclobutyl-2-[2-(methyloxy)-5-pyrimidinyl]-5,6,7,8-tetrahydro-4*H*-[1,3]thiazolo[4,5-*d*]azepin;
6-Cyclobutyl-2-(5-pyrimidinyl)-5,6,7,8-tetrahydro-4*H*-[1,3]thiazolo[4,5-*d*]azepin;
5-(6-Cyclobutyl-5,6,7,8-tetrahydro-4*H-*[1,3]thiazolo[4,5-*d*]azepin-2-yl)-2(1*H*)-pyrimidinon;
2-(2-Chlor-5-pyrimidinyl)-6-cyclobutyl-5,6,7,8-tetrahydro-4*H*-[1,3]thiazolo[4,5 - *d*]azepin;
6-Cyclobutyl-2-[2-(1-piperidinyl)-5-pyrimidinyl]-5,6,7,8-tetrahydro-4*H-*[1,3]thiazolo[4,5-*d*]azepin;
1-{4-[6-(Cyclohexylmethyl)-5,6,7,8-tetrahydro-4*H*-[1,3]thiazolo[4,5-*d*]azepin-2-yl]phenyl}-3-methyl-2-imidazolidinon;
1-[4-(6-Ethyl-5,6,7,8-tetrahydro-4*H*-[1,3]thiazolo[4,5-*d*]azepin-2-yl)phenyl]-3-methyl-2-imidazolidinon;
6-Cyclobutyl-2-{1-[(6-methyl-3-pyridazinyl)carbonyl]-4-piperidinyl}-5,6,7,8-tetrahydro-4*H-*[1,3]thiazolo[4,5-*d*]azepin;
6-Cyclobutyl-2-{1-[(6-methyl-3-pyridinyl)carbonyl]-3-pyrrolidinyl}-5,6,7,8-tetrahydro-4*H*-[1,3]thiazolo[4,5-*d*]azepin;
5-[4-(6-Cyclobutyl-5,6,7,8-tetrahydro-4*H*-[1,3]thiazolo[4,5-*d*]azepin-2-yl)-1-piperidinyl]-3-pyridincarbonitril;
4-[4-(6-Cyclobutyl-5,6,7,8-tetrahydro-4*H*-[1,3]thiazolo[4,5-*d*]azepin-2-yl)-1-piperidinyl]benzonitril;
4-(6-Cyclobutyl-5,6,7,8-tetrahydro-4*H-*[1,3]thiazolo[4,5-*d*]azepin-2-yl)-*N*-methyl-1-piperidincarboxamid;
4-(6-Cyclobutyl-5,6,7,8-tetrahydro-4*H*-[1,3]thiazolo[4,5-*d*]azepin-2-yl)-*N*-(1-methylethyl)-1-piperidincarboxamid;
4-(6-Cyclobutyl-5,6,7,8-tetrahydro-4*H*-[1,3]thiazolo[4,5-*d*]azepin-2-yl)-*N*-(2-methylphenyl)-1-piperidincarboxamid;
4-(6-Cyclobutyl-5,6,7,8-tetrahydro-4*H*-[1,3]thiazolo[4,5-*d*]azepin-2-yl)-*N*-(3-methylphenyl)-1-piperidincarboxamid;
4-(6-Cyclobutyl-5,6,7,8-tetrahydro-4*H*-[1,3]thiazolo[4,5-*d*]azepin-2-yl)-*N*-(4-methylphenyl)-1-piperidincarboxamid;
6-(6-Cyclobutyl-5,6,7,8-tetrahydro-4*H*-[1,3]thiazolo[4,5-*d*]azepin-2-yl)-3-pyridincarbonitril;
6-(6-Cyclopentyl-5,6,7,8-tetrahydro-4*H*-[1,3]thiazolo[4,5-*d*]azepin-2-yl)-3-pyridincarbonitril;
6-(6-Cyclobutyl-5,6,7,8-tetrahydro-4*H*-[1,3]thiazolo[4,5-*d*]azepin-2-yl)-3-pyridincarbonsäure;
6-(6-Cyclobutyl-5,6,7,8-tetrahydro-4*H-*[1,3]thiazolo[4,5-*d*]azepin-2-yl)-*N*-methyl-3-pyridincarboxamid;
5-[4-(6-Cyclobutyl-5,6,7,8-tetrahydro-4*H-*[1,3]thiazolo[4,5-*d*]azepin-2-yl)-1-piperidinyl]-*N*-methyl-2-pyridincarboxamid;
6-Cyclobutyl-2-{1-[(2-methylphenyl)carbonyl]-4-piperidinyl}-5,6,7,8-tetrahydro-4*H-*[1,3]thiazolo[4,5-*d*]azepin;
6-Cyclobutyl-2-{1-[(3-methyl-1*H*-pyrazol-5-yl)carbonyl]-4-piperidinyl}-5,6,7,8-tetrahydro-4*H*-[1,3]thiazolo[4,5-*d*]azepin;
2-{1-[(4-Chlorphenyl)carbonyl]-4-piperidinyl}-6-cyclobutyl-5,6,7,8-tetrahydro-4*H-*[1,3]thiazolo[4,5-*d*]azepin;
2-{1-[(3-Chlorphenyl)carbonyl]-4-piperidinyl}-6-cyclobutyl-5,6,7,8-tetrahydro-4*H-*[1,3]thiazolo[4,5-*d*]azepin;
6-Cyclobutyl-2-{1-[(3-methylphenyl)carbonyl]-4-piperidinyl}-5,6,7,8-tetrahydro-4*H-*[1,3]thiazolo[4,5-*d*]azepin;
6-Cyclobutyl-2-{1-[(3-methyl-2-pyridinyl)carbonyl]-4-piperidinyl}-5,6,7,8-tetrahydro-4*H*-[1,3]thiazolo[4,5-*d*]azepin;
6-Cyclobutyl-2-[1-(1*H*-pyrazol-3-ylcarbonyl)-4-piperidinyl]-5,6,7,8-tetrahydro-4*H-*[1,3]thiazolo[4,5-*d*]azepin;
6-Cyclobutyl-2-{1-[(1,4-dimethyl-1*H*-pyrazol-3-yl)carbonyl]-4-piperidinyl}-5,6,7,8-tetrahydro-4*H*-[1,3]thiazolo[4,5-*d*]azepin;
6-Cyclobutyl-2-{1-[(1-methyl-1*H*-pyrrol-2-yl)carbonyl]-4-piperidinyl}-5,6,7,8-tetrahydro-4*H*-[1,3]thiazolo[4,5-*d*]azepin;
6-Cyclobutyl-2-{1-[(1-methyl-1*H*-imidazol-5-yl)carbonyl]-4-piperidinyl}-5,6,7,8-tetrahydro-4*H*-[1,3]thiazolo[4,5-*d*]azepin;
6-Cyclobutyl-2-{1-[(1,5-dimethyl-1*H*-pyrazol-3-yl)carbonyl]-4-piperidinyl}-5,6,7,8-tetrahydro-4*H*-[1,3]thiazolo[4,5-*d*]azepin;
6-Cyclobutyl-2-{1-[(1-methyl-1*H*-imidazol-4-yl)carbonyl]-4-piperidinyl}-5,6,7,8-tetrahydro-4*H*-[1,3]thiazolo[4,5-*d*]azepin;
6-Cyclobutyl-2-[1-(2-pyridiriylcarbonyl)-4-piperidinyl]-5,6,7,8-tetrahydro-4*H-*[1,3]thiazolo[4,5-*d*]azepin;
6-Cyclobutyl-2-[1-(3-pyridinylcarbonyl)-4-piperidinyl]-5,6,7,8-tetrahydro-4*H-*[1,3]thiazolo[4,5-*d*]azepin;
6-Cyclobutyl-2-{1-[(6-methyl-2-pyrazinyl)carbonyl]-4-piperidinyl}-5,6,7,8-tetrahydro-4*H*-[1,3]thiazolo[4,5-*d*]azepin;
6-Cyclobutyl-2-{1-[(2-methyl-4-pyridinyl)carbonyl]-4-piperidinyl}-5,6,7,8-tetrahydro-4*H*-[1,3]thiazolo[4,5-*d*]azepin;
6-Cyclobutyl-2-[1-(4-pyridinylcarbonyl)-4-piperidinyl]-5,6,7,8-tetrahydro-4H-[1,3]thiazolo[4,5-d]azepin;
6-Cyclobutyl-2-{1-[(2-methyl-4-pyrimidinyl)carbonyl]-4-piperidinyl}-5,6,7,8-tetrahydro-4H-[1,3]thiazolo[4,5-d]azepin;
6-Cyclobutyl-2-{1-[(2-methyl-5-pyrimidinyl)carbonyl]-4-piperidinyl}-5,6,7,8-tetrahydro-4H-[1,3]thiazolo[4,5-d]azepin;
6-Cyclobutyl-2-[1-(4-morpholinylcarbonyl)-4-piperidinyl]-5,6,7,8-tetrahydro-4H-[1,3]thiazolo[4,5-d]azepin;
6-Cyclobutyl-2-[1-(1-pyrrolidinylcarbonyl)-4-piperidinyl]-5,6,7,8-tetrahydro-4H-[1,3]thiazolo[4,5-d]azepin;
6-Cyclobutyl-2-[4-(1,1-dioxido-2-isothiazolidinyl)phenyl]-5,6,7,8-tetrahydro-4H-[1,3]thiazolo[4,5-d]azepin;
5-(6-Cyclobutyl-5,6,7,8-tetrahydro-4H-[1,3]thiazolo[4,5-d]azepin-2-yl)-2-pyridincarboxamid;
6-Cyclobutyl-2-[6-(1-pyrrolidinylcarbonyl)-3-pyridinyl]-5,6,7,8-tetrahydro-4H-[1,3]thiazolo[4,5-d]azepin;
6-Cyclobutyl-2-[6-(3-methyl-1,2,4-oxadiazol-5-yl)-3-pyridinyl]-5,6,7,8-tetrahydro-4H-[1,3]thiazolo[4,5-d]azepin;
1-[5-(6-Cyclobutyl-5,6,7,8-tetrahydro-4H-[1,3]thiazolo[4,5-d]azepin-2-yl)-2-pyridinyl]-2-imidazolidinon;
Methyl-5-(6-cyclobutyl-5,6,7,8-tetrahydro-4H-[1,3]thiazolo[4,5-d]azepin-2-yl)-2-pyrazincarboxylat;
4-{[3-(6-Cyclobutyl-5,6,7,8-tetrahydro-4H-[1,3]thiazolo[4,5-d]azepin-2-yl)-1-pyrrolidinyl]carbonyl}benzonitril;
6-Cyclobutyl-2-{1-[(6-methyl-2-pyridinyl)carbonyl]-3-pyrrolidinyl}-5,6,7,8-tetrahydro-4H-[1,3]thiazolo[4,5-d]azepin;
6-Cyclobutyl-2-[6-(1-pyrrolidinyl)-3-pyridinyl]-5,6,7,8-tetrahydro-4H-[1,3]thiazolo[4,5-d]azepin;
6-Cyclobutyl-2-[6-(4-morpholinyl)-3-pyridinyl]-5,6,7,8-tetrahydro-4H-[1,3]thiazolo[4,5-d]azepin;
5-(6-Cyclobutyl-5,6,7,8-tetrahydro-4H-[1,3]thiazolo[4,5-d]azepin-2-yl)-N-methyl-2-pyrazincarboxamid;
6-Cyclobutyl-2-[4-(4-morpholinyl)phenyl]-5,6,7,8-tetrahydro-4H-[1,3]thiazolo[4,5-d]azepin;
6-Cyclobutyl-2-(4-methyl-3,4-dihydro-2H-1,4-benzoxazin-7-yl)-5,6,7,8-tetrahydro-4H-[1,3]thiazolo[4,5-d]azepin;
3-(6-Cyclobutyl-5,6,7,8-tetrahydro-4H-[1,3]thiazolo[4,5-d]azepin-2-yl)benzonitril;
3-(6-Cyclopentyl-5,6,7,8-tetrahydro-4H-[1,3]thiazolo[4,5-d]azepin-2-yl)benzonitril;
(±)-6-Cyclobutyl-2-[1-(6-methyl-2-pyridinyl)-3-pyrrolidinyl]-5,6,7,8-tetrahydro-4H-[1,3]thiazolo[4,5-d]azepin;
4-{[4-(6-Cyclobutyl-5,6,7,8-tetrahydro-4H-[1,3]thiazolo[4,5-d]azepin-2-yl)-1-piperidinyl]carbonyl}benzonitril;
4-(6-Cyclobutyl-5,6,7,8-tetrahydro-4H-[1,3]thiazolo[4,5-d]azepin-2-yl)-N,N-dimethylanilin;
oder ein pharmazeutisch verträgliches Salz oder Solvat davon.

11. Verbindung gemäß Anspruch 10, nämlich:
1-[4-(6-Cyclobutyl-5,6,7,8-tetrahydro-4*H*-[1,3]thiazolo[4,5-*d*]azepin-2-yl)phenyl]-2-pyrrolidinon;
6-Cyclobutyl-2-{1-[(6-methyl-2-pyridinyl)carbonyl]-4-piperidinyl}-5,6,7,8-tetrahydro-4*H*-[1,3]thiazolo[4,5-*d*]azepin;
6-Cyclobutyl-2-{1-[(5-methyl-3-pyridinyl)carbonyl]-4-piperidinyl}-5,6,7,8-tetrahydro-4*H*-[1,3]thiazolo[4,5-*d*]azepin;
5-[4-(6-Cyclobutyl-5,6,7,8-tetrahydro-4*H*-[1,3]thiazolo[4,5-*d*]azepin-2-yl)-1-piperidinyl]-2-pyridincarbonitril;
5-[4-(6-Cyclobutyl-5,6,7,8-tetrahydro-4*H*-[1,3]thiazolo[4,5-*d*]azepin-2-yl)-1-piperidinyl]-*N*,*N*-dimethyl-2-pyridincarboxamid;
1-[4-(6-Cyclobutyl-5,6,7,8-tetrahydro-4*H*-[1,3]thiazolo[4,5-*d*]azepin-2-yl)phenyl]-3-methyl-2-imidazolidinon;
1-[4-(6-Cyclobutyl-5,6,7,8-tetrahydro-4*H*-[1,3]thiazolo[4,5-*d*]azepin-2-yl)phenyl]-2-imidazolidinon;
(+)-5-[3-(6-Cyclobutyl-5,6,7,8-tetrahydro-4*H*-[1,3]thiazolo[4,5-*d*]azepin-2-yl)-1-pyrrolidinyl]-2-pyridincarbonitril;
(±)-6-Cyclobutyl-2-[1-(6-methyl-3-pyridinyl)-3-pyrrolidinyl]-5,6,7,8-tetrahydro-4*H-*[1,3]thiazolo[4,5-*d*]azepin;
oder ein pharmazeutisch verträgliches Salz oder Solvat davon.

12. Verbindung gemäß Anspruch 10, nämlich:
4-(6-Cyclobutyl-5,6,7,8-tetrahydro-4*H*-[1,3]thiazolo[4,5-*d*]azepin-2-yl)-*N*-methyl-1-piperidincarboxamid 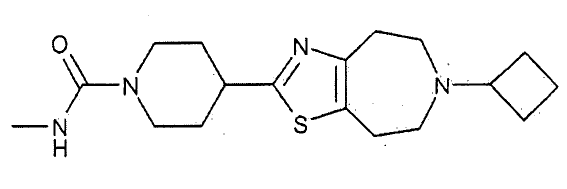
oder ein pharmazeutisch verträgliches Salz oder Solvat davon.

13. Arzneimittel, welches die Verbindung der Formel (I), wie in einem der vorstehenden Ansprüche definiert, oder ein pharmazeutisch verträgliches Salz oder Solvat davon, und einen pharmazeutisch verträglichen Träger oder Exzipienten umfasst.

14. Verbindung wie in einem der Ansprüche 1 bis 12 definiert, zur Verwendung in der Therapie.

15. Verbindung wie in einem der Ansprüche 1 bis 12 definiert, zur Verwendung bei der Behandlung von neurologischen Erkrankungen.

16. Verbindung der Formel (I), oder ein pharmazeutisch verträgliches Salz oder Solvat davon, gemäß einem der Ansprüche 1 bis 12, zur Verwendung als eine therapeutische Substanz bei der Behandlung oder Vorbeugung von kognitiven Verschlechterungen bei der Alzheimer-Krankheit oder einer damit verbundenen neurodegenerativen Störung.

17. Verwendung einer Verbindung wie in einem der Ansprüche 1 bis 12 definiert, zur Herstellung eines Medikaments zur Behandlung von neurologischen Erkrankungen.

18. Verfahren zur Herstellung einer Verbindung der Formel (I), oder eines pharmazeutisch verträglichen Salzes oder Solvats davon, wie in einem der Ansprüche 1 bis 12 definiert,
wobei das Verfahren umfasst:
(a) Umsetzen einer Verbindung der Formel (II) 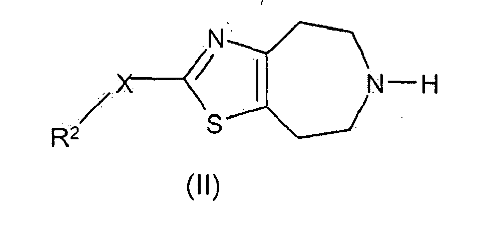 wobei X und R² wie in Anspruch 1 definiert sind, mit einer Verbindung der Formel R¹=O, wobei R^{1'} =C₂₋₆-Alkyl, =C₁₋₃-Alkyl-C₃₋₈-cycloalkyl oder =C₃₋₇-Cycloalkyl ist, wobei die Cycloalkylreste gegebenenfalls mit C₁₋₃-Alkyl substituiert sein können; oder
(b) Umsetzen einer Verbindung der Formel (III) 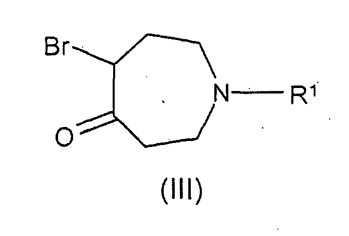 wobei R¹ wie in Anspruch 1 definiert ist, mit einer Verbindung der Formel R²X-C(=S)NH₂, wobei R² und X wie in Anspruch 1 definiert sind; oder
(c) Entschützen einer Verbindung der Formel (I), welche geschützt ist; oder
(d) Gegenseitiges Umwandeln einer Verbindung der Formel (I) in die andere; oder
(e) Umsetzen einer Verbindung der Formel (X) 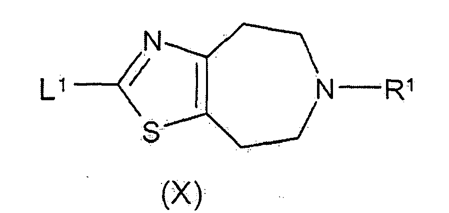 wobei R¹ wie in Anspruch 1 definiert ist und L¹ eine Abgangsgruppe, wie Halogen, darstellt, mit einer metallorganischen Verbindung der Formel (XI), R²-X-L², wobei R² und X wie in Anspruch 1 definiert sind und wobei L² eine Abgangsgruppe ist; oder
(f) Umsetzen einer Verbindung der Formel (II) 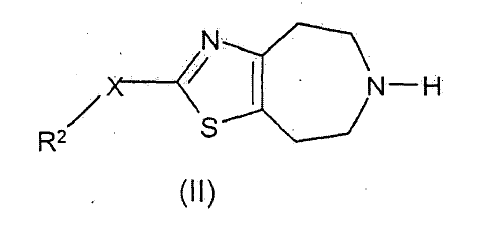 wobei X und R² wie in Anspruch 1 definiert sind, mit einer Verbindung der Formel R¹-L³, wobei R¹ wie in Anspruch 1 definiert ist und wobei L³ eine Abgangsgruppe, wie Halogen, ist.

## Revendications

1. Composé de formule (I) ou un de ses sels ou produits de solvatation pharmaceutiquement acceptables : 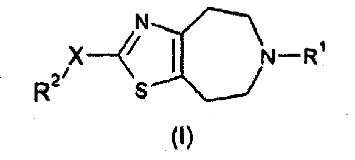 formule dans laquelle :
R¹ représente un groupe -alkyle en C₂ à C₆, -(alkyle en C₁ à C₃) (cycloalkyle en C₃ à C₈) ou -cycloalkyle en C₃ à C₇, les groupes cycloalkyle pouvant être facultativement substitués avec un substituant alkyle en C₁ à C₃ ;
X représente un groupe aryle, hétéroaryle ou hétérocyclyle ;
R² représente un atome d'hydrogène, un atome d'halogène, un groupe hydroxy, cyano, nitro, =O, -NR⁸R⁹, -Y-H, -Y-(alkyle en C₁ à C₆), -Y-(cycloalkyle en C₃ à C₈), -Y-(alkyle en C₁ à C₆) (cycloalkyle en C₃ à C₈) -Y-aryle, -Y-hétérocyclyle, -Y-hétéroaryle, -Y-(alkyle en C₁ à C₆)aryle, - (alkyle en C₁ à C₆) -Y-H, - (alkyle en C₁ à C₆) -Y- (alkyle en C₁ à C₆), - (alkyle en C₁ à C₆)-Y-(cycloalkyle en C₃ à C₈), - (alkyle en C₁ à C₆) -Y- (alkyle en C₁ à C₆) (cycloalkyle en C₃ à C₈), -(alkyle en C₁ à C₆) -Y-aryle, - (alkyle en C₁ à C₆) -Y-hétérocyclyle ou -(alkyle en C₁ à C₆)-Y-hétéroaryle, dans lequel R⁸ et R⁹ sont choisis indépendamment dans le groupe consistant en un atome d'hydrogène et un groupe méthyle ;
Y représente une liaison, un groupe alkyle en C₁ à C₆, CO, CO₂, CONR³, NR³CO, O, S, SO, SO₂, -SO₂-O-, SO₂NR³, NR³SO₂, OCONR³, NR³CO₂ ou NR³CONR⁴ (dans lequel R³ et R⁴ représentent indépendamment un atome d'hydrogène, un groupe -alkyle en C₁ à C₆, -cycloalkyle en C₃ à C₈, - (alkyle en C₁ à C₆) - (cycloalkyle en C₃ à C₈), -aryle, -hétérocyclyle ou -hétéroaryle) ;
lesdits groupes aryle, hétéroaryle et hétérocyclyle de X pouvant être facultativement substitués avec 1, 2 ou 3 substituants qui peuvent être identiques ou différents et qui sont choisis dans le groupe consistant en des substituants halogéno, hydroxy, cyano, amino, nitro, =O, alkyle en C₁ à C₆, alkoxy en C₁ à C₆ et halogénalkyle en C₁ à C₆ ;
lesdits groupes alkyle, cycloalkyle, aryle, hétéroaryle et hétérocyclyle de R² pouvant être facultativement substitués avec 1, 2 ou 3 substituants qui peuvent être identiques ou différents et qui sont des substituants halogéno, cyano, nitro, =O, ou un groupe -R⁵, -OR⁵, -O(alkyle en C₁ à C₆) -R⁶, -(alkyle en C₁ à C₆) -OR⁶, -CO₂R⁵, -COR⁵, COR⁵R⁶, -(alkyle en C₁ à C₆) -COR⁵, -SR⁵, -SO₂R⁵, -SOR⁵, -OSO₂R⁵, -(alkyle en C₁ à C₆) -SO₂R⁶, - (alkyle en C₁ à C₆) -NR⁵SO₂R⁶, -(alkyle en C₁ à C₆)-SO₂NR⁵R⁶, -NR⁵R⁶, -(alkyle en C₁ à C₆)-NR⁵R⁶, -(cycloalkyle en C₃ à C₈) -NR⁵R⁶, -CONR⁵R⁶, -NR⁵COR⁶, -(alkyle en C₁ à C₆) -NR⁵COR⁶, -(alkyle en C₁ à C₆) -CONR⁵R⁶, -NR⁵SO₂R⁶, -OCONR⁵R⁶, -NR⁵CO₂R⁶, -NR⁷CONR⁵R⁶ ou -SO₂NR⁵R⁶ (dans lesquels R⁵, R⁶ et R⁷ représentent indépendamment un atome d'hydrogène, un groupe alkyle en C₁ à C₆, -cycloalkyle en C₃ à C₈, -(alkyle en C₁ à C₆)-(cycloalkyle en C₃ à C₈), aryle, hétérocyclyle ou hétéroaryle, ou dans lesquels -NR⁵R⁶ peut représenter un groupe hétérocyclyle azoté), sous réserve que, lorsque R¹ représente un groupe -alkyle en C₂ à C₆ ou (alkyle en C₁ à C₃) - (cycloalkyle en C₃ à C₈), les groupes alkyle, cycloalkyle, aryle, hétéroaryle et hétérocyclyle de R² ne peuvent pas être substitués avec un substituant -CO₂R⁵ ;
où R³, R⁴, R⁵, R⁶ et R⁷ peuvent être facultativement substitués avec 1, 2 ou 3 substituants qui peuvent être identiques ou différents et qui sont choisis dans le groupe consistant en des substituants halogéno, hydroxy, cyano, amino, nitro, =O, alkyle en C₁ à C₆, alkoxy en C₁ à C₆ et halogénalkyle en C₁ à C₆ ; et sous réserve que les groupes aryle et hétéroaryle de X, R², R³, R⁴, R⁵, R⁶ et R⁷ peuvent seulement être substitués avec un substituant =O si le groupe substitué est aromatique ;
ou ses produits de solvatation.

2. Composé suivant la revendication 1, dans lequel R¹ représente un groupe -cycloalkyle en C₃ à C₇ non substitué.

3. Composé suivant la revendication 2, dans lequel R¹ représente un groupe cyclobutyle non substitué.

4. Composé suivant l'une quelconque des revendications précédentes, dans lequel R² représente un groupe
-hydrogène ;
-halogène ;
-cyano ;
=O ;
-Y-H ;
-Y-(alkyle en C₁ à C₆) ;
-Y-aryle ;
-Y-hétérocyclyle ; ou
-Y-hétéroaryle.

5. Composé suivant l'une quelconque des revendications précédentes, dans lequel Y représente une liaison, un groupe O, CO, CO₂ ou CONR³, dans lequel R³ représente un atome d'hydrogène.

6. Composé suivant l'une quelconque des revendications précédentes, dans lequel lesdits groupes alkyle, aryle, hétéroaryle et hétérocyclyle de R² peuvent être facultativement substitués avec 1, 2 ou 3 substituants qui peuvent être identiques ou différents et qui sont choisis dans le groupe consistant en des substituants cyano, halogéno, =O, R⁵, COR⁵, CO₂R⁵ et -CONR⁵R⁶ (dans lesquels R⁵ et R⁶ représentent indépendamment un atome d'hydrogène, un groupe -alkyle en C₁ à C₆ ou hétérocyclyle, et dans lesquels R⁵ et R⁶ peuvent être en outre facultativement substitués avec 1, 2 ou 3 substituants choisis dans le groupe consistant en des substituants halogéno et -alkyle en C₁ à C₆), sous réserve que, lorsque R¹ représente un groupe -alkyle en C₂ à C₆ ou -(alkyle en C₁ à C₃)-(cycloalkyle en C₃ à C₈), les groupes alkyle, aryle, hétéroaryle et hétérocyclyle de R² ne peuvent pas être substitués avec un substituant -CO₂R⁵, et sous réserve que les groupes aryle et hétéroaryle de R² peuvent seulement être substitués avec un substituant =O si le groupe substitué est aromatique.

7. Composé de formule (I) ou un de ses sels ou produits de solvatation pharmaceutiquement acceptables suivant la revendication 1, 2 ou 3, dans lequel :
R¹ représente un groupe -alkyle en C₂ à C₆, -(alkyle en C₁ à C₃) - (cycloalkyle en C₃ à C₈) ou -cycloalkyle en C₃ à C₇, les groupes cycloalkyle pouvant être facultativement substitués avec un substituant alkyle en C₁ à C₃ ;
X représente un groupe aryle, hétéroaryle ou hétérocyclyle ;
R² représente un atome d'hydrogène, un atome d'halogène, un groupe cyano, =O, -Y-H, -Y-(alkyle en C₁ à C₆), -Y-aryle, -Y-hétérocyclyle, -Y-hétéroaryle ou -NR⁸R⁹, dans lequel R⁸ et R⁹ sont choisis indépendamment dans le groupe consistant en un atome d'hydrogène et un groupe méthyle ;
Y représente une liaison, un groupe O, CO, CO₂ ou CONR³, dans lequel R³ représente un atome d'hydrogène ;
lesdits groupes aryle, hétéroaryle et hétérocyclyle de X pouvant être facultativement substitués avec 1, 2 ou 3 substituants qui peuvent être identiques ou différents et qui sont choisis dans le groupe consistant en des substituants halogéno, alkyle en C₁ à C₆, alkoxy en C₁ à C₆ et halogénalkyle en C₁ à C₆
lesdits groupes alkyle, aryle, hétéroaryle et hétérocyclyle de R² pouvant être facultativement substitués avec 1, 2 ou 3 substituants qui peuvent être identiques ou différents et qui sont choisis dans le groupe consistant en des substituants cyano, halogéno, =O, R⁵, COR⁵, CO₂R⁵ et -CONR⁵R⁶ (dans lesquels R⁵ et R⁶ représentent indépendamment un atome d'hydrogène, un groupe -alkyle en C₁ à C₆ ou hétérocyclyle, et dans lesquels R⁵ et R⁶ peuvent être en outre facultativement substitués avec 1, 2 ou 3 substituants choisis dans le groupe consistant en des substituants halogéno et -alkyle en C₁ à C₆) ;
sous réserve que, lorsque R¹ représente un groupe -alkyle en C₂ à C₆ ou - (alkyle en C₁ à C₃) - (cycloalkyle en C₃ à C₈), les groupes alkyle, aryle, hétéroaryle et hétérocyclyle de R² ne peuvent pas être substitués avec un substituant -CO₂R⁵, et sous réserve que les groupes aryle et hétéroaryle de X et R² peuvent seulement être substitués avec un substituant =O si le groupe substitué est aromatique.

8. Composé de formule (I) ou un de ses sels ou produits de solvatation pharmaceutiquement acceptables suivant la revendication 7, dans lequel :
R¹ représente un groupe - (alkyle en C₁ à C₃)-(cycloalkyle en C₃ à C₈) ou -cycloalkyle en C₃ à C₇ ;
X représente un groupe aryle, hétéroaryle ou hétérocyclyle ;
R² représente un atome d'hydrogène, un atome d'halogène, un groupe -Y-(alkyle en C₁ à C₆), Y-aryle, -Y-hétérocyclyle ou -Y-hétéroaryle ;
Y représente une liaison ou un groupe CO ;
lesdits groupes alkyle, aryle, hétéroaryle et hétérocyclyle de R² pouvant être facultativement substitués avec 1, 2 ou 3 substituants qui peuvent être identiques ou différents et qui sont choisis dans le groupe consistant en des substituants cyano, =O, R⁵, -COR⁵, -CO₂R⁵ et -CONR⁵R⁶ (dans lesquels R⁵ et R⁶ représentent indépendamment un atome d'hydrogène ou un groupe -alkyle en C₁ à C₆, et dans lesquels R⁵ et R⁶ peuvent être en outre facultativement substitués avec 1, 2 ou 3 atomes d'halogène) ;
sous réserve que, lorsque R¹ représente un groupe -(alkyle en C₁ à C₃) - (cycloalkyle en C₃ à C₈), les groupes alkyle, aryle, hétéroaryle et hétérocyclyle de R² ne peuvent pas être substitués avec un substituant -CO₂R⁵, et
sous réserve que les groupes aryle et hétéroaryle de R² peuvent seulement être substitués avec un substituant =O si le groupe substitué est aromatique.

9. Composé de formule (I) ou un de ses sels ou produits de solvatation pharmaceutiquement acceptables suivant la revendication 8, dans lequel :
R¹ représente un groupe -(alkyle en C₁ à C₃)-(cycloalkyle en C₃ à C₈) ou -cycloalkyle en C₃ à C₇ ;
X représente un groupe aryle, hétéroaryle ou hétérocyclyle
R² représente un atome d'hydrogène, un atome d'halogène, un groupe -Y-(alkyle en C₁ à C₆), Y-aryle, -Y-hétérocyclyle ou -Y-hétéroaryle ;
Y représente une liaison ou un groupe CO ;
lesdits groupes alkyle, aryle, hétéroaryle et hétérocyclyle de R² pouvant être facultativement substitués avec 1, 2 ou 3 substituants qui peuvent être identiques ou différents et qui sont choisis dans le groupe consistant en des substituants cyano, =O, R⁵, -COR⁵ et -CONR⁵R⁶ (dans lesquels R⁵ et R⁶ représentent indépendamment un atome d'hydrogène ou un groupe -alkyle en C₁ à C₆, et dans lesquels R⁵ et R⁶ peuvent être en outre facultativement substitués avec 1, 2 ou 3 atomes d'halogène) ;
sous réserve que les groupes aryle et hétéroaryle de R² peuvent seulement être substitués avec un substituant =O si le groupe substitué est aromatique.

10. Composé suivant la revendication 1, qui est :
la 6-cyclobutyl-2-[4-(trifluorométhyl)phényl]-5,6,7,8-tétrahydro-4*H*-[1,3]thiazolo[4,5-*d*]azépine ;
la 2-(4-bromophényl)-6-cyclobutyl-5,6,7,8-tétrahydro-4*H*-[1,3]thiazolo[4,5-*d*]azépine ;
la 1-[4-(6-cyclobutyl-5,6,7,8-tétrahydro-4*H*-[1,3]thiazolo-[4,5-*d*]azépine-2-yl)phényl]-2-pyrrolidinone ;
la 6-cyclobutyl-2-[6-(trifluorométhyl)-3-pyridinyl]-5,6,7,8-tétrahydro-4*H*-[1,3]thiazolo[4,5-*d*]azépine ;
la 6-cyclobutyl-2-[4-(1,2,3-thiadiazole-4-yl)phényl]-5,6,7,8-tétrahydro-4*H*-[1,3]thiazolo[4,5-*d*]azépine ;
le 4-(6-cyclobutyl-5,6,7,8-tétrahydro-4*H*-[1,3]thiazolo-[4,5-*d*]azépine-2-yl)-1-pipéridinecarboxylate de 1,2-diméthyléthyle ;
la 6-cyclobutyl-2-(4-pipéridinyl)-5,6,7,8-tétrahydro-4*H*-[1,3]thiazolo[4,5-*d*]azépine ;
la 6-cyclobutyl-2-{1-[(6-méthyl-3-pyridinyl)carbonyl]-4-pipéridinyl}-5,6,7,8-tétrahydro-4*H*-[1,3]thiazolo[4,5-*d*]-azépine ;
le 5-{[4-(6-cyclobutyl-5,6,7,8-tétrahydro-4*H*-[1,3]thiazolo-[4,5-*d*]azépine-2-yl)-1-pipéridinyl]carbonyl}-2-pyridinecarbonitrile ;
la 6-cyclobutyl-2-(1-{[6-(trifluorométhyl)-3-pydidinyl]-carbonyl}-4-pipéridinyl)-5,6,7,8-tétrahydro-4*H*-[1,3]thiazolo-[4,5-*d*]azépine ;
la 6-cyclobutyl-2-{1-[(6-méthyl-2-pyridinyl)carbonyl]-4-pipéridinyl}-5,6,7,8-tétrahydro-4*H*-[1,3]thiazolo[4,5-*d*]-azépine ;
la 6-cyclobutyl-2-{1-[(5-méthyl-2-pyrazinyl)carbonyl]-4-pipéridinyl}-5,6,7,8-tétrahydro-4*H*-[1,3]thiazolo[4,5-*d*]-azépine ;
la 6-cyclobutyl-2-{1-[(5-méthyl-3-pyridinyl)carbonyl]-4-pipéridinyl}-5,6,7,8-tétrahydro-4*H*-[1,3]thiazolo[4,5-*d*]-azépine ;
la 6-cyclobutyl-2-{1-[(4-méthylphényl)carbonyl]-4-pipéridinyl}-5,6,7,8-tétrahydro-4*H*-[1,3]thiazolo[4,5-*d*]-azépine ;
la 6-cyclobutyl-2-{1-[(5-méthyl-3-isoxazolyl)carbonyl]-4-pipéridinyl}-5,6,7,8-tétrahydro-4*H*-[1,3]thiazolo[4,5-*d*]-azépine ;
la 6-cyclobutyl-2-[1-(1,2,3-thiadiazole-4-ylcarbonyl)-4-pipéridinyl]-5,6,7,8-tétrahydro-4*H*-[1,3]thiazolo[4,5-*d*]-azépine ;
la 6-cyclobutyl-2-{1-[(1-méthyl-1*H*-pyrazole-3-yl)carbonyl]-4-pipéridinyl}-5,6,7,8-tétrahydro-4*H*-[1,3]thiazolo[4,5-*d*]-azépine ;
la 6-cyclobutyl-2-{1-[(3-méthyl-5-isoxazolyl)carbonyl]-4-pipéridinyl}-5,6,7,8-tétrahydro-4*H*-[1,3]thiazolo[4,5-*d*]-azépine ;
la 6-cyclobutyl-2-[1-(6-méthyl-3-pyridinyl)-4-pipéridinyl]-5,6,7,8-tétrahydro-4*H*-[1,3]thiazolo[4,5-*d*]azépine ;
le 5-[4-(6-cyclobutyl-5,6,7,8-tétrahydro-4*H*-[1,3]thiazolo-[4,5-*d*]azépine-2-yl)-1-pipéridinyl]-2-pyridinecarbonitrile ;
l'acide 5-[4-(6-cyclobutyl-5,6,7,8-tétrahydro-4*H*-[1,3]-thiazolo[4,5-*d*]azépine-2-yl)-1-pipéridinyl]-2-pyridinecarboxylique ;
le 5-[4-(6-cyclobutyl-5,6,7,8-tétrahydro-4*H*-[1,3]thiazolo-[4,5-*d*]azépine-2-yl)-1-pipéridinyl]-2-pyridinecarboxamide ;
la 6-cyclobutyl-2-{1-[6-(1*H*-imidazole-1-ylcarbonyl)-3-pyridinyl]-4-pipéridinyl}-5,6,7,8-tétrahydro-4*H*-[1,3]thiazolo-[4,5-*d*]azépine ;
le 5-[4-(6-cyclobutyl-5,6,7,8-tétrahydro-4*H*-[1,3]thiazolo-[4,5-*d*]azépine-2-yl)-1-pipéridinyl]-*N*,*N*-diméthyl-2-pyridinecarboxamide ;
la 6-cyclobutyl-2-{1-[(2-méthyl-3-pyridinyl)carbonyl]-4-pipéridinyl}-5,6,7,8-tétrahydro-4*H*-[1,3]thiazolo[4,5-*d*]-azépine ;
la 1-[4-(6-cyclobutyl-5,6,7,8-tétrahydro-4*H*-[1,3]thiazolo-[4,5-*d*]azépine-2-yl)phényl]-3-méthyl-2-imidazolidinone ;
la 1-[4-(6-cyclobutyl-5,6,7,8-tétrahydro-4*H*-[1,3]thiazolo-[4,5-*d*]azépine-2-yl)phényl]-2-imidazolidinone ;
la 1-[4-(6-cyclopentyl-5,6,7,8-tétrahydro-4*H*-[1,3]thiazolo-[4,5-*d*]azépine-2-yl)phényl]-3-méthyl-2-imidazolidinone ;
la 1-méthyl-3-{4-[6-(1-méthyléthyl)-5,6,7,8-tétrahydro-4*H*-[1,3]thiazolo[4,5-*d*]azépine-2-yl]phényl}-2-imidazolidinone ;
la 1-[4-(6-cyclohexyl-5,6,7,8-tétrahydro-4*H*-[1,3]thiazolo-[4,5-*d*]azépine-2-yl)phényl]-3-méthyl-2-imidazolidinone ;
la 1-{4-[6-(cyclopropylméthyl)-5,6,7,8-tétrahydro-4*H*-[1,3]-thiazolo[4,5-*d*]azépine-2-yl]phényl}-3-méthyl-2-imidazolidinone ;
la 1-méthyl-3-{4-[6-(3-méthylpropyl)-5,6,7,8-tétrahydro-4*H*-[1,3]thiazolo[4,5-*d*]azépine-2-yl]phényl}-2-imidazolidinone ;
le 3-(6-cyclobutyl-5,6,7,8-tétrahydro-4*H*-[1,3]thiazolo-[4,5-*d*]azépine-2-yl)-1-pyrrolidinecarboxylate de 1,1-diméthyléthyle ;
la 6-cyclobutyl-2-(3-pyrrolidinyl)-5,6,7,8-tétrahydro-4*H*-[1,3]thiazolo[4,5-*d*]azépine ;
le (±)-5-[3-(6-cyclobutyl-5,6,7,8-tétrahydro-4*H*-[1,3]-thiazolo[4,5-*d*]azépine-2-yl)-1-pyrrolidinyl]-2-pyridinecarbonitrile ;
le (-)-5-[3-(6-cyclobutyl-5,6,7,8-tétrahydro-4*H*-[1,3]-thiazolo[4,5-*d*]azépine-2-yl)-1-pyrrolidinyl]-2-pyridinecarbonitrile ;
le (+)-5-[3-(6-cyclobutyl-5,6,7,8-tétrahydro-4*H*-[1,3]-thiazolo[4,5-*d*]azépine-2-yl)-1-pyrrolidinyl]-2-pyridinecarbonitrile ;
la (±)-6-cyclobutyl-2-[1-(6-méthyl-3-pyridinyl)-3-pyrrolidinyl]-5,6,7,8-tétrahydro-4*H*-[1,3]thiazolo[4,5-*d*]-azépine ;
le 5-[3-(6-cyclobutyl-5,6,7,8-tétrahydro-4*H*-[1,3]thiazolo-[4,5-*d*]azépine-2-yl)-1-pyrrolidinyl]-3-pyridinecarbonitrile ;
le 4-(6-cyclobutyl-5,6,7,8-tétrahydro-4*H*-[1,3]thiazolo-[4,5-*d*]azépine-2-yl)benzoate de méthyle ;
l'acide 4-(6-cyclobutyl-5,6,7,8-tétrahydro-4*H*-[1,3]thiazolo-[4,5-*d*]azépine-2-yl)benzoïque ;
la 6-cyclobutyl-2-phényl-5,6,7,8-tétrahydro-4*H*-[1,3]thiazolo-[4,5-*d*] azépine ;
le 4-(6-cyclobutyl-5,6,7,8-tétrahydro-4*H*-[1,3]thiazolo-[4,5-*d*]azépine-2-yl)-*N*-méthylbenzamide ;
la 3-[4-(6-cyclobutyl-5,6,7,8-tétrahydro-4*H*-[1,3]thiazolo-[4,5-*d*]azépine-2-yl)phényl]-1,3-oxazolidine-2-one ;
la 6-cyclobutyl-2-[4-(1-pyrrolidinylcarbonyl)phényl]-5,6,7,8-tétrahydro-4*H*-[1,3]thiazolo[4,5-*d*]azépine ;
le 4-(6-cyclobutyl-5,6,7,8-tétrahydro-4*H*-[1,3]thiazolo-[4,5-*d*]azépine-2-yl)benzamide ;
le 4-(6-cyclobutyl-5,6,7,8-tétrahydro-4*H*-[1,3]thiazolo-[4,5-*d*]azépine-2-yl)-*N*-éthylbenzamide ;
le 4-(6-cyclobutyl-5,6,7,8-tétrahydro-4*H*-[1,3]thiazolo-[4,5-*d*]azépine-2-yl)benzonitrile ;
le 5-(6-cyclobutyl-5,6,7,8-tétrahydro-4*H*-[1,3]thiazolo-[4,5-*d*]azépine-2-yl)-2-pyridinecarbonitrile ;
l'acide 5-(6-cyclobutyl-5,6,7,8-tétrahydro-4*H*-[1,3]-thiazolo[4,5-*d*]azépine-2-yl)-2-pyridinecarboxylique ;
le 5-(6-cyclobutyl-5,6,7,8-tétrahydro-4*H*-[1,3]thiazolo-[4,5-*d*]azépine-2-yl)-*N*-méthyl-2-pyridinecarboxamide ;
la 1-[5-(6-cyclobutyl-5,6,7,8-tétrahydro-4*H*-[1,3]thiazolo-[4,5-*d*]azépine-2-yl)-2-pyridinyl]-2-pyrrolidinone ;
la 1-[5-(6-cyclobutyl-5,6,7,8-tétrahydro-4*H*-[1,3]thiazolo-[4,5-*d*]azépine-2-yl)-2-pyridinyl]-3-méthyl-2-imidazolidinone ;
la 2-(6-chloro-3-pyridinyl)-6-cyclobutyl-5,6,7,8-tétrahydro-4*H*-[1,3]thiazolo[4,5-*d*]azépine ;
la 6-cyclobutyl-2-[6-(1-pipéridinyl)-3-pyridinyl]-5,6,7,8-tétrahydro-4*H*-[1,3]thiazolo[4,5-*d*]azépine ;
le 5-(6-cyclobutyl-5,6,7,8-tétrahydro-4*H*-[1,3]thiazolo-[4,5-*d*]azépine-2-yl)-2-pyrimidinecarbonitrile ;
la 6-cyclobutyl-2-[2-(méthyloxy)-5-pyrimidinyl]-5,6,7,8-tétrahydro-4*H*-[1,3]thiazolo[4,5-*d*]azépine ;
la 6-cyclobutyl-2-(5-pyrimidinyl)-5,6,7,8-tétrahydro-4*H*-[1,3]thiazolo[4,5-*d*]azépine ;
la 5-(6-cyclobutyl-5,6,7,8-tétrahydro-4*H*-[1,3]thiazolo-[4,5-*d*]azépine-2-yl)-2(1*H*)-pyrimidinone ;
la 2-(2-chloro-5-pyrimidinyl)-6-cyclobutyl-5,6,7,8-tétrahydro-4*H*-[1,3]thiazolo[4,5-*d*]azépine ;
la 6-cyclobutyl-2-[2-(1-pipéridinyl)-5-pyrimidinyl]-5,6,7,8-tétrahydro-4*H*-[1,3]thiazolo[4,5-*d*]azépine ;
la 1-{4-[6-(cyclohexylméthyl)-5,6,7,8-tétrahydro-4*H*-[1,3]-thiazolo[4,5-*d*]azépine-2-yl]phényl}-3-méthyl-2-imidazolidinone ;
la 1-[4-(6-éthyl-5,6,7,8-tétrahydro-4*H*-[1,3]thiazolo-[4,5-*d*]azépine-2-yl)phényl]-3-méthyl-2-imidazolidinone ;
la 6-cyclobutyl-2-{1-[(6-méthyl-3-pyridazinyl)carbonyl]-4-pipéridinyl}-5,6,7,8-tétrahydro-4*H*-[1,3]thiazolo[4,5-*d*]-azépine ;
la 6-cyclobutyl-2-{1-[(6-méthyl-3-pyridinyl)carbonyl]-3-pyrrolidinyl}-5,6,7,8-tétrahydro-4*H*-[1,3]thiazolo[4,5-*d*]-azépine ;
le 5-[4-(6-cyclobutyl-5,6,7,8-tétrahydro-4*H*-[1,3]thiazolo-[4,5-*d*]azépine-2-yl)-1-pipéridinyl]-3-pyridinecarbonitrile ;
le 4-[4-(6-cyclobutyl-5,6,7,8-tétrahydro-4*H*-[1,3]thiazolo-[4,5-*d*]azépine-2-yl)-1-pipéridinyl]benzonitrile ;
le 4-(6-cyclobutyl-5,6,7,8-tétrahydro-4*H*-[1,3]thiazolo-[4,5-*d*]azépine-2-yl)-*N*-méthyl-1-pipéridinecarboxamide ;
le 4-(6-cyclobutyl-5,6,7,8-tétrahydro-4*H*-[1,3]thiazolo-[4,5-*d*]azépine-2-yl)-*N*-(1-méthyléthyl)-1-pipéridinecarboxamide ;
le 4-(6-cyclobutyl-5,6,7,8-tétrahydro-4*H*-[1,3]thiazolo-[4,5-*d*]azépine-2-yl)-*N*-(2-méthylphényl)-1-pipéridinecarboxamide ;
le 4-(6-cyclobutyl-5,6,7,8-tétrahydro-4*H*-[1,3]thiazolo-[4,5-*d*]azépine-2-yl)-*N*-(3-méthylphényl)-1-pipéridinecarboxamide ;
le 4-(6-cyclobutyl-5,6,7,8-tétrahydro-4*H*-[1,3]thiazolo-[4,5-*d*]azépine-2-yl)-*N*-(4-méthylphényl)-1-pipéridinecarboxamide ;
le 6-(6-cyclobutyl-5,6,7,8-tétrahydro-4*H*-[1,3]thiazolo-[4,5-*d*]azépine-2-yl)-3-pyridinecarbonitrile ;
le 6-(6-cyclopentyl-5,6,7,8-tétrahydro-4*H*-[1,3]thiazolo-[4,5-*d*]azépine-2-yl)-3-pyridinecarbonitrile ;
l'acide 6-(6-cyclobutyl-5,6,7,8-tétrahydro-4*H*-[1,3]-thiazolo[4,5-*d*]azépine-2-yl)-3-pyridinecarboxylique ;
le 6-(6-cyclobutyl-5,6,7,8-tétrahydro-4*H*-[1,3]thiazolo-[4,5-*d*]azépine-2-yl)-*N*-méthyl-3-pipéridinecarboxamide ;
le 5-[4-(6-cyclobutyl-5,6,7,8-tétrahydro-4*H*-[1,3]thiazolo-[4,5-*d*]azépine-2-yl)-1-piperidinyl]-*N*-méthyl-2-pyridinecarboxamide ;
la 6-cyclobutyl-2-{1-[(2-méthylphényl)carbonyl]-4-pipéridinyl}-5,6,7,8-tétrahydro-4*H*-[1,3]thiazolo[4,5-*d*]azépine ;
la 6-cyclobutyl-2-{1-[(3-méthyl-1*H*-pyrazole-5-yl)carbonyl]-4-pipéridinyl}-5,6,7,8-tétrahydro-4*H*-[1,3]thiazolo[4,5-*d*]-azépine ;
la 2-{1-[(4-chlorophényl)carbonyl]-4-pipéridinyl}-6-cyclobutyl-5,6,7,8-tétrahydro-4*H*-[1,3]thiazolo[4,5-*d*]azépine ;
la 2-{1-[(3-chlorophényl)carbonyl]-4-pipéridinyl}-6-cyclobutyl-5,6,7,8-tétrahydro-4*H*-[1,3]thiazolo[4,5-*d*]azépine ;
la 6-cyclobutyl-2-{1-[(3-méthylphényl)carbonyl]-4-pipéridinyl}-5,6,7,8-tétrahydro-4*H*-[1,3]thiazolo[4,5-*d*]azépine ;
la 6-cyclobutyl-2-{1-[(3-méthyl-2-pyridinyl)carbonyl]-4-pipéridinyl}-5,6,7,8-tétrahydro-4*H*-[1,3]thiazolo[4,5-*d*]-azépine ;
la 6-cyclobutyl-2-[1-(1*H*-pyrazole-3-ylcarbonyl)-4-pipéridinyl]-5,6,7,8-tétrahydro-4*H*-[1,3]thiazolo[4,5-*d*]azépine ;
la 6-cyclobutyl-2-{1-[(1,4-diméthyl-1*H*-pyrazole-3-yl)-carbonyl]-4-pipéridinyl}-5,6,7,8-tétrahydro-4*H*-[1,3]thiazolo-[4,5-*d*]azépine ;
la 6-cyclobutyl-2-{1-[(1-méthyl-1*H*-pyrrole-2-yl)carbonyl]-4-pipéridinyl}-5,6,7,8-tétrahydro-4*H*-[1,3]thiazolo[4,5-*d*]-azépine ;
la 6-cyclobutyl-2-{1-[(1-méthyl-1*H*-imidazole-5-yl)carbonyl]-4-pipéridinyl}-5,6,7,8-tétrahydro-4*H*-[1,3]thiazolo[4,5-*d*]azépine ;
la 6-cyclobutyl-2-{1-[(1,5-diméthyl-1*H*-pyrazole-3-yl)-carbonyl]-4-pipéridinyl]-5,6,7,8-tétrahydro-4*H*-[1,3]thiazolo-[4,5-*d*]azépine ;
la 6-cyclobutyl-2-{1-[(1-méthyl-1*H*-imidazole-4-yl)carbonyl]-4-pipéridinyl}-5,6,7,8-tétrahydro-4*H*-[1,3]thiazolo[4,5-*d*]azépine ;
la 6-cyclobutyl-2-[1-(2-pyridinylcarbonyl)-4-pipéridinyl]-5,6,7,8-tétrahydro-4*H*-[1,3]thiazolo[4,5-*d*]azépine ;
la 6-cyclobutyl-2-[1-(3-pyridinylcarbonyl)-4-pipéridinyl]-5,6,7,8-tétrahydro-4*H*-[1,3]thiazolo[4,5-*d*]azépine ;
la 6-cyclobutyl-2-{1-[(6-méthyl-2-pyrazinyl)carbonyl]-4-pipéridinyl}-5,6,7,8-tétrahydro-4*H*-[1,3]thiazolo[4,5-*d*]-azépine ;
la 6-cyclobutyl-2-{1-[(2-méthyl-4-pyridinyl)carbonyl]-4-pipéridinyl}-5,6,7,8-tétrahydro-4*H*-[1,3]thiazolo[4,5-*d*]-azépine ;
la 6-cyclobutyl-2-[1-(4-pyridinylcarbonyl)-4-pipéridinyl]-5,6,7,8-tétrahydro-4H-[1,3]thiazolo[4,5-d]azépine ;
la 6-cyclobutyl-2-{1-[(2-méthyl-4-pyrimidinyl)carbonyl]-4-pipéridinyl}-5,6,7,8-tétrahydro-4H-[1,3]thiazolo[4,5-d]-azépine ;
la 6-cyclobutyl-2-{1-[(2-méthyl-5-pyrimidinyl)carbonyl]-4-pipéridinyl}-5,6,7,8-tétrahydro-4H-[1,3]thiazolo[4,5-d]-azépine ;
la 6-cyclobutyl-2-[1-(4-morpholinylcarbonyl)-4-pipéridinyl] - 5,6,7,8-tétrahydro-4H-[1,3]thiazolo[4,5-d]azépine ;
la 6-cyclobutyl-2-[1-(1-pyrrolidinylcarbonyl)-4-pipéridinyl]-5,6,7,8-tétrahydro-4H-[1,3]thiazolo[4,5-d]azépine;
la 6-cyclobutyl-2-[4-(1,1-dioxydo-2-isothiazolidinyl)-phényl]-5,6,7,8-tétrahydro-4H-[1,3]thiazolo[4,5-d]azépine ;
le 5-(6-cyclobutyl-5,6,7,8-tétrahydro-4H-[1,3]thiazolo-[4,5-d]azépine-2-yl)-2-pyridinecarboxamide ;
la 6-cyclobutyl-2-[6-(1-pyrrolidinylcarbonyl)-3-pyridinyl]-5,6,7,8-tétrahydro-4H-[1,3]thiazolo[4,5-d]azépine ;
la 6-cyclobutyl-2-[6-(3-méthyl-1,2,4-oxadiazole-5-yl)-3-pyridinyl]-5,6,7,8-tétrahydro-4H-[1,3]thiazolo[4,5-d]azépine ;
la 1-[5-(6-cyclobutyl-5,6,7,8-tétrahydro-4H-[1,3]thiazolo-[4,5-d]azépine-2-yl)-2-pyridinyl]-2-imidazolidinone ;
le 5-(6-cyclobutyl-5,6,7,8-tétrahydro-4H-[1,3]thiazolo-[4,5-d]azépine-2-yl)-2-pyrazinecarboxylate de méthyle ;
le 4-{[3-(6-cyclobutyl-5,6,7,8-tétrahydro-4H-[1,3]thiazolo-[4,5-d]azépine-2-yl)-1-pyrrolidinyl]carbonyl}benzonitrile ;
la 6-cyclobutyl-2-{1-[(6-méthyl-2-pyridinyl)carbonyl]-3-pyrrolidinyl}-5,6,7,8-tétrahydro-4H-[1,3]thiazolo[4,5-d]-azépine ;
la 6-cyclobutyl-2-[6-(1-pyrrolidinyl)-3-pyridinyl]-5,6,7,8-tétrahydro-4H-[1,3]thiazolo[4,5-d]azépine ;
la 6-cyclobutyl-2-[6-(4-morpholinyl)-3-pyridinyl]-5,6,7,8-tétrahydro-4H-[1,3]thiazolo[4,5-d]azépine ;
le 5-(6-cyclobutyl-5,6,7,8-tétrahydro-4H-[1,3]thiazolo-[4,5-d]azépine-2-yl)-N-méthyl-2-pyrazinecarboxamide ;
la 6-cyclobutyl-2-[4-(4-morpholinyl)phényl]-5,6,7,8-tétrahydro-4H-[1,3]thiazolo[4,5-d]azépine ;
la 6-cyclobutyl-2-(4-méthyl-3,4-dihydro-2H-1,4-benzoxazine-7-yl)-5,6,7,8-tétrahydro-4H-[1,3]thiazolo[4,5-d]azépine ;
le 3-(6-cyclobutyl-5,6,7,8-tétrahydro-4H-[1,3]thiazolo-[4,5-d]azépine-2-yl)benzonitrile ;
le 3-(6-cyclopentyl-5,6,7,8-tétrahydro-4H-[1,3]thiazolo-[4,5-d]azépine-2-yl)benzonitrile ;
la (±)-6-cyclobutyl-2-[1-(6-méthyl-2-pyridinyl)-3-pyrrolidinyl]-5,6,7,8-tétrahydro-4H-[1,3]thiazolo[4,5-d]azépine ;
le 4-{[4-(6-cyclobutyl-5,6,7,8-tétrahydro-4H-[1,3]thiazolo-[4,5-d]azépine-2-yl)-1-pipéridinyl]carbonyl}benzonitrile ;
la 4-(6-cyclobutyl-5,6,7,8-tétrahydro-4H-[1,3]thiazolo-[4,5-d]azépine-2-yl)-N,N-diméthylaniline ;
ou un de ses sels ou produits de solvatation pharmaceutiquement acceptables.

11. Composé suivant la revendication 10, qui est :
la 1-[4-(6-cyclobutyl-5,6,7,8-tétrahydro-4*H*-[1,3]thiazolo-[4,5-*d*]azépine-2-yl)phényl]-2-pyrrolidinone ;
la 6-cyclobutyl-2-{1-[(6-méthyl-2-pyridinyl)carbonyl]-4-pipéridinyl}-5,6,7,8-tétrahydro-4*H*-[1,3]thiazolo[4,5-*d*]-azépine ;
la 6-cyclobutyl-2-{1-[(5-méthyl-3-pyridinyl)carbonyl]-4-pipéridinyl}-5,6,7,8-tétrahydro-4*H*-[1,3]thiazolo[4,5-*d*]-azépine ;
le 5-[4-(6-cyclobutyl-5,6,7,8-tétrahydro-4*H*-[1,3]thiazolo-[4,5-*d*]azépine-2-yl)-1-pipéridinyl]-2-pyridinecarbonitrile ;
le 5-[4-(6-cyclobutyl-5,6,7,8-tétrahydro-4*H*-[1,3]thiazolo-[4,5-*d*]azépine-2-yl)-1-pipéridinyl]-*N*,*N*-diméthyl-2-pyridinecarboxamide ;
la 1-[4-(6-cyclobutyl-5,6,7,8-tétrahydro-4*H*-[1,3]thiazolo-[4,5-*d*]azépine-2-yl)phényl]-3-méthyl-2-imidazolidinone ;
la 1-[4-(6-cyclobutyl-5,6,7,8-tétrahydro-4*H*-[1,3]thiazolo-[4,5-*d*]azépine-2-yl)phényl]-2-imidazolidinone ;
le (+)-5-[3-(6-cyclobutyl-5,6,7,8-tétrahydro-4*H*-[1,3]-thiazolo[4,5-*d*]azépine-2-yl)-1-pyrrolidinyl]-2-pyridinecarbonitrile ;
la (±)-6-cyclobutyl-2-[1-(6-méthyl-3-pyridinyl)-3-pyrrolidinyl]-5,6,7,8-tétrahydro-4*H*-[1,3]thiazolo[4,5-*d*]-azépine ;
ou un de ses sels ou produits de solvatation pharmaceutiquement acceptables.

12. Composé suivant la revendication 10, qui est
le 4-(6-cyclobutyl-5,6,7,8-tétrahydro-4*H*-[1,3]thiazolo-[4,5-*d*]azépine-2-yl)-*N*-méthyl-1-pipéridinecarboxamide 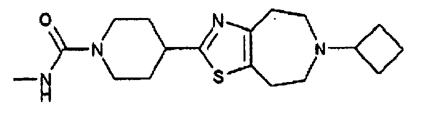 ou un de ses sels ou produits de solvatation pharmaceutiquement acceptables.

13. Composition pharmaceutique qui comprend le composé de formule (I) tel que défini dans l'une quelconque des revendications précédentes ou un de ses sels ou produits de solvatation pharmaceutiquement acceptables et un support ou excipient pharmaceutiquement acceptable.

14. Composé suivant l'une quelconque des revendications 1 à 12, destiné à être utilisé en thérapie.

15. Composé suivant l'une quelconque des revendications 1 à 12, destiné à être utilisé dans le traitement de maladies neurologiques.

16. Composé de formule (I) ou un de ses sels ou produits de solvatation pharmaceutiquement acceptables suivant l'une quelconque des revendications 1 à 12, destiné à être utilisé comme substance thérapeutique dans le traitement ou la prophylaxie d'altérations cognitives dans la maladie d'Alzheimer ou un trouble neurodégénératif apparenté.

17. Utilisation d'un composé suivant l'une quelconque des revendications 1 à 12 dans la production d'un médicament destiné au traitement de maladies neurologiques.

18. Procédé pour la préparation d'un composé de formule (I) ou d'un de ses sels ou produits de solvatation pharmaceutiquement acceptables tel que défini dans l'une quelconque des revendications 1 à 12, procédé qui comprend :
(a) la réaction d'un composé de formule (II) 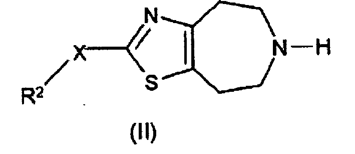 dans laquelle X et R² sont tels que définis dans la revendication 1, avec un composé de formule R^{1'}=O, dans laquelle R^{1'} représente un groupe =alkyle en C₂ à C₆, =(alkyle en C₁ à C₃) - (cycloalkyle en C₃ à C₈) ou =cycloalkyle en C₃ à C₇, où les groupes cycloalkyle peuvent être facultativement substitués avec un substituant alkyle en C₁ à C₃ ; ou
(b) la réaction d'un composé de formule (III) 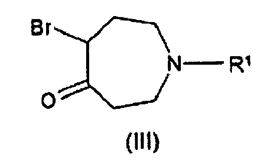 dans laquelle R¹ est tel que défini dans la revendication 1, avec un composé de formule R²X-C(=S)NH₂ dans laquelle R² et X sont tels que définis dans la revendication 1 ; ou
(c) la suppression de la protection d'un composé de formule (I) qui est protégé ; ou
(d) l'interconversion d'un composé de formule (I) en un autre ; ou
(e) la réaction d'un composé de formule (X) 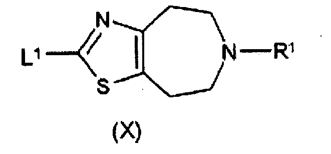 dans laquelle R¹ est tel que défini dans la revendication 1 et L¹ représente un groupe partant tel qu'un groupe halogéno, avec un composé organométallique de formule (XI), R²-X-L², dans laquelle R² et X sont tels que définis dans la revendication 1 et dans laquelle L² représente un groupe partant ; ou bien
(f) la réaction d'un composé de formule (II) 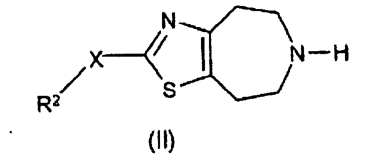
dans laquelle X et R² sont tels que définis dans la revendication 1, avec un composé de formule R¹-L³ dans laquelle R¹ est tel que défini dans la revendication 1 et dans laquelle L³ représente un groupe partant tel qu'un groupe halogéno.
